(19) Europäisches Patentamt
European Patent Office
Office européen des brevets

(11) EP 2 283 008 B1

(12) **FASCICULE DE BREVET EUROPEEN**

(45) Date de publication et mention de la délivrance du brevet:
**02.11.2011 Bulletin 2011/44**

(21) Numéro de dépôt: **09750014.4**

(22) Date de dépôt: **07.05.2009**

(51) Int Cl.:
***C07D 401/12*** (2006.01) ***C07D 401/14*** (2006.01)
***A61K 31/4025*** (2006.01) ***A61P 25/00*** (2006.01)
***A61P 29/00*** (2006.01) ***A61P 3/00*** (2006.01)
***A61P 1/00*** (2006.01) ***C07D 401/06*** (2006.01)
***C07D 207/34*** (2006.01) ***C07D 211/64*** (2006.01)
***C07D 211/62*** (2006.01) ***C07D 211/38*** (2006.01)
***C07D 207/38*** (2006.01) ***A61K 31/454*** (2006.01)
***A61K 31/4545*** (2006.01)

(86) Numéro de dépôt international:
**PCT/FR2009/000536**

(87) Numéro de publication internationale:
**WO 2009/141533 (26.11.2009 Gazette 2009/48)**

(54) **DERIVES DE 4,5-DIPHENYLEPYRROLE-2-YLCARBOXAMIDE, LEUR PREPARATION ET LEUR UTILISATION COMME ANTAGONISTES DES RECEPTEURS CB1 DES CANNABINOIDES**

PYRROLDERIVATE, IHRE HERSTELLUNG UND IHRE THERAPEUTISCHE VERWENDUNG

PYRROLE DERIVATIVES, PREPARATION OF SAME AND THERAPEUTIC APPLICATION THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **09.05.2008 FR 0802553**

(43) Date de publication de la demande:
**16.02.2011 Bulletin 2011/07**

(73) Titulaire: **SANOFI**
**75013 Paris (FR)**

(72) Inventeurs:
- **BARTH, Francis**
**F-75013 Paris (FR)**
- **CONGY, Christian**
**F-75013 Paris (FR)**
- **JEANJEAN, Audrey**
**F-75013 Pariss (FR)**
- **RINALDI-CARMONA, Murielle**
**F-75013 Paris (FR)**

(74) Mandataire: **Werner, Alain Henri et al**
**Sanofi-Aventis**
**Département Brevets**
**174, avenue de France**
**75013 Paris (FR)**

(56) Documents cités:
**FR-A1- 2 874 012** **FR-A1- 2 908 766**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 283 008 B1

## Description

La présente invention se rapporte à des dérivés de 4,5-diarylpyrrole-2-carboxamide, à leur préparation et à leur application en thérapeutique.

Des dérivés de 4,5-diphénylpyrrole-2-carboxamide, présentant une affinité pour les récepteurs $CB_1$ des cannabinoïdes ont été décrits dans la demande de brevet WO 2006/024 777. On a maintenant trouvé des nouveaux dérivés de 4,5-diarylpyrrole-2-carboxamide portant un substituant particulier sur l'un des groupes aryle, qui possèdent des propriétés antagonistes des récepteurs $CB_1$ des cannabinoïdes. En particulier, ces nouveaux dérivés ont des propriétés antagonistes des récepteurs CB1 périphériques et présentent une faible pénétration au niveau du cerveau.

La présente invention a pour objet des composés répondant à la formule :

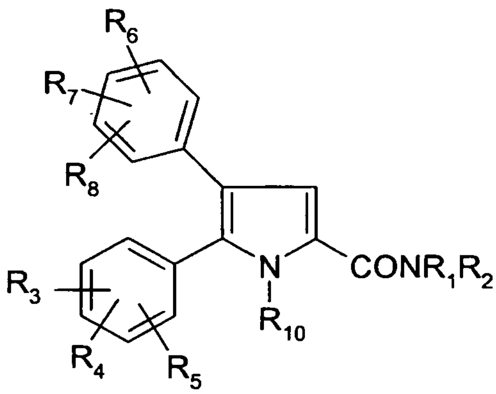

**(I)**

dans laquelle :

- $R_1$ représente un atome d'hydrogène ou un groupe ($C_1$-$C_4$)alkyle non substitué ou substitué par un ou plusieurs atomes de fluor ;
- $R_2$ représente :
- soit un radical homopipéridin-1-yle, pipéridin-1-yle, pyrrolidin-1-yle ou azétidin-1-yle, lesdits radicaux étant non substitués ou substitués une ou deux fois par un substituant choisi chacun indépendamment parmi un atome de fluor, un groupe ($C_1$-$C_4$)alkyle, ($C_1$-$C_4$)alcoxy, trifluorométhyle, $-OCF_3$, $-CH_2OH$, $-CONH_2$ et/ou un groupe phényle, ledit groupe phényle étant non substitué on substitué une ou deux fois par un substituant choisi chacun indépendamment parmi un atome d'halogène, un groupe $-CF_3$, un groupe méthoxy et/ou un groupe trifluorométhoxy ;
- soit un groupe amino ($C_1$-$C_6$)alkyle non substitué ou substitué par un ou plusieurs substituants choisis chacun indépendamment parmi un atome de fluor, un groupe hydroxyle, $-CONH_2$ et/ou un groupe phényle, ledit groupe phényle étant non substitué ou substitué une ou deux fois par un substituant choisi chacun indépendamment parmi un atome d'halogène, un groupe $-CF_3$, un groupe méthoxy et/ou un groupe trifluorométhoxy ;
- ou $R_1$ et $R_2$ ensemble avec l'atome d'azote auquel ils sont liés constituent :

    o soit un radical pipérazin-1-yle ou 1,4-diazépan-1-yle, lesdits radicaux étant non substitués ou substitués par un substituant choisi parmi un groupe phényle, benzodioxolyle, benzodioxolylméthyle, tétrahydrofuranylcarbonyle, $-COR_{11}$, et/ou $-CH_2COR_{11}$, le groupe phényle étant lui-même non substitué ou substitué une ou plusieurs fois par un substituant choisi chacun indépendamment parmi un atome d'halogène, un groupe ($C_1$-$C_4$)alkyle, trifluorométhyle, hydroxyle, ($C_1$-$C_4$)alcoxy et/ou cyano ;
    o soit un radical homopipéridin-1-yle, pipéridin-1-yle, pyrrolidin-1-yle ou azétidin-1-yle, lesdits radicaux étant non substitués ou substitués une ou deux fois par un substituant choisi chacun indépendamment parmi :

    - un atome de fluor, un groupe cyano, $-COR_{11}$, $-NR_{12}R_{13}$, $-NHCOR_{14}$, $-CH_2COR_{11}$, $-SO_2R_{14}$ , et/ou $-SO_2NR_{12}R_{13}$;
    - et/ou un groupe phényle, un groupe pyridinyle ; lesdits groupes étant non substitués ou substitués une ou plusieurs fois par un substituant choisi chacun indépendamment parmi un atome d'halogène, un groupe ($C_1$-$C_4$)alkyle, trifluorométhyle, hydroxyle, ($C_1$-$C_4$)alcoxy et/ou cyano ;
    - et/ou un groupe benzyle non substitué ou substitué une ou plusieurs fois par un substituant choisi chacun indépendamment parmi un atome d'halogène, un groupe ($C_1$-$C_4$)alkyle, trifluorométhyle, hydroxyle, ($C_1$-$C_4$)

alcoxy et/ou cyano ;

- et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou OCF3;
- et/ou un groupe aminophényle, aminobenzyle, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
- et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
- et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$alkyle, $(C_1-C_4)$ alcoxy et/ou cyano, ledit groupe $(C_1-C_4)$alkyle étant non substitué ou substitué une ou plusieurs fois par un atome de fluor ;

- $R_3$, $R_4$, $R_5$, $R_6$, $R_7$, $R_8$ représentent chacun indépendamment un atome d'hydrogène, un atome d'halogène, un groupement -CN, $-S(O)_nR_{14}$, $-OSO_2R_{14}$, un groupe $(C_1-C_6)$alkyle non substitué ou substitué une ou plusieurs fois par un atome de fluor, et/ou un groupe $(C_1-C_6)$alcoxy non substitué ou substitué par un ou plusieurs un atome de fluor, à la condition que l'un des deux substituants $R_3$, $R_6$ représente un groupe $Y-A-R_9$ ;
- Y représente une liaison directe, un atome d'oxygène, un groupe $-S(O)_n-$, $-OSO_2-$ ou $-N(R_{18})-$ ;
- A représente un groupe alkylène en $(C_1-C_4)$ non substitué ou substitué une ou plusieurs fois par un substituant choisi chacun indépendamment parmi un groupe $(C_1-C_3)$alkyle et/ou par un atome de fluor ;
- $R_9$ représente un groupe $-OR_{12}$, -CN, $-CO_2H$, $NR_{12}R_{13}$, $-CONR_{12}R_{13}$, $-NR_{15}COR_{12}$, $-CONHNH_2$, -CONHOH, $-CONHSO_2R_{14}$, $-S(O)_nR_{14}$, $-SO_2NR_{12}R_{13}$, $-NR_{18}SO_2R_{14}$, $-NR_{15}SO_2NR_{12}R_{13}$, ou un hétérocycle aromatique choisi parmi :

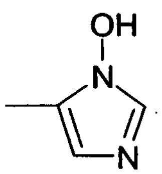 ; 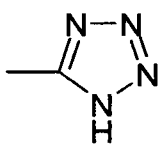 ; 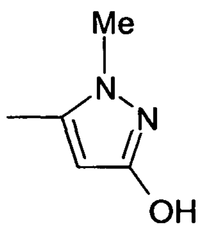 ; 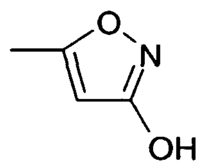 ;

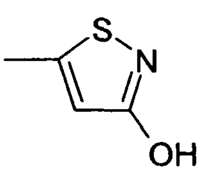 ; 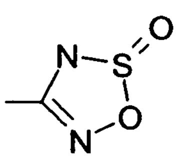 ; 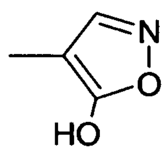 ; 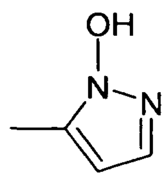

- $R_{10}$ représente un hydrogène ou un groupe $(C_1-C_4)$alkyle ;
- $R_{11}$ représente :

  o un groupe $(C_1-C_4)$alkyle, phényle, benzyle, $(C_1-C_4)$alcoxy, ou $(C_1-C_3)$alkylène-O-$(C_1-C_3)$alkyle, lesdits groupes étant non substitués ou substitués par un ou plusieurs substituants choisis chacun indépendamment parmi un groupe $(C_1-C_4)$alcoxy, un groupe hydroxy et/ou un atome de fluor ;
  o un trifluorométhyle ;
  o et/ou un groupement $-NR_{16}R_{17}$;

- $R_{12}$ et $R_{13}$ représentent chacun indépendamment un atome d'hydrogène ou un groupe $(C_1-C_6)$alkyle éventuellement substitué par un ou plusieurs substituants choisis chacun indépendamment parmi un atome de fluor, -OH et/ou $-OR_{14}$,
- ou $R_{12}$ et $R_{13}$ ensemble avec l'atome d'azote auquel ils sont liés constituent un radical hétérocyclique de 4 à 7 chainons pouvant comporter un second hétéroatome choisi parmi un atome d'azote, d'oxygène ou de soufre ;
- n représente 0, 1 ou 2 ;

- $R_{14}$ représente un groupe $(C_1-C_4)$alkyle non substitué ou substitué par un ou plusieurs atomes de fluor ;
- $R_{15}$ représente un atome d'hydrogène ou un groupe $(C_1-C_4)$alkyle ;
- $R_{16}$ et $R_{17}$ représentent chacun indépendamment :

  o un atome d'hydrogène ;
  o et/ou un groupe benzyle non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$ alcoxy et/ou cyano ;
  o et/ou un groupe $(C_1-C_6)$alkyle éventuellement substitué par un ou plusieurs substituants choisis chacun indépendamment parmi un atome d'halogène, -OH et/ou -OR 14 ;

- $R_{18}$ représente un atome d'hydrogène ou un groupe $(C_1-C_4)$alkyle non substitué ou substitué par un ou plusieurs atomes de fluor.

à l'état de bases (= correspondent aux formes libres des composés) ainsi que leurs sels acceptables pharmaceutiquement ou acceptables pour la purification et/ou isolement desdits composés de formule (I).

Les composés de formule (I) peuvent comporter un ou plusieurs atomes de carbone asymétriques. Ils peuvent donc exister sous forme d'énantiomères ou de diastéréoisoméres. Ces énantiomères, diastéréoisomères, ainsi que leurs mélanges, y compris les mélanges racémiques font partie de l'invention.

Les composés de formule (I) peuvent exister à l'état de bases (c'est-à-dire tels quels sous leurs formes libres), de sels d'addition à des acides ou de sels d'addition à des bases. Ces sels sont avantageusement préparés avec des sels pharmaceutiquement acceptables mais les sels d'autres acides utiles, par exemple, pour la purification ou l'isolement des composés de formule (I) font également partie de l'invention.

Par groupe $(C_1-C_3)$alkyle, $(C_1-C_4)$alkyle ou $(C_1-C_6)$alkyle, on entend respectivement un radical alkyle linéaire ou ramifié de un à trois atomes de carbone, de un à quatre atomes de carbone ou de un à six atomes de carbone, tel que le radical méthyle, éthyle, propyle, isopropyle, butyle, isobutyle, sec-butyle, tert-butyle, pentyle, isopentyle, n-hexyle, isohexyle. Les groupes méthyle et éthyles sont préférés pour un $(C_1-C_3)$alkyle, pour un $(C_1-C_4)$alkyle et pour un $(C_1-C_6)$ alkyle. Plus particulièrement, le groupe méthyle est préféré

Par groupe alkylène, on entend un radical carboné bivalent linéaire tel que $-(CH_2)-$, $-(CH_2)_2-$, $-(CH_2)_3-$, $-(CH_2)_4-$, $-(CH_2)_5-$.

Par $(C_1-C_4)$alcoxy, on entend un atome d'oxygène lié à un radical carboné linéaire ou ramifié de un à quatre atomes de carbone tel que le radical méthoxy, éthoxy, propoxy, isopropoxy, butoxy, *sec*-butoxy, *tert*-butoxy. Le groupe méthoxy est préféré.

Par atome d'halogène, on entend un atome de fluor, de chlore, de brome ou d'iode ; les atomes de fluor, chlore ou brome étant préférés.

Par groupe amino-dycloalkyle, on entend pour la partie cycloakyle un radical carboné cyclopropyle, cyclobutyle, cyclopentyle, cyclohexyle, cycloheptyle.

Par radical hétérocyclique, saturé ou insaturé, de 4 à 7 chainons, contenant ou non un deuxième hétéroatome tel que O, N ou S, on entend notamment des radicaux tels que homopipéridin-1-yle morpholin-4-yle, pipéridin-1-yle, pipérazin-1-yle, pyrrolidin-1-yle, azétidin-1-yle , les radicaux pipéridin-1-yle et pyrrolidin-1-yle étant préférés.

Selon la présente invention, on distingue :

- les composés de formule (IA) dans laquelle Y représente un atome d'oxygène ;
- les composés de formule (IB) dans laquelle Y correspond à une liaison directe ;
- les composés de formule (IC) dans laquelle Y représente un groupe $-S(O)_n-$ ;
- les composés de formule (ID) dans laquelle Y représente un groupe $-O(SO_2)-$
- et les composés de formule (IE) dans laquelle Y représente un groupe $-N(R^{18})-$
- les autres substituants étant tels que définis pour les composés de formule (I).

Au sein des composés de formules (I), (IA), (IB), (IC), (ID) et (IE), on distingue en particulier :

- les composés dans lesquels le substituant $R_6$ représente un groupe Y-A-$R_9$ et le substituant $R_3$ représente un atome d'hydrogène, un atome d'halogène, un groupement -CN, $-S(O)_nR_{14}$, $-OSO_2R_{14}$, un groupe $(C_1-C_6)$alkyle non substitué ou substitué une ou plusieurs fois par un atome de fluor, ou un groupe $(C_1-C_6)$alcoxy non substitué ou substitué par un ou plusieurs un atome de fluor ;
- et les composés dans lesquels le substituant $R_3$ représente un groupe Y-A-$R_9$ et le substituant $R_6$ représente un atome d'hydrogène, un atome d'halogène, un groupement -CN, $-S(O)_nR_{14}$, $-OSO_2R_{14}$, un groupe $(C_1-C_6)$alkyle non substitué ou substitué une ou plusieurs fois par un atome de fluor, ou un groupe $(C_1-C_6)$alcoxy non substitué

ou substitué par un ou plusieurs un atome de fluor ;

les autres substituants étant tels que définis ci-avant pour les composés de formule (I).

Selon la présente invention, on préfère les composés de formule (I) dans lesquels :

- A représente un groupe alkylène en ($C_1$-$C_4$) non substitué ;
- $R_9$ représente un groupe -$OR_{12}$, -$NR_{12}R_{13}$, -$CONR_{12}R_{13}$, -$NR_{15}COR_{12}$, -$CONHNH_2$, -CONHOH, -$S(O)_nR_{14}$, -$SO_2NR_{12}R_{13}$, -$NR_{18}SO_2R_{14}$ ou -$NR_{15}SO_2NR_{12}R_{13}$ ;
- et les autres substituants étant tels que définis ci-avant pour les composés de formule (I).

En particulier pour Y, on préfère un atome d'oxygène.

En particulier pour $R_9$, on préfère un groupe -$OR_{12}$, -NP-$_{12}R_{13}$, -$CONR_{12}R_{13}$, -$NR_{15}COR_{12}$ ou -$NR_{18}SO_2R_{14}$.

Rev11 Selon une première variante, on préfère les composés de formules (IA), (IB), (IC), (ID) et/ou (IE) dans lesquelles :

- $R_1$ représente un atome d'hydrogène ou un groupe ($C_1$-$C_4$)alkyle non substitué ou substitué par un ou plusieurs atomes de fluor ;
- $R_2$ représente :

  o soit un radical homopipéridin-1-yle, pipéridin-1-yle, pyrrolidin-1-yle ou azétidin-1-yle, lesdits radicaux étant substitués une ou deux fois par un substituant choisi chacun indépendamment parmi un atome de fluor, un groupe ($C_1$-$C_4$)alkyle , ($C_1$-$C_4$)alcoxy, trifluorométhyle, -$OCF_3$, -$CH_2OH$, -$CONH_2$ et/ou un groupe phényle, ledit groupe phényle étant non substitué ou substitué une ou deux fois par un substituant choisi chacun indépendamment parmi un atome d'halogène, un groupe -$CF_3$, un groupe méthoxy et/ou un groupe trifluorométhoxy ;

  o soit un groupe amino ($C_1$-$C_6$)alkyle substitué par un ou plusieurs substituants choisis chacun indépendamment parmi un atome de fluor, un groupe hydroxyle, -$CONH_2$ et/ou un groupe phényle, ledit groupe phényle étant non substitué ou substitué une ou deux fois par un substituant choisi chacun indépendamment parmi un atome d'halogène, un groupe -$CF_3$, un groupe méthoxy et/ou un groupe trifluorométhoxy ;

- les autres substituants étant tels que définis pour les composés de formule (I).

Pour cette première variante, on préfère les composés dans lesquels le radical $R_2$ représente en particulier un radical homopipéridin-1-yle, pipéridin-1-yle, pyrrolidin-1-yle ou azétidin-1-yle, lesdits radicaux étant substitués une ou deux fois par un substituant choisi chacun indépendamment parmi un atome de fluor, un groupe ($C_1$-$C_4$)alkyle, ($C_1$-$C_4$)alcoxy, trifluorométhyle, -$OCF_3$, -$CH_2OH$, -$CONH_2$ et/ou un groupe phényle, ledit groupe phényle étant non substitué ou substitué une ou deux fois par un substituant choisi chacun indépendamment parmi un atome d'halogène, un groupe -$CF_3$, un groupe méthoxy et/ou un groupe trifluorométhoxy ;

Pour cette première variante, on préfère les composés dans lesquels $R_1$ représente en particulier un atome d'hydrogène.

Selon une deuxième variante, on préfère les composés de formule (IA), (IB), (IC), (ID) et/ou (IE) dans lesquels :

- $R_1$ et $R_2$ ensemble avec l'atome d'azote auquel ils sont liés constituent :

  o soit un radical pipérazin-1-yle ou 1,4-diazépan-1-yle, lesdits radicaux étant substitués par un substituant choisi parmi un groupe phényle, benzodioxolyle, benzodioxolylméthyle, tétrahydrofuranylcarbonyle, -$COR_{11}$, et/ou -$CH_2COR_{11}$, le groupe phényle étant lui-même non substitué ou substitué une ou plusieurs fois par un substituant choisi chacun indépendamment parmi un atome d'halogène, un groupe ($C_1$-$C_4$)alkyle, trifluorométhyle, hydroxyle, ($C_1$-$C_4$)alcoxy et/ou cyano ;

  o soit un radical homopipéridin-1-yle, pipéridin-1-yle, pyrrolidin-1-yle ou azétidin-1-yle, lesdits radicaux étant substitués une ou deux fois par un substituant choisi chacun indépendamment parmi :

    - un atome de fluor, un groupe cyano, -$COR_{11}$, -$NR_{12}R_{13}$, -$NHCOR_{14}$, -$CH_2COR_{11}$, -$SO_2R_{14}$ et/ou -$SO_2NR_{12}R_{13}$;
    - et/ou un groupe phényle, pyridinyle ; lesdits groupes étant non substitués ou substitués une ou plusieurs fois par un substituant choisi chacun indépendamment parmi un atome d'halogène, un groupe ($C_1$-$C_4$) alkyle, trifluorométhyle, hydroxyle, ($C_1$-$C_4$)alcoxy et/ou cyano ;
    - et/ou un groupe benzyle substitué une ou plusieurs fois par un substituant choisi chacun indépendamment parmi un atome d'halogène, un groupe ($C_1$-$C_4$)alkylé, trifluorométhyle, hydroxyle, ($C_1$-$C_4$)alcoxy et/ou

cyano ;

- et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou - $OCF_3$ ;
- et/ou un groupe aminophényle, aminobenzyle, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
- et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
- et/ou un groupe amino $(C_3-C_7)$cycloakyle non substitué ou substitué une ou plusieurs fois par un substituant choisi chacun indépendamment parmi un atome d'halogène, un groupe hydroxyle, $(C_1-C_4)$alkyle, $(C_1-C_4)$alcoxy et/ou cyano, ledit groupe $(C_1-C_4)$alkyle étant non substitué ou substitué une ou plusieurs fois par un atome de fluor ;

- les autres substituants étant tels que définis pour les composés de formule (I).

En particulier pour cette deuxième variante, on préfère les composés dans lesquels :

- $R_1$ et $R_2$ ensemble avec l'atome d'azote auquel ils sont liés constituent un radical homopipéridin-1-yle, pipéridin-1-yle, pyrrolidin-1-yle ou azétidin-1-yle, lesdits radicaux étant substitués une ou deux fois par un substituant choisi chacun indépendamment parmi :

  o un atome de fluor, un groupe cyano, $-COR_{11}$, $-NR_{12}R_{13}$, $-NHCOR_{14}$, $-CH_2COR_{11}$, $-SO_2R_{14}$ et/ou $-SO_2NR_{12}R_{13}$ ;
  o et/ou un groupe phényle, pyridinyle ; lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  o et/ou un groupe benzyle substitué une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et cyano ;
  o et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou $-OCF_3$ ;
  o et/ou un groupe aminophényle, aminobenzyle, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  o et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
  o et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$alkyle, $(C_1-C_4)$alcoxy et/ou cyano, ledit groupe $(C_1-C_4)$alkyle étant non substitué ou substitué une ou plusieurs fois par un atome de fluor ;

- les autres substituants étant tels que définis pour les composés de formule (I).

Parmi les composés selon l'invention, on peut notamment citer les composés ci après, tels quels ainsi que leurs sels :

| Nom IUPAC | Structure Chimique |
|---|---|
| 1' [5-(4-(3-aminopropoxy)phényl)-4-(2,4-dichlorophényl)-1-méthyl-1*H* pyrrole-2-carbonyl]-[1,4']bipipéridinyl-4'-carboxamide | 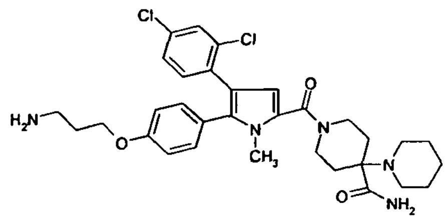 |
| 1'[5-[4-(3-carbamoylpropoxy)-phényl]-4-(2,4-dichlorophényl)-1-méthyl-1*H*-pyrrole-2-carbonyl]-[1,4']bipipéridinyl-4'-carboxamide | 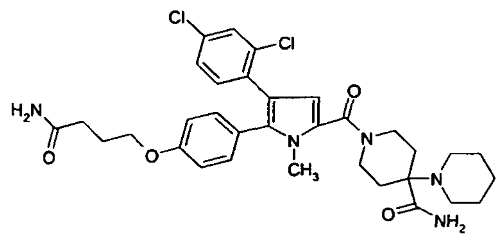 |
| 1'[4-(2,4-dichlorophényl)-5-(4-(3-méthanesulfonylaminopropoxy)phény 1)-1-méthyl-1*H*-pyrrole-2-carbonyl]-[1,4'] bipipéridinyl-4'-carboxamide | 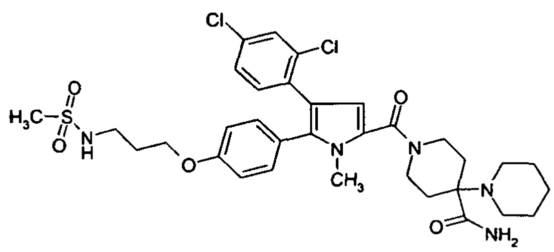 |
| 1'[5-(4-carbamoylméthoxyphényl)-4-(2,4-dichlorophényl)-1-méthyl-1*H*-pyrrole-2-carbonyl]-[1,4']bipipéridinyl-4'-carboxamide | 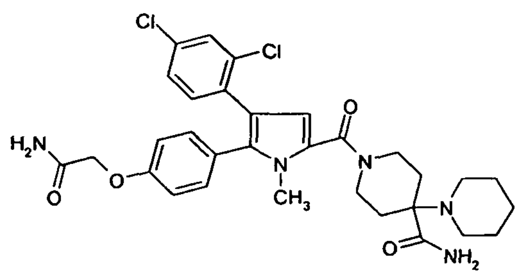 |
| 1'(4-(2,4-dichlorophényl)-5- {4-[3-(2-hydroxyacétylamino)-propoxy]-phényl}-1-méthyl-1*H*-pyrrole-2-carbonyl)-[1,4'] bipipéridinyl-4'-carboxamide | 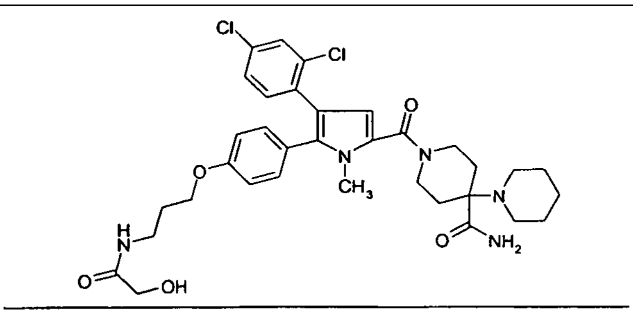 |

(suite)

| Nom IUPAC | Structure Chimique |
|---|---|
| 1'[4-(2,4-dichlorophényl)-5-[4-(3-hydroxypropoxy)-phényl]-1-méthyl-1*H*-pyrrole-2-carbonyl]-[1,4']bipipéridinyl-4'-carboxamide | 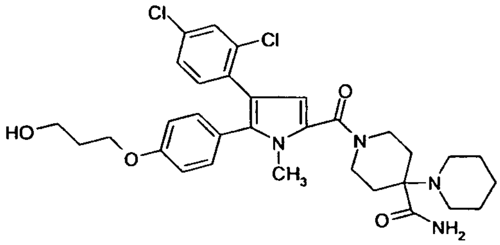 |
| 1'[4-(2,4-dichlorophényl)-5-[4-(3-méthanesulfonylaminopropoxy)-phényl]-1-méthyl-1*H*-pyrrole-2-carbonyl]-4,4-difluoro-[1,4']bipipéridinyl-4'-carboxamide | 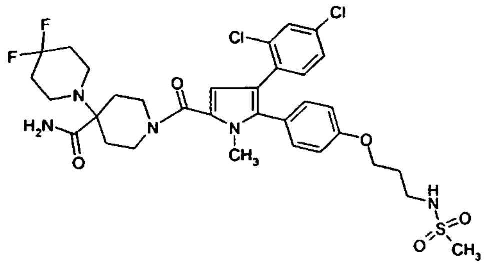 |
| 1'[4-(2,4-dichlorophényl)-5-(4-(3-méthylaminopropoxy)phényl)-1-méthyl-1*H*-pyrrole-2-carbonyl]-[1,4']bipipéridinyl-4'-carboxamide | 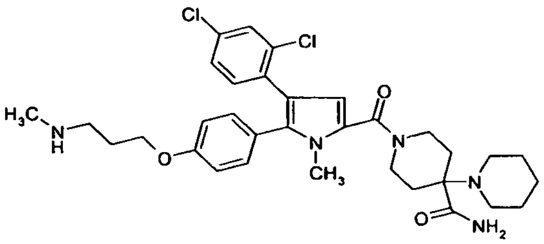 |
| 1'-[4-(2,4-dichlorophényl)-1-méthyl-5-[4-(3-pyrrolidin-1-yl-propoxy)-phényl]-1*H*-pyrrole-2-carbonyl]-[1,4']bipipéridinyl-4'-carboxamide | 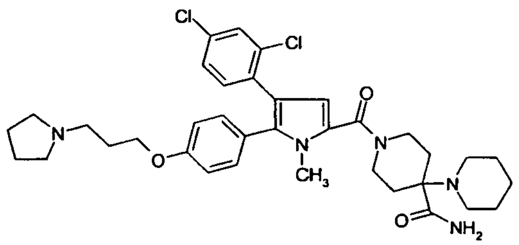 |
| 1[4-(2,4-dichlorophényl)-5-(4-(3-hydroxypropoxy)phényl)-1-méthyl-1*H*-pyrrole-2-carbonyl]-4-phénylpipéridine-4-carboxamide | 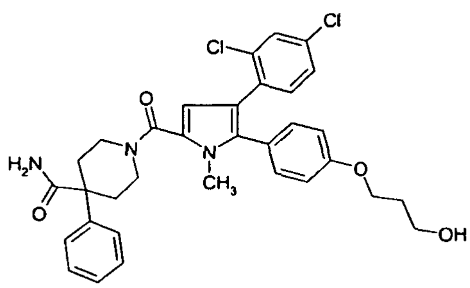 |

(suite)

| Nom IUPAC | Structure Chimique |
|---|---|
| 1[4-(2,4-dichlorophényl)-5-[4-(3-hydroxypropoxy)-phényl]-1-méthyl-1*H*-pyrrole-2-carbonyl]-4-(3-fluoro-benzylamino)-pipéridine-4-carboxamide | 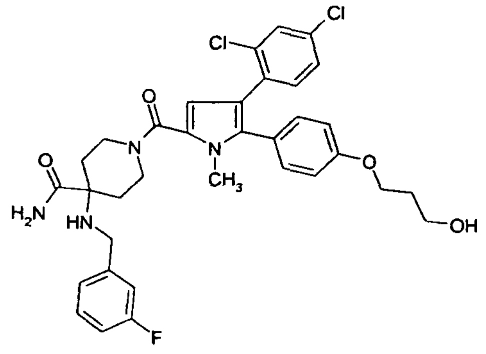 |
| 1[4-(2,4-dichlorophényl)-5-[4-(2-hydroxyéthoxy)-phényl]-1-méthyl-1*H*-pyrrole-2-carbonyl]-4-phényl-pipéridine-4-carboxamide | 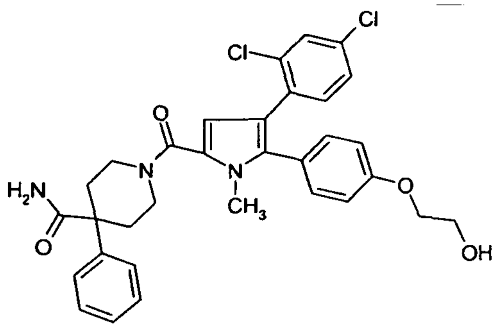 |
| 1[4-(2,4-dichlorophényl)-5-[4-(2-hydroxyéthoxy)-phényl]-1-méthyl-1*H*-pyrrole-2-carbonyl]-4-(3-fluoro-benzylamino)-pipéridine-4-carboxamide | 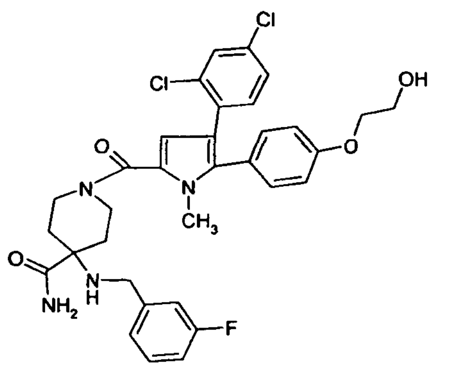 |
| 1-[5-[4-(3-carbamoylpropoxy)-phényl]-4-(2,4-dichlorophényl)-1-méthyl-1H-pyrrole-2-carbonyl]-4-[(3-fluoro-phénylamino)-méthyl]-pipéridine-4-carboxamide | 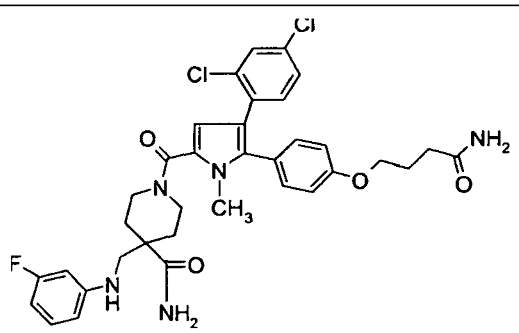 |

(suite)

| Nom IUPAC | Structure Chimique |
|---|---|
| 1-[5-[4-(3-carbamoylpropoxy)-phényl]-4-(2,4-dichlorophényl)-1-méthyl-1*H*-pyrrole-2-carbonyl]-4-phénylipéridine-4-carboxamide | 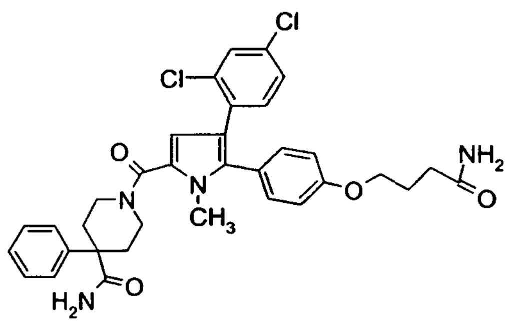 |
| 1-[5-(4-carbamoylméthoxy)-phényl-4-(2,4-dichlorophényl)-1-méthyl-1*H*-pyrrole-2-carbonyl]-4-phénylpipéridine-4-carboxamide | 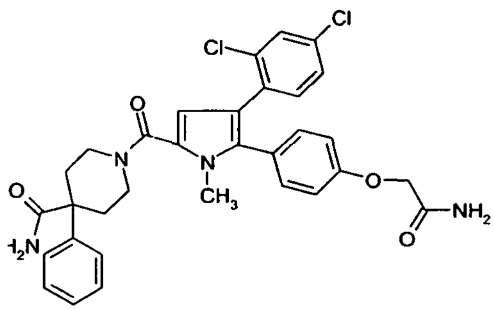 |
| Sel d'acide trifluoroacétique du 1-[5-(4-carbamoylméthoxyphényl)-4-(2,4-dichlorophényl)-1-méthyl-1*H*-pyrrole-2-carbonyl]-4-(4-fluoro-benzylamino)-pipéridine-4-carboxamide | 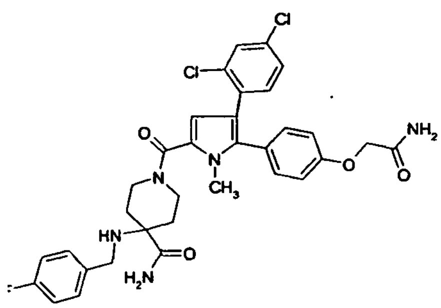 |
| 4-(4-chlorophényl)-1-[4-(2,4-dichlorophényl)-5-[4-(2-hydroxyéthoxy)-phényl]-1-méthyl-1*H*-pyrrole-2-carbonyl]-piperidine-4-carboxamide | 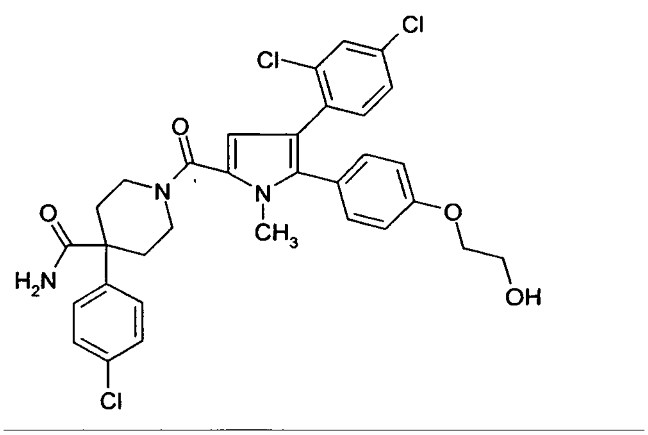 |

(suite)

| Nom IUPAC | Structure Chimique |
|---|---|
| 1-[4-(2,4-dichlorophényl)-5-[4-(2-hydroxyéthoxy)-phényl]-1-méthyl-1*H*-pyrrole-2-carbonyl]-4-(4-fluoro-benzylamino)-pipéridine-4-carboxamide | 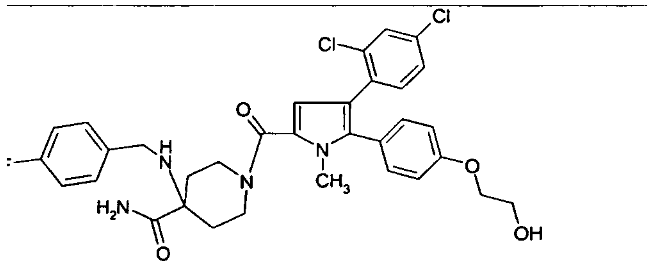 |
| 1-[4-(2-chlorophényl)-5-[4-(2-hydroxyéthoxy)-phényl]-1-méthyl-1*H*-pyrrole-2-carbonyl]-4-(4-fluoro-benzylamino)-pipéridine-4-carboxamide | 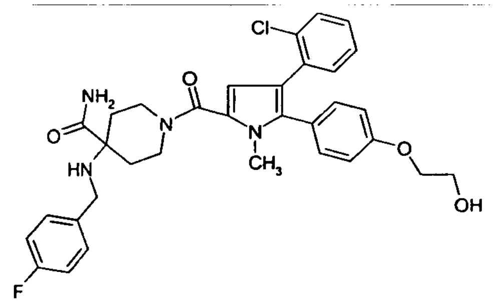 |
| 4-(4-chlorophényl)-1-[4-(2-chlorophényl)-5-[4-(2-hydroxyéthoxy)-phényl]-1-méthyl-1*H*-pyrrole-2-carbonyl]-piperidine-4-carboxamide | 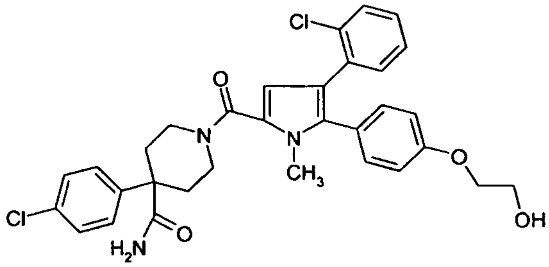 |
| 4-benzylamino-1-[4-(2-chlorophényl)-5-[4-(2-hydroxyéthoxy)-phényl]-1-méthyl-1*H*-pyrrole-2-carbonyl]-pipéridine-4-carboxamide | 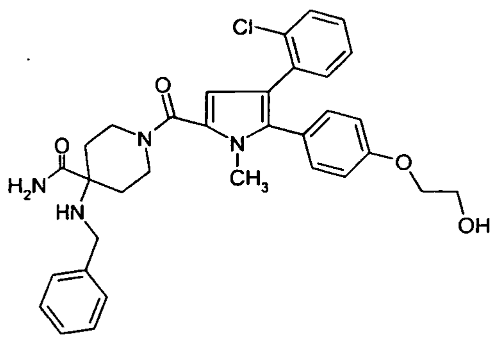 |

(suite)

| Nom IUPAC | Structure Chimique |
|---|---|
| 1-[4-(2-chlorophényl)-5-[4-(2-hydroxyéthoxy)-phényl]-1-méthyl-1*H*-pyrrole-2-carbonyl]-4-(3-fluoro-benzylamino)-pipéridine-4-carboxamide | 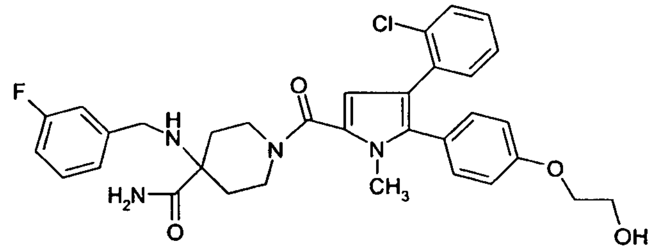 |
| 1-[4-(2-chlorophényl)-5-[4-(3-hydroxypropoxy)-phényl]-1-méthyl-1*H*-pyrrole-2-carbonyl]-4-(4-fluoro-benzylamino)-pipéridine-4-carboxamide | 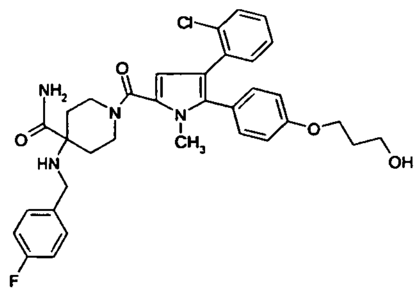 |
| 4-benzylamino-1-[4-(2-chlorophényl)-5-[4-(3-hydroxypropoxy)-phényl]-1-méthyl-1*H*-pyrrole-2-carbonyl]-pipéridine-4-carboxamide | 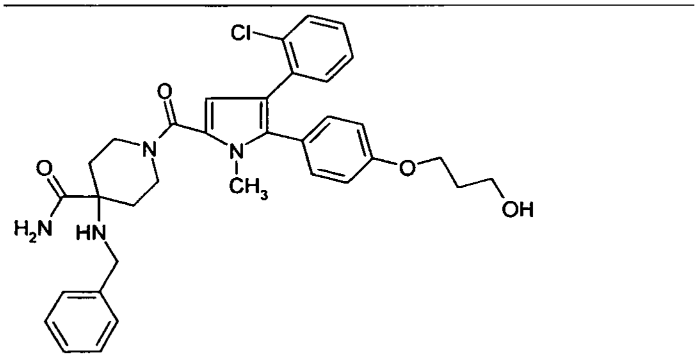 |
| 4-(4-chlorophényl)-1-[4-(2-chlorophényl)-S-[4-(3-hydroxypropoxy)-phényl]-1-méthyl-1*H*-pyrrole-2-carbonyl]-pipéridine-4-carboxamide | 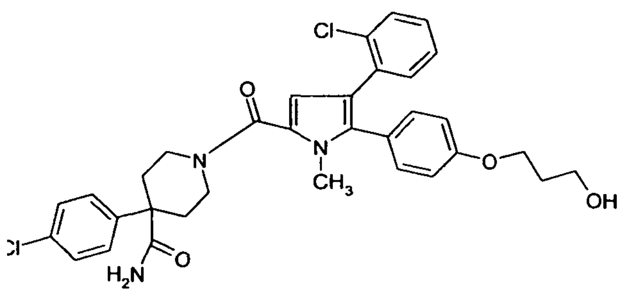 |
| 1-[4-(2-chlorophényl)-5-[4-(3-hydroxypropoxy)-phényl]-1-méthyl-1*H*-pyrrole-2-carbonyl]-4-(3-fluoro-benzylamino)-pipéridine-4-carboxamide | 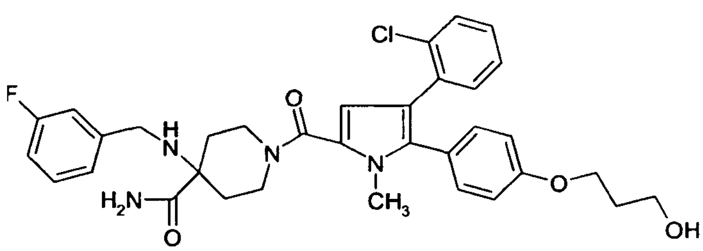 |

(suite)

| Nom IUPAC | Structure Chimique |
|---|---|
| 1-[5-(4-carbamoylméthoxyphényl)-4-(2-chlorophényl)-1-méthyl-1*H*-pyrrole-2-carbonyl]-4-(4-fluoro-benzylamino)-pipéridine-4-carboxamide | 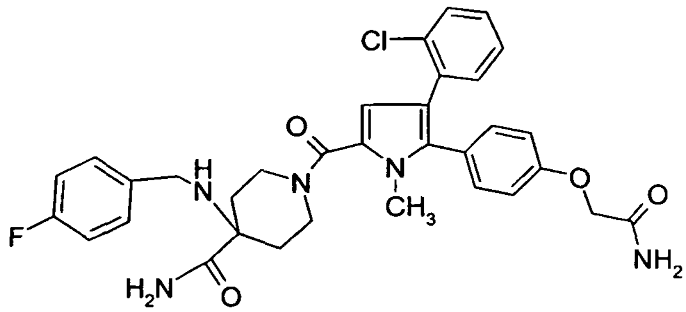 |
| 1-[5-(4-carbamoylméthoxyphényl)-4-(2-chlorophényl)-1-méthyl-1*H*-pyrrole-2-carbonyl]-4-(3-fluoro-benzylamino)-pipéridine-4-carboxamide | 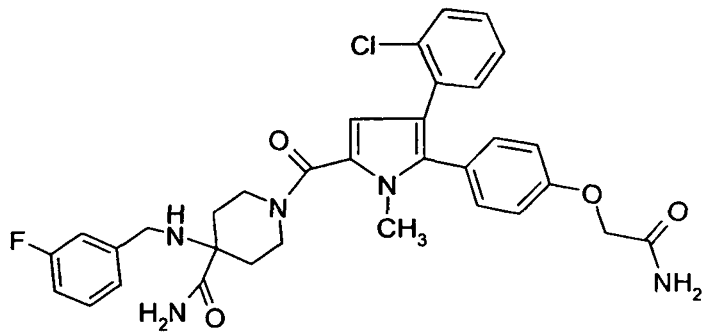 |

Parmi les composés listés ci-avant, sont préférés :

- 1[4-(2,4-dichlorophényl)-5-[4-(3-hydroxypropoxy)-phényl]-1-méthyl-1H-pyrrole-2-carbonyl]-4-(3-fluoro-benzylamino)-pipéridine-4-carboxamide,
- 1-[5-(4-carbamoylméthoxy)-phényl-4-(2,4-dichlorophényl)-1-méthyl-1H-pyrrole-2-carbonyl]-4-phénylpipéridine-4-carboxamide,
- 1-[4-(2-chlorophényl)-5-[4-(3-hydroxypropoxy)-phényl]-1-méthyl-1H-pyrrole-2-carbonyl]-4-(4-fluoro-benzylamino)-pipéridine-4-carboxamide,
- et leurs sels.

La présente invention a également pour objet un procédé de préparation des composés selon l'invention.

Ce procédé est caractérisé en ce que :

on traite l'acide de formule (II) ou un dérivé fonctionnel de cet acide de formule (II) :

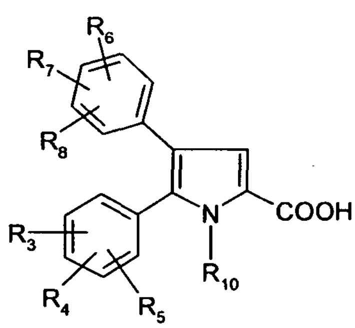

**(II)**

dans laquelle $R_3$, $R_4$, $R_5$, $R_6$, $R_7$, $R_8$ et $R_{10}$ sont tels que définis pour (I) avec une amine de formule $HNR_1R_2$ dans laquelle $R_1$ et $R_2$ sont tels que définis pour (I).

Les composés de formule (I) obtenus par les différents modes opératoires peuvent être ultérieurement séparés du milieu réactionnel et purifiés selon les méthodes classiques, par exemple par cristallisation ou chromatographie.

Eventuellement, on transforme le composé de formule (I) ainsi obtenu en un de ses sels.

Comme dérivé fonctionnel de l'acide (II) on peut utiliser notamment le chlorure d'acide, l'anhydride, un anhydride mixte, un ester alkylique en $C_1$-$C_4$ dans lequel l'alkyle est droit ou ramifié, un ester benzylique, un ester activé, par exemple l'ester de p-nitrophényle, ou l'acide libre opportunément activé, par exemple, avec le N,N-dicyclohexylcarbodiimide ou avec l'hexafluorophosphate de benzotriazol-1-yloxotris(diméthylamino)-phosphonium (BOP) ou hexafluorophosphate de benzotriazol-1-yloxotris-(pyrrolidino)phosphonium (PyBOP) ou hexafluorophosphate de N-[N-(dimethylamino)-1-1,2,3-triazolo[4,5-b]pyridin-1-ylméthylène] - N - méthylméthanaminium N-oxyde] (HBTU). Ces dérivés fonctionnels de l'acide (II) correspondent aux composés (IIbis).

Ainsi dans le procédé selon l'invention, on peut faire réagir le chlorure de l'acide de formule (II), obtenu par réaction du chlorure de thionyle ou du 1-chloro-N,N-2-triméthyl-1-propèn-1-amine effectué selon Chem. Comm., 1988, 475-477, à 0°C sur l'acide de formule (II), avec une amine $HNR_1R_2$, dans un solvant inerte, tel que par exemple un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la TA, en présence d'une amine tertiaire telle que par exemple la triéthylamine, la N-méthylmorpholine ou la pyridine.

Une variante consiste à préparer l'anhydride mixte de l'acide de formule (II) par réaction du chloroformiate d'éthyle avec l'acide de formule (II), en présence d'une base telle que par exemple la triéthylamine, et à le faire réagir avec une amine $HNR_1R_2$, dans un solvant tel que par exemple le dichlorométhane, sous une atmosphère inerte, à la TA, en présence d'une base telle que la triéthylamine.

Les composés de formule (II) peuvent être préparés selon le schéma 1 ci-après. Pour ce mode de préparation, le groupe chimique Y-A-$R_9$ est :

- soit présent au sein du composé (V) ; dans ce cas $R_3$ représente le groupe Y-A-$R_9$.
- soit présent au sein du composé (VIII) ; dans ce cas $R_6$ représente le groupe Y-A-$R_9$.

## Schéma 1

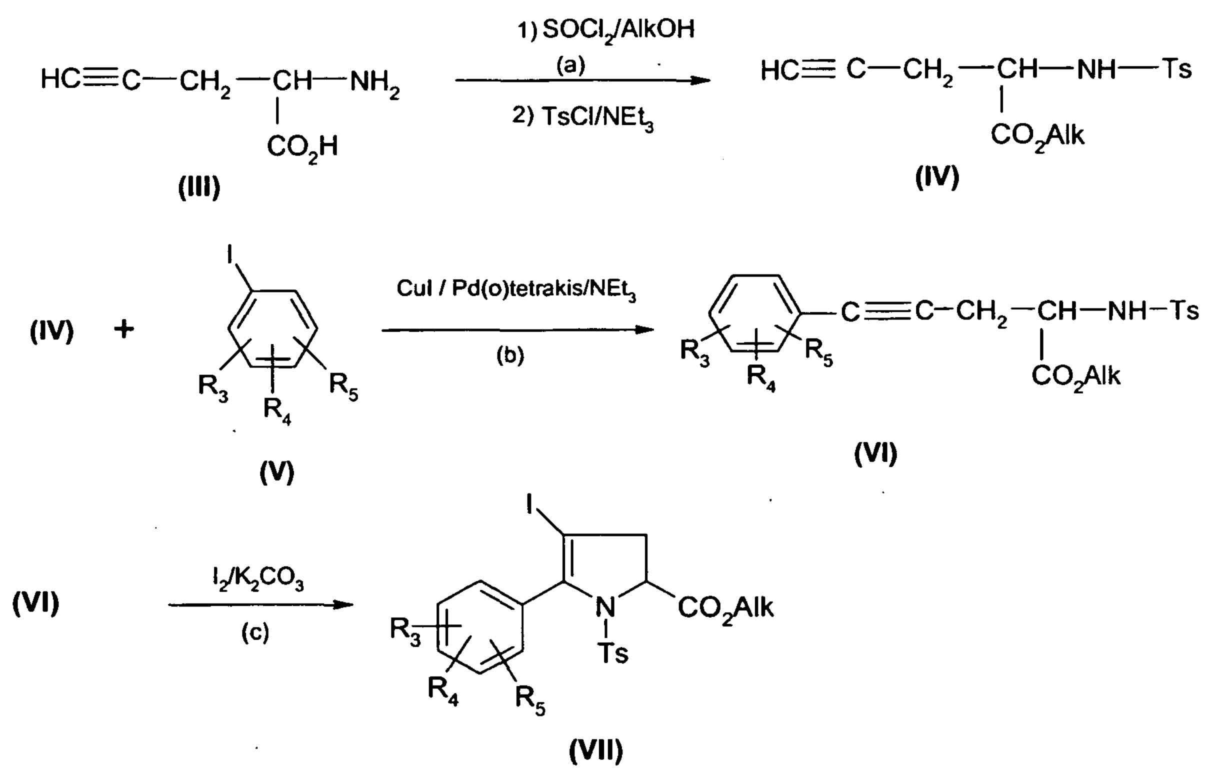


(III) (IV) (IV) + (V) (VI) (VI) (VII)

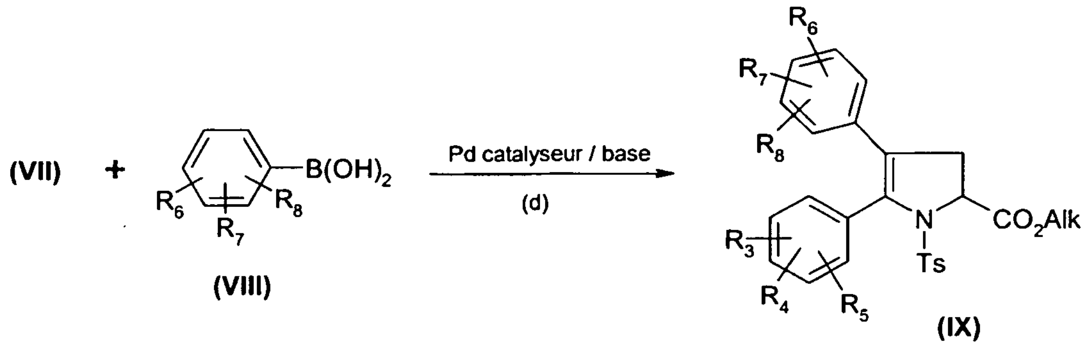


(VII) (VIII) (IX)

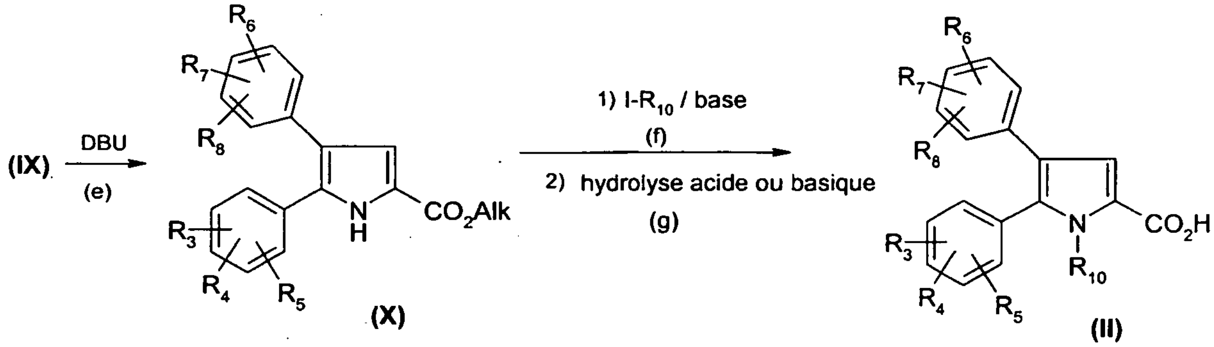


(IX) (X) (II)

La préparation du dérivé de dihydropyrrole de formule (VII) par les étapes a), b) et c) est effectuée selon J. Chem. Soc. Perkin Trans. 1, 2002, 622-628. Le groupe alkyle Alk correspond à un groupe $(C_1-C_4)$alkyle et le groupe Ts correspond à un groupe protecteur tel que par exemple le radical tosyle de formule $-SO_2-C_6H_4-CH_3$.

La substitution du noyau dihydropyrrole par un groupe phényle substitué est effectuée à l'étape d) par action d'un acide phénylboronique substitué de formule (VIII) en présence d'un catalyseur au palladium tel que par exemple le tetrakis(triphénylphosphine)Pd(0), le palladium(0) bisdibenzylidène acétone [$Pd(dba)_2$], le tris(dibenzylideneacétone) dipalladium(0), l'acétate de palladium Pd(II)[$Pd(OCOCH_3)_2$], ou le dichloro(diphénylphosphinoferrocène) Pd(II) [$PdCl_2$dppf], et en présence d'une base telle que par exemple le carbonate de sodium.

A l'étape e), la protection de l'azote par le groupe Ts est enlevée par action d'une diamine telle que par exemple la DBU (1,8-diazabicyclo[5.4.0]undécène) et simultanément le noyau dihydropyrrole est aromatisé.

Le composé de formule (X) est ensuite traité par un iodure de formule $R_{10}$-I en présence d'une base telle que par exemple le carbonate de potassium et dans le DMF pour donner à l'étape f) un composé de formule (XI).

A l'étape g), le composé de formule (XI) est hydrolyse en milieu acide ou basique. L'acide (II) ainsi formé est traité par l'amine $HNR_1R_2$ pour former le composé (I) selon l'invention.

Alternativement, les composés de formule (I) peuvent être obtenus à partir des acides de formule (IIa) et (IIa') dans lesquels $R_3$, $R_4$, $R_5$, $R_6$, $R_7$, $R_8$ et $R_{10}$ sont tels que définis pour (I) et le groupe Y-A-$R_9$ est remplacé par un groupe Z précurseur chimique du groupe Y-A-$R_9$.

Par groupe Z, on entend des groupes chimiques qui conduisent après une ou plusieurs étapes réactionnelles connues de l'homme de l'art aux groupes Y-A-$R_9$. Par exemples, le groupe précurseur Z correspond à Y-H, un atome d'halogène, un groupe Y-A-OH, Y-A-Cl, Y-A-$CO_2$Alk, Y-A-NHPg.

Ainsi, selon le schéma 2 ci-après, on part de l'acide de formule (IIa) dans laquelle $R_6$ est remplacé par le groupe Z. Cet acide est traité avec une amine de formule $HNR_1R_2$, $R_1$ et $R_2$ étant tels que définis pour (I). On obtient un amide de formule (Ia).

Puis le groupe Z du composé de formule (Ia) obtenu est transformé en une ou plusieurs étapes en groupe Y-A-$R_9$ par une des méthodes connues de l'homme de l'art pour conduire aux composés de formule (I).

**Schéma 2**

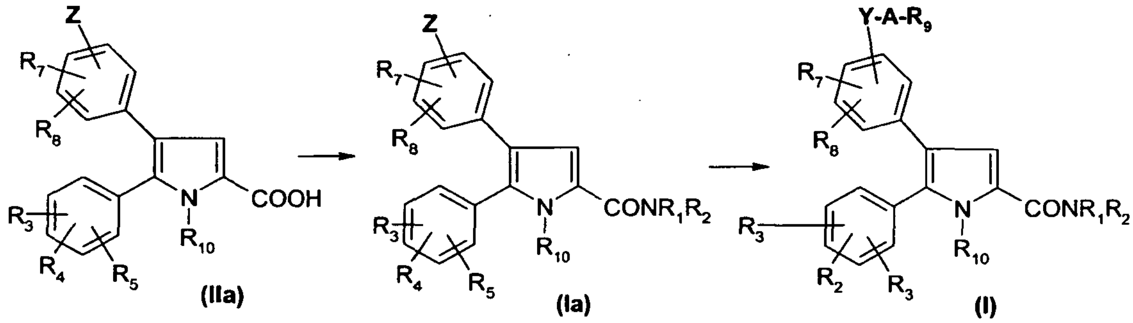

(IIa) (Ia) (I)

Ou bien selon le schéma 2' ci-après, on part de l'acide de formule (IIa') dans laquelle $R_3$ est remplacé par le groupe Z. Cet acide est traité avec une amine de formule $HNR_1R_2$, $R_1$ et $R_2$ étant tels que définis pour (I). On obtient un amide de formule (Ia').

Puis le groupe Z du composé de formule (Ia') obtenu est transformé en une ou plusieurs étapes en groupe Y-A-$R_9$ par une des méthodes connues de l'homme de l'art pour conduire aux composés de formule (I).

**Schéma 2'**

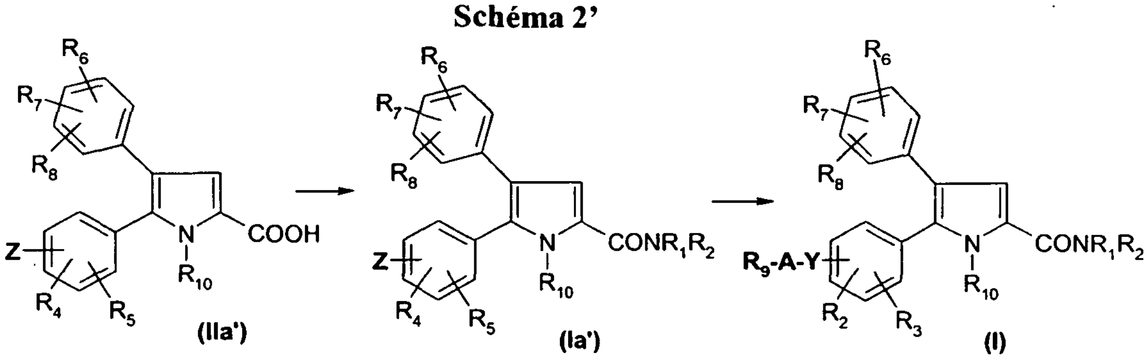

(IIa') (Ia') (I)

A titre d'exemples, les composés de formule (I) dans laquelle Y correspond à un atome d'oxygène ou de soufre et $R_9$ représente un groupe $NR_{12}R_{13}$ peuvent être obtenus à partir d'un composé de formule (Ia') dans laquelle $R_3$ est remplacé par Z, et Z correspond à YH.

Selon le schéma 3, le composé (Ia') est traité avec un dérivé dihalogéné de formule Cl-A-Br pour obtenir un composé de formule ($Ia_3$') dans laquelle Z est devenu Y-A-Cl.

Puis, le composé de formule ($Ia_3$') obtenu est traité par de l'iodure de sodium formant un composé de formule ($Ia_3$") contenant le groupement Y-A-I.

Le composé de formule ($Ia_3$') est ensuite mis en réaction avec une amine de formule $NHR_{12}R_{13}$ pour obtenir le composé de formule (I) dans laquelle Y-A-$R_9$ correspond à Y-A-$NR_{12}R_{13}$.

**Schéma 3**

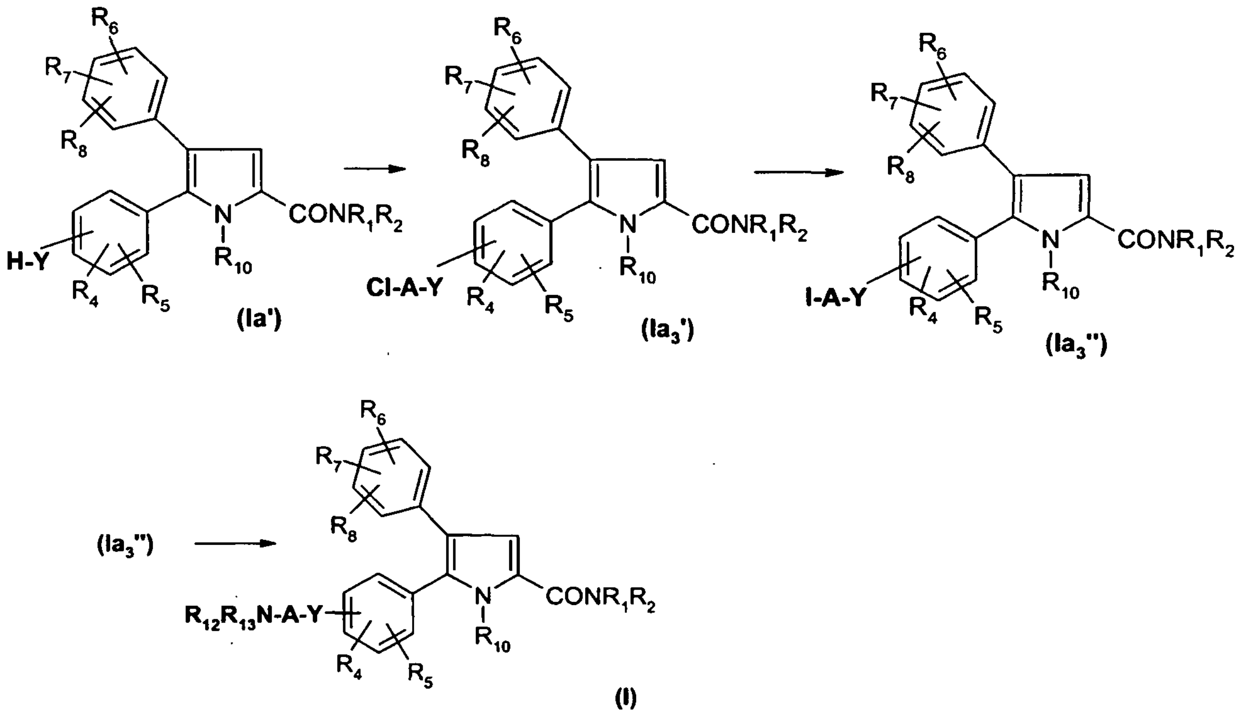

(Ia') (Ia$_3$') (Ia$_3$") (Ia$_3$") (I)

De façon analogue, les composés de formule (I) dans laquelle Y correspond à un atome d'oxygène ou de soufre et $R_9$ représente un groupe $CONR_{12}R_{13}$ peuvent être obtenus à partir de ce même composé de formule (Ia'). Selon le schéma 4, le composé de formule (Ia') est alkylé avec un dérivé halogéné de formule Br-A-$CO_2$Alk pour obtenir un composé de formule (Ia4') dans laquelle Z est devenu Y-A-$CO_2$Alk.

Ce composé de formule (Ia4') est ensuite saponifié selon les méthodes connues de l'homme de l'art pour conduire au composé de formule (Ia4") contenant le groupement Y-A-$CO_2$H.

Après activation de l'acide du composé de formule (Ia4") par le carbonyldiimidazole, la réaction avec l'ammoniac permet d'obtenir le composé de formule (I) dans laquelle Y-A-$R_9$ correspond à Y-A-$CONH_2$.

## Schéma 4

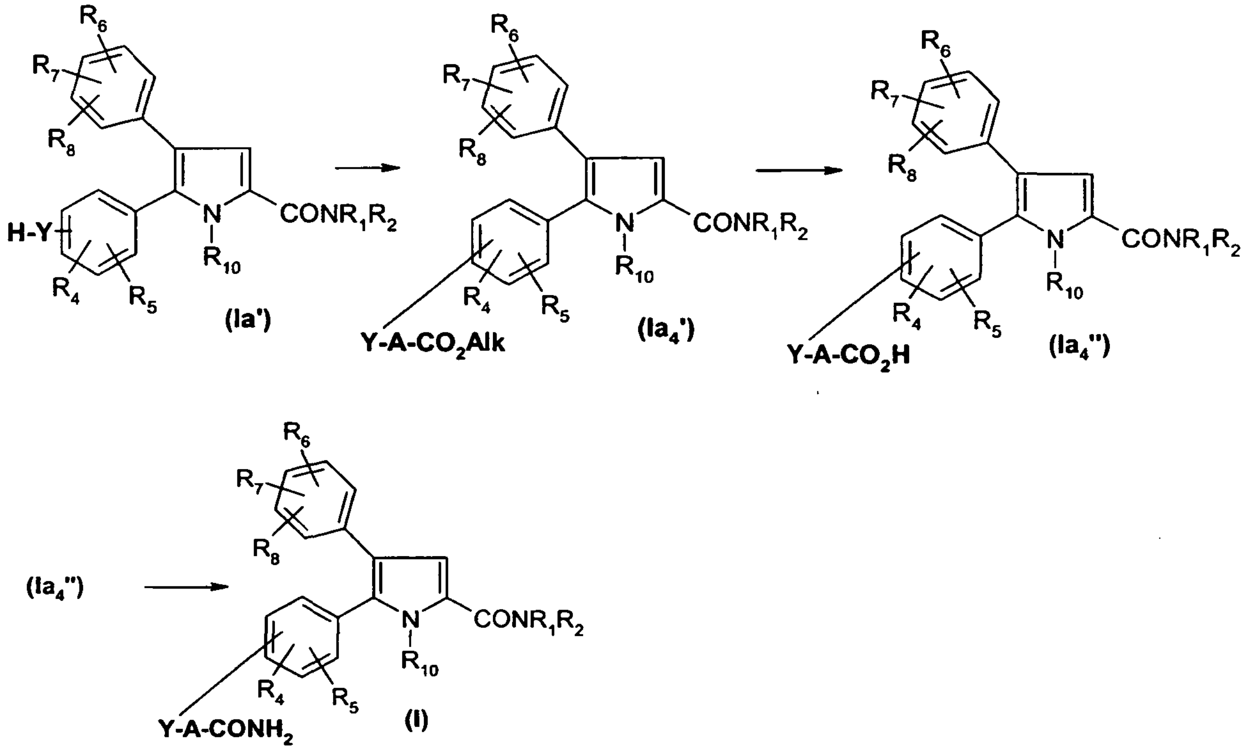

(Ia') (Ia4') (Ia4'') (Ia4'') (I)

Le composé de formule (Ia') permet également de conduire à des composés de formule (I) dans laquelle Y correspond à un atome d'oxygène ou de soufre et $R_9$ représente un groupe $NHSO_2R_{14}$.

Selon le schéma 5, le composé de formule (Ia') est dans ce cas alkylé par un dérivé halogéné de formule Br-A-NPgH pour obtenir un composé de formule (Ia5') dans laquelle Z est devenu Y-A-NPgH, Pg étant un groupe protecteur de la fonction amine, par exemple le tert-butyloxycarbonyle. D'autres exemples de groupes protecteurs Pg sont donnés dans «Protective Group in Organic Synthesis», Green et al, 4e edition, John Wiley & Sons, Inc., New York, 2007.

Le composé de formule (I) dans laquelle Y-A-$R_9$ correspond à Y-A-$NHSO_2R_{14}$ est obtenu par déprotection de l'amine suivi de la réaction avec le chlorure d'alkylsulfonyle de formule $ClSO_2R_{14}$.

## Schéma 5

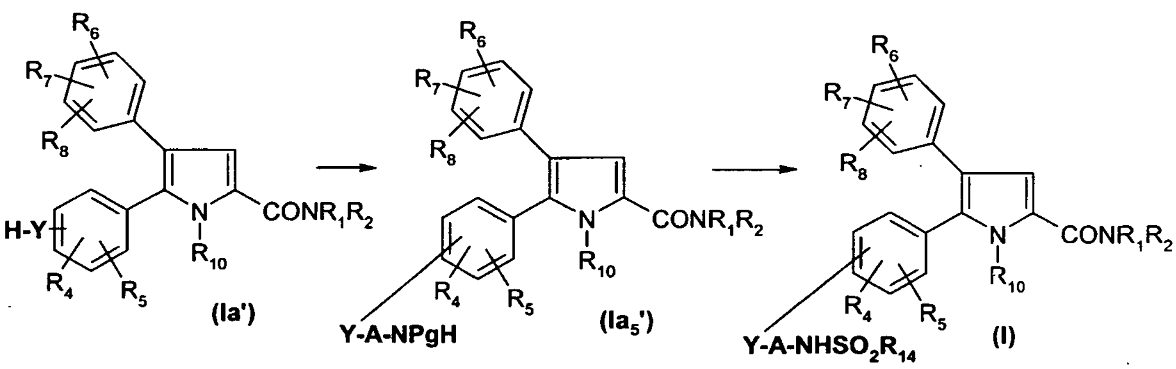

(Ia') (Ia5') (I)

A titre d'exemples concernant la préparation de composés de formule (I) dans laquelle $R_6$ correspond à Y-A-$NR_{12}R_{13}$, Y-A-$CO_2H$, Y-A-$CONH_2$ ou Y-A-$NHSO_2R_{14}$, on utilise les composés de formule (Ia) en leur appliquant les schémas réactionnels 3, 4 et 5 indiqués ci-avant.

Les acides (IIa) et (IIa') sont préparés de la même façon que les acides (II) selon le schéma réactionnel 1.

Pour préparer l'acide de formule (IIa) ou (IIa') avec Z correspondant à Y-H, l'acide de formule (IIa) ou (IIa') dans laquelle Z est remplacé par Y-$CH_3$ est mis en réaction avec $BBr_3$, dans un solvant tel que par exemple le dichlorométhane et à une température comprise entre -20°C et 0°C.

Les amines de formule $HNR_1R_2$ sont connues ou préparées par des méthodes connues, par exemple celles décrites dans J. Med. Chem., 7, 1964, 619-622, ou dans J. Org. Chem., 55, 1990, 4207-4209.

La présente invention a également pour objet les composés de formule (II) et leurs dérivés fonctionnels de formule (IIbis). Parmi ces composés de formule (II) et (IIbis), on distingue notamment ceux de formule (IIter) correspondant à la formule :

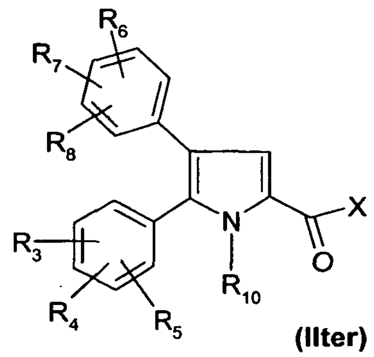

**(IIter)**

dans laquelle :

- X représente un groupe hydroxyles un atome d'halogène, $(C_1-C_4)$alcoxy ou benzyloxy ;
- et $R_3$, $R_4$, $R_5$, $R_6$, $R_7$, $R_8$ et $R_{10}$ sont tels que définis pour les composés de formule (I).

Ainsi, pour préparer un composé de formule (I) selon l'invention, on traite le composé de formule (IIter) avec notamment une amine de formule $HNR_1R_2$ dans laquelle $R_1$ et $R_2$ sont tels que définis pour les composés de formule (I).

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Dans les exemples, on utilise les abréviations suivantes :

| | |
|---|---|
| AcOEt : | acétate d'éthyle |
| $AcONH_4$ : | acétate d'ammonium |
| DCM : | dichlorométhane |
| DIPEA : | diisopropyléthylamine |
| DMF : | N,N-diméthylformamide. |
| HBTU : | hexafluorophosphate de N-[N-(diméthylamino)-1-1,2,3-triazolo[4,5-b] pyridin-1-ylméthylène]-N-méthylméthanaminium N-oxyde] |
| HOBt : | 1-hydroxybenzotriazole |
| HPLC : | chromatographie en phase liquide sous haute pression |
| MeOH : | méthanol |
| Méthanol ammoniacal : | NH3 7N en solution dans le MeOH |
| PyBOP : | hexafluorophosphate de benzotriazol-1-yloxotris-(pyrrolidino) phosphonium |
| TA : | température ambiante |
| Tf : | point de fusion |
| TFA : | acide trifluoroacétique |
| THF : | tetrahydrofuranne |
| UPLC : | chromatographie en phase liquide 'Ultra Performance' |

Les spectres de résonance magnétique nucléaire sont enregistrés à 200 MHz ou 250MHz dans le DMSO-d6. Pour l'interprétation des spectres, on utilise les abréviations suivantes : s : singulet, d : doublet, t : triplet, q : quadruplet, qui : quintuplet, m : massif, s1 : singulet large, dd : doublet de doublet.

Les composés selon l'invention sont analysés par couplage LC/UV/MS (chromatographie liquide/détection UV/spectrométrie de masse). On mesure le pic moléculaire caractéristique ($MH^+$) et le temps de rétention (tr) en minutes (min).

Les composés sont analysés par couplage HPLC-UV-MS ou bien UPLC-UV-MS (chromatographie liquide-détection UV et détection masse).

Les conditions analytiques sont les suivantes :

Conditions A (HPLC) :

On utilise une colonne: Symmetry C18 (50 x 2,1 mm ; 3,5 μm)
Eluant A : 0,005 % d'acide trifluoroacétique (TFA) dans l'eau à environ pH 3.1
Eluant B : 0,005% de TFA 0.005% dans l'acetonitrile.
Gradient :

| Temps (min) | %A | %B |
|---|---|---|
| 0 | 100 | 0 |
| 10 | 10 | 90 |
| 15 | 10 | 90 |
| 16 | 100 | 0 |
| 20 | 100 | 0 |

Température colonne: 30°C ; débit: 0,4 ml/minute.
Détection: λ = 210 nm - 220nm

Conditions B (HPLC) :

On utilise une colonne XTerra MS C18 (50 x 2,1 mm ; 3.5 μm)
Eluant A : $AcONH_4$ 10mM à environ pH 7
Eluant B : acétonitrile
Gradient :

| Temps (min) | %A | %B |
|---|---|---|
| 0 | 100 | 0 |
| 10 | 10 | 90 |
| 15 | 10 | 90 |
| 16 | 100 | 0 |
| 20 | 100 | 0 |

Température colonne: 30°C ; débit: 0,4 ml/minute.
Détection: λ= 220 nm

Conditions C (UPLC):

On utilise une colonne Acquity BEH C18 (50 x 2,1 mm ; 1,7 μm)
Eluant A: 0,005 % de TFA dans l'eau à environ pH 3,1 / acétonitrile (97/3)
Eluant B: 0,035% de TFA dans l'acétonitrile.
Gradient:

| Temps (min) | %A | %B |
|---|---|---|
| 0 | 100 | 0 |
| 2.3 | 5 | 95 |
| 2.9 | 5 | 95 |
| 3 | 100 | 0 |
| 3.5 | 100 | 0 |

Température colonne: 40°C ; débit: 1 ml/minute.

Détection: λ = 220 nm

Conditions D (UPLC):

On utilise une colonne Acquity BEH C18 (50 x 2,1 mm ; 1,7 μm)
Eluant A: 0,005 % de TFA dans l'eau à environ pH 3,1 / acétonitrile (97/3)
Eluant B: 0,035% de TFA dans l'acétonitrile.
Gradient:

| Temps (min) | %A | %B |
|---|---|---|
| 0 | 100 | 0 |
| 4.8 | 5 | 95 |
| 6 | 5 | 95 |
| 6.1 | 100 | 0 |
| 6.6 | 100 | 0 |

Température colonne: 40°C ; débit: 1 ml/minute.
Détection: λ = 220 nm

Conditions de spectrométrie de masse :

L'enregistrement des spectres de masse est effectué en mode électrospray (ESI) positif, afin d'observer les ions issus de la protonation de composés analysés ($MH^+$), ou de la formation d'adduits avec d'autres cations tels que $Na^+$, $K^+$, etc.

**PREPARATIONS**

Préparation 1 :

**4-(2,4-dichlorophényl)-5-(4-hydroxyphényl)-1-méthyl-1*H*-pyrrole-2-carboxylate d'éthyle.**

**1a)** Ester éthylique de l'acide 2-(((4-méthylphényl)sulfonyl)amino)pent-3-ynoïque.

2,5 g d'acide 2-aminobut-3-ynoique sont mis en suspension dans 45 ml d'éthyle à 0°C. On coule au goutte à goutte 1,8 ml de chlorure de thionyle à cette température puis le mélange est porté à reflux pendant 3 heures. La solution est concentrée et le résidu est séché sous pression réduite. Ce dernier est solubilisé dans 60 ml d'acétonitrile suivi de 5,4 ml de triéthylamine, puis on ajoute 4,6 g de chlorure de tosyle. Le mélange est agité à TA pendant 19 heures, puis à 50°C une heure supplémentaire. Après concentration, le brut est solubilisé dans le dichlorométhane, et la phase organique est lavée successivement par une solution aqueuse saturée de $KHSO_4$ puis de $K_2CO_3$. La phase organique est séchée sur sulfate de magnésium puis filtrée et enfin concentrée pour obtenir 5,18 g du composé attendu.
RMN$^1$H, (200MHz) : δ (ppm) : 1,13: t: 3H ; 2,35 : s : 3H ; 2,45 : m : 2H ; 3,69-4,05 : m : 3H ; 7,35 : d : 2H ; 7,65 : d : 2H ; 8,4 : d : 1H.

**1b)** Ester éthylique de l'acide 5-(4-méthoxyphényl)-2-(4-tosylsulfonylamino)pent-4-ynoïque.

40 g du composé de l'étape précédente 1a) et 31,7 g d'iodoanisole sont solubilisés dans 750 ml de DMF anhydre. 24,4 ml de triéthylamine sont ajoutés puis la solution est dégazée sous vide pendant 30 minutes. Le milieu réactionnel est placé sous azote, puis 6,3 g de tetrakis(triphénylphosphinepalladium(0) et 3,6 g de iodure de cuivre sont ajoutés. Le mélange est agité à TA sous atmosphère d'argon pendant 20 heures. Le brut réactionnel est concentré et purifié par chromatographie sur gel de silice dans le dichorométhane. On récupère 48 g du composé.
LC/MS (A) : $MH^+$ = 402, tr = 9,92 min

**1c)** Ester éthylique de l'acide 5-(4-méthoxyphényl-4-iodo-1-(4-tosylsulfonyl)-2,3-dihydro-1*H*-pyrrole-2-carboxylique.

On dissout 25,2 g du composé obtenu à l'étape précédente 1b) dans 140 ml d'acétonitrile anhydre en présence

de 26 g de carbonate de potassium à 0˚C. Sous agitation à cette température de 0˚C, on ajoute 48 g d'iode solide en plusieurs petites fractions. On laisse le mélange revenir à TA pendant 18 heures. La réaction est arrêtée avec une solution de thiosulfate de sodium jusqu'à décoloration, et la phase organique est extraite à l'acétate d'éthyle. Après séchage sur sulfate de magnésium, filtration et concentration, on obtient 30 g du composé attendu.
LC/MS (A) : $MH^+$ = 528, tr = 10,57 min.

**1d)** Ester éthylique de l'acide 4-(2,4-dichlorophényl)- 5-(4-méthoxyphényl)-1-tosylsulfonyl-2,3-dihydro-1*H*-pyrrole-2-carboxylique.

57 g du composé obtenu à l'étape précédente 1c) et 24,7 g de l'acide 2,4-dichlorophényle boronique sont solubilisés dans un mélange de 500 ml de méthanol, 1600 ml de toluène, en présence de 180 ml d'une solution de carbonate de sodium (2N). On dégaze le milieu réactionnel sous argon pendant 30 minutes puis on ajoute 17,5 g de tetrakis(triphénylphoshine)palladium(0). La solution est chauffée à 70˚C pendant 6 heures sous atmosphère inerte. Après refroidissement, le brut est filtré sur gel célite puis concentré et la phase organique est extraite à l'acétate d'éthyle. Après séchage sur sulfate de magnésium, filtration et concentration, le brut est repris dans le méthanol et le précipité obtenu est filtré. On obtient 49 g du composé attendu sous forme d'une poudre beige.
LC/MS (A) : $MH^+$ = 546, tr = 11,91 min

**1e)** Ester éthylique de l'acide 4-(2,4-dichlorophényl)-5-(4-méthoxyphényl)-1H-pyrrole-2-carboxylique.

48,6 g du composé obtenu à l'étape précédente 1d) sont solubilisés dans 500 ml de N,N-diméthylformamide anhydre. Puis 26,8 ml de DBU (1,8-diazabicyclo[5.4.0]undecene) sont ajoutés et le mélange est chauffé à 100˚C pendant 18 heures. Après concentration, le brut est solubilisé dans l'acétate d'éthyle et la phase organique est lavée successivement par une solution d'HCl 10% puis par une solution aqueuse saturée de $KHSO_4$ puis de NaCl saturée. Après séchage sur sulfate de magnésium, filtration et concentration, le résidu est trituré dans l'éther isopropylique. Ce dernier est filtré, on recueille 30,6 g du composé attendu.
LC/MS (A) : $MH^+$ = 390, tr = 11,46 min

**1f)** Ester éthylique de l'acide 4-(2,4-dichlorophényl)-5-(4-méthoxyphényl)-1-méthyl-1*H*-pyrrole-2-carboxylique.

15 g du composé obtenu à l'étape précédente 1e) sont solubilisés dans 85 ml de N,N-diméthylformamide anhydre. Puis 26,6 g de $K_2CO_3$ et 12 ml d'iodométhane sont ajoutés et le mélange est laissé sous agitation à TA pendant 4 heures et 30 minutes. Après concentration, le brut est solubilisé dans l'acétate d'éthyle et la phase organique est lavée par une solution de $KHSO_4$ 5%. Après séchage sur sulfate de magnésium, filtration et concentration, on obtient 15,5 g du composé attendu.
LC/MS (A) : $MH^+$ = 404, tr = 12,34 min

**1g)** Ester éthylique de l'acide 4-(2,4-dichlorophényl)- 5-(4-hydroxyphényl)-1-méthyl-1*H*-pyrrole-2-carboxylique.

14,8 g du composé obtenu à l'étape précédente 1f) sont solubilisés dans 750 ml de dichorométhane anhydre. La solution est placée à -50˚C puis 260 ml de $BBr_3$ sont ajoutés goutte à goutte. On laisse la température remonter jusqu'à -5˚C et l'agitation est maintenue pendant 4 heures. La réaction est arrêtée par ajout de méthanol à froid puis évaporation à TA et co-évaporation au méthanol. Le brut réactionnel est concentré et purifié par chromatographie sur gel de silice dans le dichorométhane. On récupère 11,56 g du composé attendu.
RMN$^1$H, (200MHz): δ (ppm) : 1,29 : t : 3H ; 3,72 : s : 3H ; 4,25 : q : 2H ; 6,75 : d : 2H ; 6,97-7,08 : m : 4H ; 7,25 : dd : 1H ; 7,54 : d : 1H ; 9,70 : s1 : 1H.

Préparation 2 :

**Acide 4-(2,4-dichlorophényl)-5-(4-(3-hydroxypropoxy)phényl)-1-méthyl-1*H*-pyrrole-2-carboxylique.**

**2a)** Ester éthylique de l'acide 4-(2,4-dichlorophényl)-5-(4-(3-hydroxypropoxy) phényl)-1-méthyl-1*H*-pyrrole-2-carboxylique.

0,70 g du composé obtenu à l'étape 1g) de la préparation 1 sont solubilisés dans 90 ml d'acétone anhydre puis 0,50 g de $K_2CO_3$ suivis de 0,37 g de 3-bromo-1-propanol. Le mélange est porté à reflux pendant 20 heures. Le brut est filtré et le filtrat est concentré. Le brut réactionnel est purifié par chromatographie sur gel de silice dans un gradient cyclohexane/acétate d'éthyle (9/1 à 6/4 en 45 min.). On obtient 0,70 g du composé attendu.

LC/MS (C) : $MH^+$ = 448, tr = 2,02 min

**2b)** Acide 4-(2,4-dichlorophényl)- 5-(4-(3-hydroxypropoxy)phényl)-1-méthyl-1*H*-pyrrole-2-carboxylique.

0,70 g de l'ester obtenu à l'étape précédente 2a) sont solubilisés dans 15 ml de méthanol. 5 ml de $H_2O$, puis 0,44 g de KOH sont ajoutés et le mélange est porté à reflux pendant 4 heures. Une fois refroidi, le milieu est acidifié par une solution de HCl aqueuse (10%) puis le produit est extrait à l'AcOEt. La phase organique est séchée sur $MgSO_4$ puis filtrée et concentrée. Le brut réactionnel est purifié par chromatographie sur gel de silice dans un gradient dichlorométhane/méthanol (100/0 à 96/4 en 20 min.). On obtient 0,54 g du composé attendu.
LC/MS (B): $MH^+$ = 420, tr = 7.01 min
RMN$^1$H, (200MHz) : δ (ppm) : 1,84 : qui : 2H ; 3,54 : t : 2H ; 3,73 : s : 3H ; 4,01: t : 2H ; 4,52 : s1 ; 1H ; 6,88-6,98 : m : 3H ; 7,03-7,14 : m : 3H ; 7,26 : dd : 1H ; 7,55 : d : 1H ; 12,36 : s1 : 1H.

**EXEMPLES**

**Exemple 1 : 1[4-(2,4-dichlorophényl)-5-(4-(3-hydroxypropoxy)phényl)-1-méthyl-1*H*-pyrrole-2-carbonyl]-4-phénylpipéridine-4-carboxamide.**

0,30 g du composé obtenu à l'étape 2b) de la préparation 2, sont mis en solution dans 15 ml de DMF en présence de 0,28 g (0,37 ml) de DIPEA. Puis 0,34 g. du chlorhydrate de la 4-phénylpipéridine-4-carboxamide sont ajoutés, suivi de l'ajout de 0,41 g de HBTU et 0,05 g de HOBt. Le mélange est laissé agité pendant 2 heures 30 minutes à TA. Une fois concentré, le brut est chromatographié sur gel de silice dans un gradient dichlorométhane/méthanol (99/1 à 93/7 en 40 min.). Le résidu obtenu, de couleur orange, est ensuite cristallisé dans l'AcOEt pour obtenir 0,37 g d'un produit blanc.
RMN$^1$H, (200MHz) : δ (ppm) : 1,76-1,92 : m : 4H ; 2,52-2,60 : m : 2H ; 3,21-3,42 : m : 2H ; 3,47-3,59 : m : 5H ; 4,01-4,08 : m, 2H ; 4,11-4,25 : m : 2H ; 4,53 : t : 1H ; 6,52 : s : 1H ; 6,90 : d : 2H ; 7,05-7,13 : m : 4H ; 7,18-7,46 : m : 7H ; 7,54: d : 1H.

**Exemple 2:1'[5-(4-(3-aminopropoxy)phényl)-4-(2,4-dichlorophényl)-1-méthyl-1*H*-pyrrole-2-carbonyl]-[1,4']bipipéridinyl-4'-carboxamide.**

**2A)** Acide 4-(2,4-dichlorophényl)-5-(4-hydroxyphényl)-1-méthyl-1*H*-pyrrole-2-carboxylique.

8,9 g de l'ester obtenu à l'étape 1g) de la préparation 1 sont solubilisés dans 220 ml de MeOH. 55 ml de $H_2O$, puis 7,7 g de KOH sont ajoutés et le mélange est porté à reflux pendant 4 heures. Une fois refroidi, le milieu est acidifié par une solution de HCl aqueuse (10%) puis le produit est extrait à l'AcOEt. La phase organique est séchée sur $MgSO_4$ puis filtrée et concentrée. On obtient 9,85 g du composé attendu.
LC/MS (A): $MH^+$ = 362, tr = 8,91 min

**2B)** 1'[4-(2,4-dichlorophényl)-5-(4-hydroxyphényl)-1-méthyl-1*H*-pyrrole-2-carbonyl]- [1,4']bipipéridinyl-4'-carboxamide.

9,3 g de l'acide carboxylique obtenu à l'étape précédente 2A) sont mis en solution dans 500 ml de DMF en présence de 10 g (13.5 ml) de DIPEA, puis 10,9 g de [1,4']bipipéridinyl-4'-carboxamide sont ajoutés, suivi de l'ajout de 14,6 g de HBTU et 1,7 g de HOBt. Le mélange est laissé agité 18 heures à TA.
Une fois concentré, le brut est chromatographié sur gel de silice dans un gradient dichlorométhane/méthanol (100/0 à 90/10). Le résidu obtenu est ensuite précipité dans le dichlorométhane pour obtenir 8,88 g du produit attendu.
LC/MS (A) : $MH^+$ = 555, tr = 6,69 min

**2C)** Ester tert-butylique de l'acide (3-{4-[5-(4'-Carbamoyl-[1,4']bipipéridinyl-1'-carbonyl)-3-(2,4-dichloro-phényl)-1-méthyl-1*H*-pyrrol-2-yl]-phénoxy}-propyl)-carbamique

0,44 g du composé obtenu à l'étape précédente 2B), 0,24 g d'ester *tert*-butylique de l'acide 3-bromopropylcarbamique, 0,39 g de $CsCO_3$ ainsi que 0,03 g de NaI sont mis en suspension dans 13 ml d'acétonitrile. Le mélange est agité sous atmosphère inerte à 70°C pendant 16 heures. Le brut est filtré et le filtrat est concentré. Le brut réactionnel est purifié par chromatographie sur gel de silice dans un gradient dichlorométhane/méthanol (100/0 à 98/2 en 20 min.). On obtient 0,50 g du composé attendu.
LC/MS (A) : $MH^+$ = 712, tr = 7,95 min

**2D)** 1'[5-(4-(3-aminopropoxy)phényl)-4-(2,4-dichlorophényl)-1-méthyl-1H-pyrrole-2-carbonyl]-[1,4']bipipéridinyl-4'-carboxamide.

0,48 g du composé obtenu à l'étape précédente 2C) sont solubilisés dans 3 ml de dichlorométhane puis 0,77 g (0,5 ml) de TFA sont ajoutés. Le mélange est laissé sous agitation à TA pendant 18 heures. Le brut est concentré et co-évaporé au dichlorométhane. Le brut réactionnel est purifié par chromatographie sur gel de silice dans un gradient dichlorométhane/méthanol (100/0 à 80/20). Le résidu est alors repris dans le minimum de dichlorométhane puis l'éther chlorhydrique est ajouté. Le milieu est alors dilué par de l'éther éthylique et laissé sous agitation pendant 1 heure à TA. Après filtration et lavages à l'éther éthylique, on obtient 0,40 g du composé attendu.
RMN$^1$H, (200MHz) : δ (ppm) : 1,22-1,41 : m : 4H ; 1,52-2,18 : m : 8H ; 2,61-2,93 : m : 6H ; 3,41-3,51 : m : 5H ; 4,00 : t : 2H ; 4,36-4,49 : m : 2H ; 6,51 : s : 1H ; 6,88 : d : 2H ; 7,05-7,13 : m : 4H ; 7,05-7,10 : m : 3H ; 7,26 : dd : 1H ; 7,51: d : 1H ; 7,87-7,95 : s1 : 3H ; 8,15-8,30 : 2sl : 2H.

**Exemple 3 : 1'[4-(2,4-dichlorophényl)-5-(4-(3-méthanesulfonylamino propoxy)phényl)-1-méthyl-1*H*-pyrrole-2-carbonyl]-[1,4']bipipéridinyl-4' -carboxamide.**

0,39 g du composé obtenu à l'étape **2D)** de l'exemple 2, sont solubilisés dans 4 ml de DCM, 0,129 g (0,18 ml) de triéthylamine puis lentement 0,08 g de chlorure de méthanesulfonyle. Le mélange est laissé à TA et sous agitation pendant 20 heures. Après concentration, le brut est solubilisé dans l'acétate d'éthyle et la phase organique est lavée successivement par une solution d'HCl 10% puis par une solution aqueuse saturée NaCl. Après séchage sur sulfate de magnésium, filtration et concentration, le résidu est purifié par chromatographie sur gel de silice dans un gradient dichlorométhane/méthanol (100/0 à 95/5). Ce dernier est filtré, on recueille 0,32 g du composé attendu.
RMN$^1$H, (200MHz) : δ (ppm) : 1,28-1,54 : m : 6H ; 1,66-1,99 : m : 6H ; 2,39-2,50 : m : 2H ; 2,89: s : 3H ; 3,03-3,15 : m : 2H ; 3,37-3,53 : m : 5H ; 3,84-4,05 : m : 4H ; 6,50 : s : 1H ; 6,90 : d : 2H ; 7,02-7,14 : m : 6H ; 7,26 : dd : 1H ; 7,54: d : 1H.

**Exemple 4 : 1'-[5-(4-Carbamoylméthoxyphényl)-4-(2,4-dichlorophényl)-1-méthyl-1*H*-pyrrole-2-carbonyl]-[1,4'] bipipéridinyl-4'-carboxamide.**

**4A)** Ester tert-butylique de l'acide {4-[5-(4'-Carbamoyl-[1,4']bipipéridinyl-1'-carbonyl)-3-(2,4-dichloro-phényl)-1-méthyl-1*H*-pyrrol-2-yl]-phénoxy}-acétique.

0,60 g du composé obtenu à l'étape 2B) de l'exemple 2 sont solubilisés dans 20 ml de DMF puis 0,22 g de $K_2CO_3$ suivis de 0,42 g de bromoacétate de *tert*-butyle. Le mélange est porté à reflux pendant 19 heures. Le brut est filtré et le filtrat est concentré. Le brut réactionnel est purifié par chromatographie sur gel de silice dans un gradient cyclohexane/acétate d'éthyle (20/80 à 0/100 en 20 min.). On obtient 0,72 g du composé attendu.
LC/MS (A): $MH^+$ = 669, tr = 8,01 min

**4B)** Acide {4-[5-(4'-Carbamoyl-[1,4']bipipéridinyl-1'-carbonyl)-3-(2,4-dichlorophényl)-1-méthyl-1*H*-pyrrol-2-yl]-phénoxy}-acétique.

0,72 g du composé obtenu à l'étape précédente **4A)** sont solubilisés dans 15 ml de dichlorométhane puis 8 g (5,4 ml) de TFA sont ajoutés. Le milieu réactionnel est laissé à TA sous agitation pendant 6 heures. Le mélange est concentré et co-évaporé au dichlorométhane. Le brut est repris dans l'acétate d'éthyle puis la phase organique est lavée successivement par une solution de $NaHCO_3$ saturée, par une solution de $KHSO_4/K_2SO_4$ 5% puis par une solution de NaCl saturée. Après séchage sur sulfate de magnésium, filtration et concentration, on obtient 0,46 g du composé attendu.
LC/MS (A) : $MH^+$ = 613, tr = 6,73 min

**4C)** 1'-[5-(4-Carbamoylméthoxyphényl)-4-(2,4-dichlorophényl)-1-méthyl-1*H*-pyrrole-2-carbonyl]-[1,4']bipipéridinyl-4'-carboxamide.

0,46 g du composé de l'étape précédente **4B)** sont solubilisés dans 15 ml de DMF et 0,13 g de carbonyle diimidazole sont ajoutés. L'agitation est maintenue pendant 1 heure sous atmosphère d'azote, puis l'ammoniac est mis à buller pendant 30 min. Le mélange est alors bouché et laissé sous agitation à TA pendant 48 heures. Après concentration, le brut est solubilisé dans le dichlorométhane et la phase organique est lavée par une solution aqueuse saturée de NaCl. Après séchage sur sulfate de magnésium, filtration et concentration, le résidu est purifié par chromatographie sur gel de silice dans un gradient dichlorométhane/méthanol ammoniacal (100/0 à 96/4). On obtient 0,39 g du composé attendu.
RMN$^1$H, (250MHz) : δ (ppm) : 1,23-1,50 : m : 6H ; 1,51-1,91 : m : 4H ; 2,34-2,42 : m : 2H ; 3,31-3,49 : m : 5H ; 3,79-3,81 : m, 2H ; 4,38 : s : 2H ; 6,48 : s : 1H ; 6,89 : d : 2H ; 6,99-7,11 : m : 5H ; 7,21 : dd : 1H ; 7,37 : s1 : 1H ; 7,50 : d : 1H.

**Exemple 5 : 1'[4-(2,4-dichlorophényl)-5-(4-(3-méthylaminopropoxy) phényl)-1-méthyl-1*H*-pyrrole-2-carbonyl]-[1,4']bipipéridinyl-4'-carboxamide.**

**5A)** 1'[5-(4-(3-chloropropoxy)phényl)-4-(2,4-dichlorophényl)-1-méthyl-1*H*-pyrrole-2-carbonyl]-[1,4']bipipéridinyl-4'-carboxamide.

1 g du composé obtenu à l'étape **2B)** de l'exemple 2 est solubilisé dans 30 ml de DMF anhydre, puis 0,75 g de $K_2CO_3$ suivi de 0,85 g (0,53 ml) de 1-bromo-3-chloropropane sont ajoutés. Le mélange est porté à reflux pendant 20 heures. Le brut est filtré et le filtrat est concentré. Le brut réactionnel est purifié par chromatographie sur gel de silice dans un gradient dichlorométhane/méthanol (100/0 à 95/5). On obtient 1,1 g du composé attendu.
LC/MS (A) : $MH^+$ = 633, tr = 8,10 min

**5B)** 1'[4-(2,4-dichlorophényl)-5-(4-(3-iodopropoxy)phényl)-1-méthyl-1*H*-pyrrole-2-carbonyl]-[1,4']bipipéridinyl-4'-carboxamide.

1,1 g du composé de l'étape précédente **5A)** sont solubilisés dans 12 ml d'acétonitrile puis 1,9 g de NaI sont ajoutés. Le mélange est laissé sous agitation à 75˚C pendant 18 heures. Après concentration, le brut est solubilisé dans le dichlorométhane et la phase organique est lavée par une solution aqueuse saturée de NaCl. Après séchage sur sulfate de magnésium, filtration et concentration, on obtient 1,16 g du composé attendu.
LC/MS (A) : $MH^+$ = 723, tr = 8,42 min

**5C)** 1'[4-(2,4-dichlorophényl)- 5-(4-(3-méthylaminopropoxy)phényl)-1-méthyl-1*H*-pyrrole-2-carbonyl]- [1,4']bipipéridinyl-4'-carboxamide.

0,20 g du composé obtenu à l'étape précédente **5B)** sont solubilisés dans 10 ml de méthylamine (en solution 2M dans le THF), puis la solution est laissée bouchée et sous agitation à 105˚C pendant 20 heures. Après concentration, le brut est solubilisé dans le dichlorométhane et la phase organique est lavée par une solution aqueuse saturée de NaCl. Après séchage sur sulfate de magnésium, filtration et concentration, le résidu est purifié par chromatographie sur gel de silice dans un gradient dichlorométhane/méthanol ammoniacal (95/5 à 70/30). On obtient 0,25 g du composé attendu.
RMN$^1$H, (200MHz) : $\delta$ (ppm) : 1,34-1,55 : m : 6H ; 1,68-1,97 : m : 6H ; 2,26 : s : 3H ; 2,41-2,48 : m : 4H ; 2,57 : t, 2H ; 3,37-3,53 : m : 5H ; 3,84-4,04 : m : 4H ; 6,49 : s : 1H ; 6,89 : d : 2H ; 7,02-7,13 : m : 5H ; 7,26 : dd : 1H ; 7,54: d : 1H.

**Exemple 6 : 1[4-(2,4-dichlorophényl)-5-[4-(3-hydroxypropoxy)-phényl]-1-méthyl-1H-pyrrole-2-carbonyl]-4-(3-fluoro-benzylamino)-pipéridine-4-carboxamide**

Ce composé est préparé selon un mode opératoire similaire à celui de l'exemple 1, en partant de 0,18 g du composé obtenu à l'étape 2B) de la préparation 2. Le réactif utilisé dans ce cas est le dichlorhydrate de la 4-(3-fluoro-benzylamino)-pipéridine-4-carboxamide.

La purification est réalisée par chromatographie sur gel de silice dans un gradient dichlorométhane/méthanol (99/1 à 95/5 en 40 min.). On obtient 0,11 g du produit attendu.
RMN$^1$H, (250MHz) : $\delta$ (ppm) : 1,68-1,95 : m : 6H ; 2,56-2,60 : m : 1H ; 3,52-3,68 : m : 6H ; 3,54 : s : 3H ; 3,89-3,97 : m : 2H ; 4,03 : t : 2H ; 4,54 : t : 1H ; 6,51 : s : 1H ; 6,92 : d : 2H ; 7,03-7,14 : m : 5H ; 7,21-7,28 : m : 3H ; 7,33-7,41 m : 2H; 7,56 : s : 1H.

**Exemple 7 : 1-[5-(4-carbamoylméthoxy)-phényl-4-(2,4-dichlorophényl)-1-méthyl-1H-pyrrole-2-carbonyl]-4-phénylpipéridine-4-carboxamide.**

**7A)** 1'[4-(2,4-dichlorophényl)-5-(4-hydroxyphényl)-1-méthyl-1*H*-pyrrole-2-carbonyl] -4-phénylpipéridine-4-carboxamide.

Le composé est préparé selon un mode opératoire similaire à celui de l'étape 2B) de l'exemple 2, en partant de 0,4 g du composé obtenu à l'étape 2A). Le réactif utilisé est le chlorhydrate de la 4-phénylpipéridine-4-carboxamide.

La purification est réalisée par chromatographie sur gel de silice dans un gradient dichlorométhane/méthanol ammoniacal (100/0 à 90/10). On obtient 0,26 g du produit attendu.
RMN$^1$H, (200MHz) : $\delta$ (ppm) : 1,74-1,82 : m : 2H ; 2,50-2.60 : m : 2H ; 3,18-3,37 : m : 2H ; 3,45 : s : 3H ; 4.12-4,27 : m : 2H ; 6,51 : s : 1H ; 6,71 : d : 2H ; 6,94-7,12 : m : 4H ; 7,18-7,47 : m : 7H; 7,53: d: 1H ; 9.8 : s1 : 1H.

7B) 1-[5-(4-carbamoylméthoxy)-phényl-4-(2,4-dichlorophényl)-1-méthyl-1H-pyrrole-2-carbonyl]-4-phénylpipéridine-4-carboxamide.

0,25 g du composé obtenu à l'étape précédente **7A),** 0,065 g de 2-chloroacétamide, 0,13 g de $K_2CO_3$ ainsi que 0,03 g de NaI sont mis en suspension dans 9 ml d'acétone. Le mélange est agité sous atmosphère inerte à 50°C pendant 10 heures. Après ajout de 0,043 g de 2-chloroacétamide, le milieu réactionnel est laissé sous agitation à 50°C pendant 60 heures. Ensuite, 0,043 g de 2-chloroacétamide est ajouté au milieu réactionnel.

Après filtration et concentration, le brut réactionnel est purifié par chromatographie sur gel de silice dans un mélange isocratique heptane/AcOEt/méthanol ammoniacal (40/60/10). On obtient 0,15 g du composé attendu.
RMN$^1$H, (250MHz) : δ (ppm) : 1,79-1,89 : m : 2H ; 2,50-2,57 : m : 2H ; 3,28-3,30 : m : 2H ; 3,52 : s : 3H ; 4,16-4,22 : m, 2H ; 4,43 : s : 2H ; 6,53 : s : 1H ; 6,94 : d : 2H ; 7,09-7,15 : m : 4H ; 7,22-7,28 : m : 3H ; 7,33-7,44 : m : 5H; 7,52-7,56: m: 2H.

**Exemple 8 : 1-[4-(2-chlorophényl)-5-[4-(3-hydroxypropoxy)-phényl]-1-méthyl-1H-pyrrole-2-carbonyl]-4-(4-fluoro-benzylamino)-pipéridine-4-carboxamide.**

Le composé est préparé selon un mode opératoire identique à celui de l'exemple 1, à partir de 0,30 g du composé obtenu à l'étape 2B) de la préparation 2. Le réactif utilisé dans ce cas est le dichlorhydrate de 4-(4-fluoro-benzylamino)-pipéridine-4-carboxamide.

La purification est réalisée par chromatographie sur gel de silice dans un gradient dichlorométhane/méthanol (99/1 à 96/4 en 20 min.). On obtient 0,27g du produit attendu.
RMN$^1$H, (250MHz) : δ (ppm) : 1,64-1,94 : m : 6H ; 2,49-2.50 : m : 1H ; 3,49-3,70 : m : 6H ; 3,52 : s : 3H ; 3,90-4,05 : m : 4H ; 4,55 : t : 1H ; 6,49 : s : 1H ; 6,90 : d : 2H ; 7,06-7,24 : m : 8H ; 7,39-7,48 : m : 4H.

Les tableaux 1 et 2 indiquent les structures chimiques de quelques composés selon l'invention ainsi que leurs propriétés physiques (analyse par couplage LC/UV/MS : chromatographie liquide/détection UV/spectrométrie de masse). Les composés listés sont préparés selon les procédés de préparations décrits ci-avant et notamment en suivant des modes opératoires similaires à ceux décrits aux exemples 1 à 8.

**TABLEAU 1**

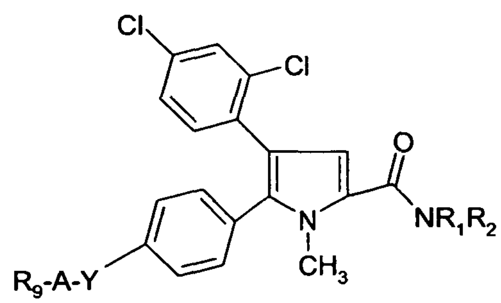

| ***Composés*** | ***-Y-A-R$_9$*** | ***-CONR$_1$R$_2$*** | ***Tf*** | ***LC/MS* MH$^+$; tr;** (conditions) |
|---|---|---|---|---|
| 1 (Ex 2) | -O-$(CH_2)_3$-$NH_2$ | 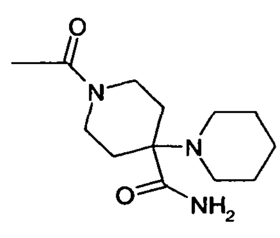 | 245°C | MH$^+$= 612 tr=5,71 (A) |

(suite)

| ***Composés*** | ***-Y-A-$R_9$*** | ***-CON$R_1R_2$*** | ***Tf*** | ***LC/MS* $MH^+$; tr;** (conditions) |
|---|---|---|---|---|
| 2 | -O-$(CH_2)_3$-$CONH_2$ | 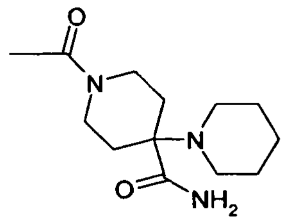 | 163°C | $MH^{*}$= 604 tr=6,69 (A) |
| 3 (Ex 3) | -O-$(CH_2)_3$-$NHSO_2CH_3$ | 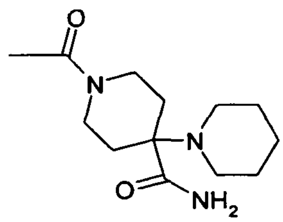 | 209°C | $MH^+$= 690 tr=7,08 (A) |
| 4 (Ex 4) | -O-$CH_2$-$CONH_2$ | 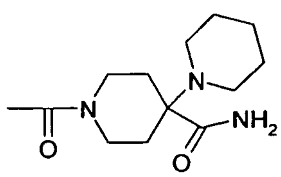 | 225°C | $MH^+$= 612 tr=6,50 (A) |
| 5 | -O-$(CH_2)_3$-$NHCOCH_2OH$ | 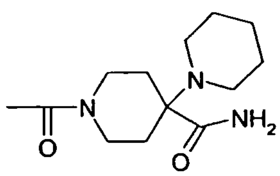 | | $MH^+$= 670 tr=6,63 (A) |
| 6 | -O-$(CH_2)_3$-OH | 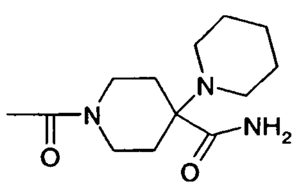 | 136°C | $MH^+$= 613 tr=22,3 (B) |
| 7 | -O-$(CH_2)_3$-$NSO_2CH_3$ | 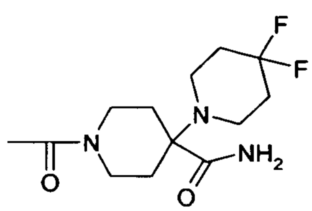 | 224°C | $MH^+$= 726 tr=9,38 (A) |

(suite)

| *Composés* | *-Y-A-R$_9$* | *-CONR$_1$R$_2$* | *Tf* | *LC/MS* **MH$^+$; tr;** (conditions) |
|---|---|---|---|---|
| 8 (Ex 5) | -O-$(CH_2)_3$-$NHCH_3$ | 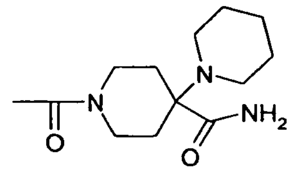 | 186°C | MH$^+$ = 626 tr=5,78 (A) |
| 9 | 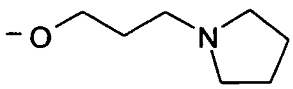 | 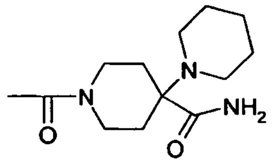 | 233°C | MH$^+$ = 666 tr=5,94 (A) |
| 10 (Ex 1) | -O-$(CH_2)_3$-OH | 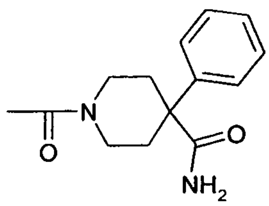 | 121°C | MH$^+$ = 605 tr=2,86 (C) |
| 11 (Ex 6) | -O-$(CH_2)_3$-OH | 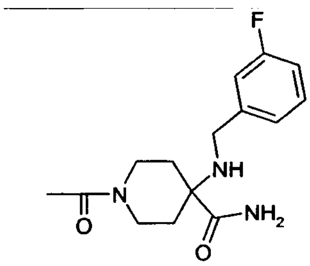 | | MH$^+$ = 653 tr=2,34 (C) |
| 12 | -O-$(CH_2)_2$-OH | 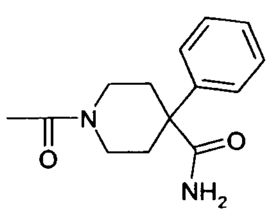 | 205°C | |

(suite)

| *Composés* | *-Y-A-R*$_9$ | *-CONR*$_1$*R*$_2$ | *Tf* | *LC/MS* MH$^+$; tr; (conditions) |
|---|---|---|---|---|
| 13 | $-O-(CH_2)_2-OH$ | 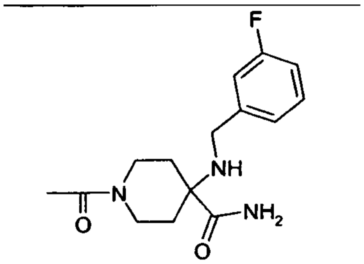 | 149°C | |
| 14 | $-O-(CH_2)_3-CONH_2$ | 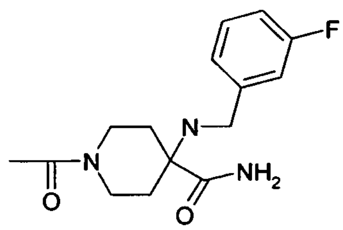 | 130°C | MH$^+$ = 680 Tr = 8.84 (B) |
| 15 | $-O-(CH_2)_3-CONH_2$ | 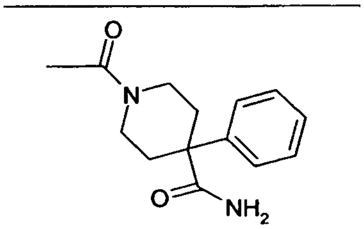 | 143°C | MH$^+$ = 633 tr=1.50 (C) |
| 16 (Ex 7) | $-O-CH_2-CONH_2$ | 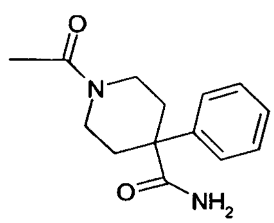 | 141°C | MH$^+$ = 605 tr= 2.53 (D) |
| 17 | $-O-CH_2-CONH_2$ | 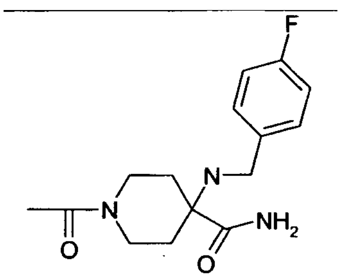 | | MH$^+$=652 tr=1.16 (C) |
| 18 | $-O-(CH_2)_2-OH$ | 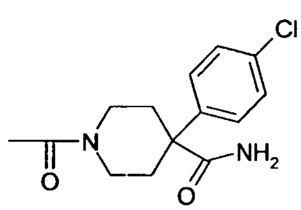 | 161°C | MH$^+$ = 626 tr = 2.96 (D) |

(suite)

| *Composés* | *-Y-A-$R_9$* | *-$CONR_1R_2$* | *Tf* | *LC/MS* **$MH^+$; tr;** (conditions) |
|---|---|---|---|---|
| 19 | -O-$(CH_2)_2$-OH | 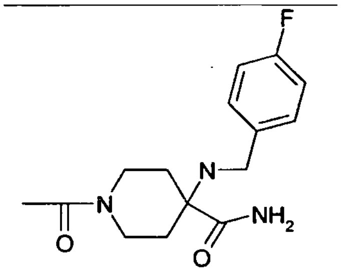 | 149°C | $MH^+$ = 639 tr=8.99 (B) |

**TABLEAU 2**

| 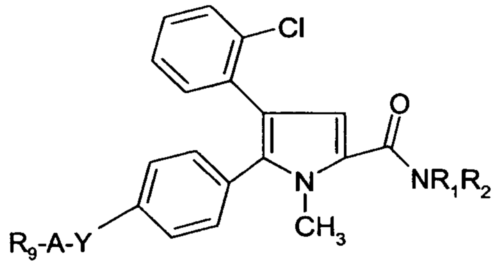 | | | | |
|---|---|---|---|---|
| 20 | -O-$(CH_2)_2$-OH | 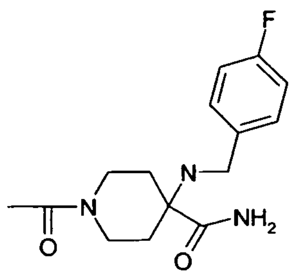 | 195°C | $MH^+$ = 605 tr= 1.91 (D) |
| 21 | -O-$(CH_2)_2$-OH | 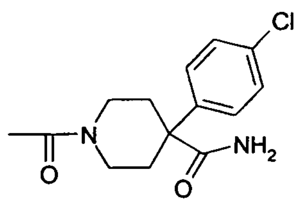 | 188°C | $MH^+$ = 592 tr= 2.65 (D) |
| 22 | -O-$(CH_2)_2$-OH | 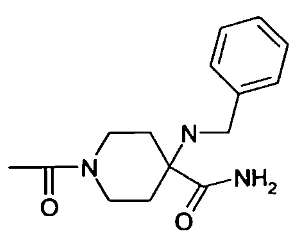 | 169°C | $MH^+$ = 587 tr= 1.85 (D) |

(suite)

| | | | | |
|---|---|---|---|---|
| 23 | -O-$(CH_2)_2$-OH | 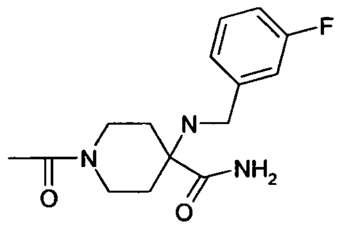 | 123°C | $MH^+$ = 605 tr= 1.93 (D) |
| 24 | -O-$(CH_2)_3$-OH | 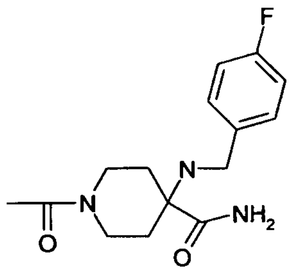 | 220°C | $MH^+$ = 619 tr= 2.03 (D) |
| 25 (Ex 8) | -O-$(CH_2)_3$-OH | 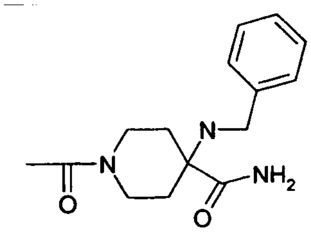 | 171°C | $MH^+$ = 601 tr=1.99 (D) |
| 26 | -O-$(CH_2)_3$-OH | 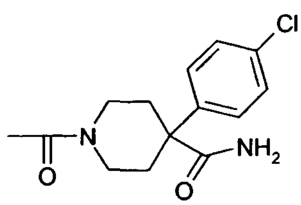 | 176°C | $MH^+$ = 606 tr= 2,77 (D) |
| 27 | -O-$(CH_2)_3$-OH | 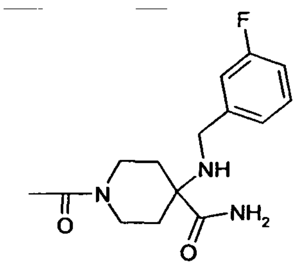 | 237°C | $MH^+$ = 619 tr= 2,04 (D) |
| 28 | -O-$(CH_2)_3$-OH | 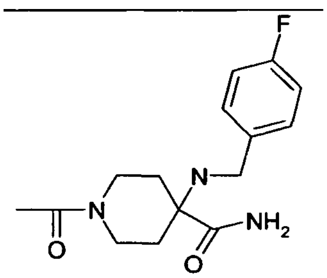 | 190°C | $MH^+$ = 618 tr= 1.78 (D) |

(suite)

| | | | | |
|---|---|---|---|---|
| 29 | -O-$(CH_2)_3$-OH | 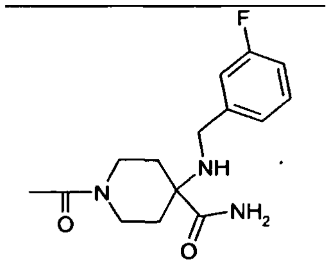 | 175°C | $MH^+$ = 618 tr= 1.80 (D) |

Les composés de formule (I) possèdent une très bonne affinité *in vitro* ($IC_{50} \leq 5.10^{-7}$ M) pour les récepteurs aux cannabinoïdes $CB_1$, dans les conditions expérimentales décrites par M. Rinaldi-Carmona et al. (FEBS Letters, 1994, 350, 240-244).

La nature antagoniste des composés de formule (I) a été démontrée *in vitro* par les résultats obtenus dans les modèles de l'inhibition de l'adénylate-cyclase comme décrits dans M. Bouaboula et al., J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

La faible pénétration des composés de formule (I) au niveau de la barrière hémato-encéphalique (BHE) a été évaluée *in vivo* par :

- **Mesure (1)** : la quantification des composés de formule (I) (inchangé) dans des échantillons de cerveau de souris après une administration par voie intraveineuse ou orale, à l'aide de technique analytique (LC-MS/MS).
  Le ratio $\frac{\text{quantité présente dans le cerveau}}{\text{quantité présente dans le plasma}}$ inférieur à 0,2 traduit une faible pénétration du composé au niveau du cerveau.
- **Mesure (2)** : la mesure de l'interaction des composés de formule (I) avec les récepteurs CB1 présents dans le cerveau chez la souris à l'aide d'un test de binding *ex vivo* du [3H]-CP55940 (agoniste CB1) après une administration par voie intraveineuse (10 mg/kg) comme décrit dans M. Rinaldi-Carmona et al., FEBS Letters, 1994, 350, 240-244 et M. Rinaldi-Carmona et al., Life Sciences, 1995, 56, 1941-1947, M. Rinaldi-Carmona et al., J. Pharmacol. Exp. Ther., 2004, 310, 905-914.

Un pourcentage d'inhibition de la liaison du [3H]-CP55940 au niveau du cerveau inférieur à 50% à 10 mg/kg traduit une faible pénétration au niveau du cerveau. De préférence, ce pourcentage est inférieur à 40% et plus préférentiellement inférieur à 30%.

- **Mesure (3)** : la mesure du blocage par les composés de formule (I) de l'effet hypothermique induit par un agoniste des récepteurs CB1 (CP55940), après une administration par voie intraveineuse, comme décrit dans Rinaldi-Carmona M. et al., JPET 2004, 310, 905-914).
  Un pourcentage de réversion de l'effet du CP55940 inférieure à 50 % à 10 mg/kg traduit une faible pénétration au niveau du cerveau. De préférence, ce pourcentage est inférieur à 40% et plus préférentiellement inférieur à 30%.

L'interaction des composés de formule (I) selon l'invention avec les récepteurs CB1 présents à la périphérie a été démontrée chez la souris par la mesure du blocage de l'effet inhibiteur induit par un agoniste des récepteurs CB1 (CP55940) sur le transit gastro-intestinal, après une administration orale, comme décrit dans M. Rinaldi-Carmona et al., J. Pharmacol. Exp. Ther., 2004, 310, 905-914). Un pourcentage de réversion de l'effet du CP55940 supérieur à 50 % à 10 mg/kg traduit un pouvoir antagoniste significatif au niveau des récepteurs CB1 présent à la périphérie. De préférence, le pourcentage de réversion est compris entre 70% et 100%.

A titre d'exemples, les mesures suivantes ont été faites pour les composés n°11, 16 et 24 des Tableaux 1 et 2, et rimonabant comme composé témoin.

| | *Ratio : Quantité présente dans le cerveau Quantité présenté dans le plasma* [voie iv à 3 mg/kg , selon mesure (1)] | % d'inhibition de la liaison du [3H]-CP55940 au niveau du cerveau, par voie iv à 10 mg/kg [récepteurs CB1 présents dans le cerveau, selon mesure (2)]. | % de réversion de l'effet hypothermique du CP55940, par voie iv à 10 mg/kg [récepteurs CB 1 présents dans le cerveau, selon mesure (3)]. | % de réversion de l'effet du CP55940 sur le TGI, par voie po à 10 mg/kg [récepteurs CB1 présents à la périphérie]. |
|---|---|---|---|---|
| Témoin : rimonabant | 1.8 | 100% | 100% | 100% |
| Composé n°11 | / | 25% | 64% | / |
| Composé n°16 | 0.05 | 8% | 3% | 76% |
| Composé n°24 | / | 1% | 20% | 100% |

Les composés de formule (I) sont compatibles avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide pharmaceutiquement acceptable du composé de formule (I).

Ainsi les composés selon l'invention peuvent être utilisés chez l'homme ou chez l'animal (notamment chez les mammifères incluant de façon non limitative les chiens, les chats, les chevaux, les bovins, les moutons) dans le traitement ou la prévention de maladies impliquant les récepteurs aux cannabinoïdes $CB_1$

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles du déficit de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance.

De plus, les composés de formule (I) peuvent être utiles comme neuroprotecteurs, dans le traitement de l'ischémie, des traumatismes crâniens et le traitement des maladies neurodégénératives aiguës ou chroniques : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans la prévention et le traitement des troubles du métabolisme, de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement de l'obésité et des risques associés à l'obésité, notamment les risques cardio-vasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie d'origine alcoolique ou non telles que la cirrhose chronique, la fibrose, la stéatose hépatique, la stéatohépatite ; ainsi que des troubles d'origine endocrinienne, des troubles cardio-vasculaires, de l'hypotension, de l'athérosclérose, du choc hémorragique, du choc septique, de l'asthme, de la bronchite chronique, des maladies pulmonaires chroniques obstructives, du syndrome de Raynaud, du glaucome, des troubles

de la fertilité, de l'accouchement prématuré, de l'interruption de grossesse, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose.

De plus, les composés de formule (I) selon l'invention peuvent être utilisés pour leurs effets protecteurs contre la cardiotoxicité induite par les médicaments.

Selon la présente invention, les composés de formule (I) sont tout particulièrement utiles pour la préparation de médicaments utiles pour la prévention et le traitement des désordres psychiatriques, en particulier la schizophrénie, les troubles de l'attention et de la vigilance, les troubles du déficit de l'attention et de l'hyperactivité (TDAH) chez les enfants hyperkinétiques; pour la prévention et le traitement des déficits mnésiques et des troubles cognitifs ; de la dépendance et du sevrage à une substance, en particulier la dépendance alcoolique, la dépendance nicotinique, le sevrage alcoolique et le sevrage tabagique ; des maladies neurodégénératives aiguës ou chroniques.

Selon la présente invention, les composés de formule (I) sont également tout particulièrement utiles pour la préparation de médicaments utiles dans le traitement et la prévention des troubles de l'appétit, les troubles de l'appétence, des troubles du métabolisme, l'obésité, le diabète de type II, le syndrome métabolique, la dyslipidémie, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à l'utilisation d'un composé de formule (I), ou l'un de ses sels pharmaceutiquement acceptables pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de homme du métier.

Les compositions pharmaceutiques selon la présente invention peuvent contenir à côté d'un composé de formule (I) un (ou plusieurs) autre principe actif utile dans le traitement des troubles et maladies indiquées ci-dessus.

Ce principe actif peut être choisi parmi l'une des classes thérapeutiques suivantes :

- un autre antagoniste ou modulateurs allostériques des récepteurs $CB_1$ aux cannabinoïdes ;
- un modulateur des récepteurs $CB_2$ aux cannabinoïdes ;
- un antagoniste des récepteurs $AT_1$ de l'angiotensine II ;
- un inhibiteur de l'enzyme de conversion ;
- un antagoniste calcique ;
- un diurétique ;
- un béta-bloquant ;
- un antihyperlipémiant ou un antihypercholestérolémiant ;
- un antidiabétique ;
- un autre agent anti-obésité ou agissant sur les troubles du métabolisme ;
- un agoniste nicotinique, un agoniste nicotinique partiel ;
- un antidépresseur, un antipsychotique, un anxiolytique ;
- un anticancéreux ou un agent antiprolifératif ;
- un antagoniste des opioïdes ;

ainsi que :

- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Par antagoniste des récepteurs $AT_1$ de l'angiotensine II, on entend un composé tel que candésartan cilexitil, éprosartan, irbésartan, losartan potassium, olmésartan médoxomil, telmisartan, valsartan, chacun de ces composés

pouvant être lui-même associé à un diurétique tel que l'hydrochlorothiazide.

Par inhibiteur de l'enzyme de conversion, on entend un composé tel que alacépril, bénazépril, captopril, cilazapril, énalapril, énalaprilat, fosinopril, imidapril, lisinopril, moexipril, périndopril, quinapril, ramipril, spirapril, temocapril, trandolapril, zofénopril, chacun de ces composés pouvant lui-même être associé à un diurétique tel que l'hydrochlorothiazide ou l'indapamide ou à un antagoniste calcique tel que l'amlodipine, le diltiazem, le félodipine ou le vérapamil.

Par antagoniste calcique, on entend un composé tel que amlodipine, aranidipine, bénidipine, bépridil, cilnidipine, diltiazem, éfonidipine hydrochloride éthanol, fasudil, félodipine, isradipine, lacidipine, lercanidipine hydrochloride, manidipine, mibéfradil hydrochloride, nicardipine, nifédipine, nilvadipine, nimodipine, nisoldipine, nitrendipine, térodiline, vérapamil.

Par béta-bloquant, on entend un composé tel que acébutolol, alprénolol, amosulalol, arotinolol, aténolol, béfunolol, bétaxolol, bévantolol, bisoprolol, bopindolol, bucumolol, bufétolol, bunitrolol, butofilolol, carazolol, cartéolol, carvédilol, cloranolol, épanolol, esmolol, indénolol, labétalol, landiolol, lévobunolol, lévomoprolol, mépindolol, métipranolol, métoprolol, nadolol, nébivolol, niфénalol, nipradilol, oxprénolol, penbutolol, pindolol, propranolol, salmétérol, sotalol, talinolol, tertatolol, tilisolol, timolol, xamotérol, xibénolol.

Par antihyperlipémiant ou antihypercholestérolémiant, on entend un composé choisi parmi les fibrates tels que alufibrate, béclobrate, bézafibrate, ciprofibrate, clinofibrate, clofibrate, étofibrate, fénofibrate ; les statines (inhibiteurs de HMG-CoA reductase), telles que atorvastatine, fluvastatine sodium, lovastatine, pravastatine, rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterol, tiadénol.

Par antidiabétique, on entend un composé appartenant à l'une des classes suivantes : les sulfonylurées, les biguanidines, les inhibiteurs d'alpha glucosidase, les thiazolidinedione, les métiglinides, tel que acarbose, acétohexamide, carbutamide, chlorpropamide, glibenclamide, glibomuride, gliclazide, glimépiride, glipizide, gliquidone, glisoxepide, glybuzole, glymidine, métahexamide, métformin, miglitol, natéglinide, pioglitazone, répaglinide, rosiglitazone, tolazamide, tolbutamide, troglitazone, voglibose, ainsi que l'insuline et les analogues de l'insuline.

Par autre agent anti-obésité ou agissant sur les troubles du métabolisme, on entend un composé tel que amfépramone, benfluorex, benzphétamine, indanorex, mazindole, méfénorex, méthamphétamine, D-norpseudoéphédrine, sibutramine, un topiramate, un inhibiteur de lipase (orlistat cetilistat), un agoniste PPAR (de l'anglais Peroxisome Proliferator Activated Receptor Agonist), un agoniste de la dopamine, un agoniste des récepteurs de leptine, un inhibiteur du reuptake de la sérotonine, un béta-3 agoniste, un CCK-A agoniste, un inhibiteur de NPY, un agoniste des récepteurs MC 4 (de l'anglais melanocortine 4), un antagoniste des récepteurs MCH (de l'anglais Melanin Concentrating Hormone), un antagoniste de l'orexine, un inhibiteur de phosphodiesterase, un inhibiteur de 11□HSD (11-□-hydroxy steroid deshydrogenase), un inhibiteur de DPP-IV (dipeptidyl peptidase IV), un antagoniste (ou agoniste inverse de l'histamine H3, un dérivé CNTF (de l'anglais Ciliary Neurotrophic Factor), un agoniste des récepteurs GHS (de l'anglais Growth Hormone Secretagogue, un modulateur de la ghréline, un inhibiteur de diacyglycerol acyltransferase (DGAT), un inhibiteur de phosphodiesterase (PDE), un agoniste d'hormone thyroïdienne, un antagoniste des récepteurs glucocorticoïdes, un inhibiteur de stearoyl-CoA- desaturase (SCD), un modulateur des transporteurs de phosphate, de glucose, d'acide gras, de dicarboxylate, un antagoniste $5HT_2$, un antagoniste $5HT_6$, un agoniste de la bombesine.

Par antagoniste des opioïdes on entend un composé tel que naltrexone, naloxone ou nalméfène.

Par agent utile dans le traitement de l'alcoolisme ainsi que des symptômes de sevrage on entend l'acamprosate, les benzodiazepines, les béta-bloquants, la clonidine, la carbamazépine.

Par agent utile, pour traiter l'ostéoporose, on entend par exemple, les biphosphonates tels que étidronate, clodronate, tiludronate, risédronate.

Selon la présente invention, on peut également associer d'autres composés ayant des propriétés antihyperlipemiantes, antihypercholesterolemiantes, antidiabétiques ou anti-obésité. Plus particulièrement on peut associer des composés appartenant à l'une des classes suivantes :

inhibiteurs de PTP 1 B (de l'anglais Protein Tyrosine Phosphase -1B), les agonistes des récepteurs VPAC 2, les modulateurs de GLK, les modulateurs rétinoïdes, les inhibiteurs de glycogène phosphorylase (HGLPa), les antagonistes du glucagon, les inhibiteurs de glucose-6 phosphate, les activateurs de pyruvate dehydrogenase kinase (PKD), les modulateurs de RXR, FXR, LXR, les inhibiteurs de SGLT (de l'anglais Sodium Dependant Glucose Transporter), les inhibiteurs de CETP (de l'anglais Cholesterylester Transfer Protein), les inhibiteurs de squalène synthétase, les inhibiteurs de squalène époxidase, les inhibiteurs de synthèse des triglycérides, les inducteurs de récepteurs LDL (de l'anglais Low Density Lipoprotein), les inhibiteurs de IBAT, les inhibiteurs de FBPase (fructose-1,6-biphosphatase), les modulateurs de CART (de l'anglais Cocaïne-Amphétamine-Regulated Transcript), les antagonistes des récepteurs de l'oréxine.

Le composé de formule (I) ou un de ses sels pharmaceutiquement acceptables et l'autre principe actif associé peuvent être administrés de manière simultanée, séparée ou étalée dans le temps.

On entend par « utilisation simultanée » l'administration des composés de la composition selon l'invention compris dans une seule et même forme pharmaceutique.

On entend par « utilisation séparée » l'administration, en même temps, des deux composés de la composition selon l'invention chacun compris dans une forme pharmaceutique distincte.

On entend par « utilisation étalée dans le temps » l'administration successive, du premier composé de la composition de l'invention, compris dans une forme pharmaceutique, puis du deuxième composé de la composition selon l'invention, compris dans une forme pharmaceutique distincte. Dans ce cas, le laps de temps écoulé entre l'administration du premier composé de la composition selon l'invention et l'administration du deuxième composé de la même composition selon l'invention n'excède généralement pas 24 heures.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé de formule (I) :

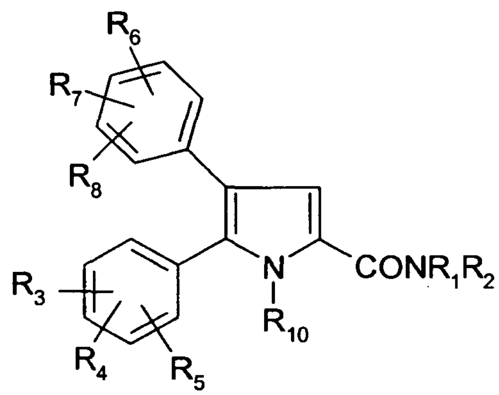

**(I)**

dans laquelle :

- $R_1$ représente un atome d'hydrogène ou un groupe $(C_1-C_4)$alkyle non substitué ou substitué par un ou plusieurs atomes de fluor ;
- $R_2$ représente :
- soit un radical homopipéridin-1-yle, pipéridin-1-yle, pyrrolidin-1-yle ou azétidin-1-yle, lesdits radicaux étant non substitués ou substitués une ou deux fois par un substituant choisi chacun indépendamment parmi un atome de fluor, un groupe $(C_1-C_4)$alkyle, $(C_1-C_4)$alcoxy, trifluorométhyle, $-OCF_3$, $-CH_2OH$, $-CONH_2$ et/ou un groupe phényle, ledit groupe phényle étant non substitué on substitué une ou deux fois par un substituant choisi chacun indépendamment parmi un atome d'halogène, un groupe $-CF_3$, un groupe méthoxy et/ou un groupe trifluorométhoxy ;
- soit un groupe amino $(C_1-C_6)$alkyle non substitué ou substitué par un ou plusieurs substituants choisis chacun indépendamment parmi un atome de fluor, un groupe hydroxyle, $-CONH_2$ et/ou un groupe phényle, ledit groupe phényle étant non substitué ou substitué une ou deux fois par un substituant choisi chacun indépendamment parmi un atome d'halogène, un groupe $-CF_3$, un groupe méthoxy et/ou un groupe trifluorométhoxy ;
- ou $R_1$ et $R_2$ ensemble avec l'atome d'azote auquel ils sont liés constituent :
- soit un radical pipérazin-1-yle ou 1,4-diazépan-1-yle, lesdits radicaux étant non substitués ou substitués par un substituant choisi parmi un groupe phényle, benzodioxolyle, benzodioxolylméthyle, tétrahydrofuranylcarbonyle, $-COR_{11}$, et/ou $-CH_2COR_{11}$, le groupe phényle étant lui-même non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
- soit un radical homopipéridin-1-yle, pipéridin-1-yle, pyrrolidin-1-yle ou azétidin-1-yle, lesdits radicaux étant non substitués ou substitués une ou deux fois par un substituant choisi chacun indépendamment parmi :

  . un atome de fluor, un groupe cyano, $-COR_{11}$, $-NR_{12}R_{13}$, $-NHCOR_{14}$, $-CH_2COR_{11}$, $-SO_2R_{14}$ , et/ou $-SO_2NR_{12}R_{13}$;
  . et/ou un groupe phényle, un groupe pyridinyle ; lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  . et/ou un groupe benzyle non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  . et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou $OCF_3$ ;
  . et/ou un groupe aminophényle, aminobenzyle, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  . et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
  . et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$alkyle, $(C_1-C_4)$alcoxy et/ou cyano, ledit groupe $(C_1-C_4)$alkyle étant non substitué ou substitué une ou plusieurs fois par un atome de fluor ;

- $R_3$, $R_4$, $R_5$, $R_6$, $R_7$, $R_8$ représentent chacun indépendamment un atome d'hydrogène, un atome d'halogène, un groupement -CN, $-S(O)_nR_{14}$, $-OSO_2R_{14}$, un groupe $(C_1-C_6)$alkyle non substitué ou substitué une ou plusieurs fois par un atome de fluor, et/ou un groupe $(C_1-C_6)$alcoxy non substitué ou substitué par un ou plusieurs un atome de fluor, à la condition que l'un des deux substituants $R_3$, $R_6$ représente un groupe $Y-A-R_9$ ;
- Y représente une liaison directe, un atome d'oxygène, un groupe $-S(O)_n-$, $-OSO_2-$ ou $-N(R_{18})-$;
- A représente un groupe alkylène en $(C_1-C_4)$ non substitué ou substitué une ou plusieurs fois par un substituant choisi chacun indépendamment parmi un groupe $(C_1-C_3)$alkyle et/ou par un atome de fluor ;
- $R_9$ représente un groupe $-OR_{12}$, -CN, $-CO_2H$, $NR_{12}R_{13}$, $-CON-R_{12}R_{13}$, $-NR_{15}COR_{12}$, $-CONHNH_2$, -CONHOH, $-CONHSO_2R_{14}$, $-S(O)_nR_{14}$, $-SO_2NR_{12}R_{13}$, $-NR_{18}SO_2R_{14}$, $-NR_{15}SO_2NR_{12}R_{13}$, ou un hétérocycle aromatique choisi parmi :

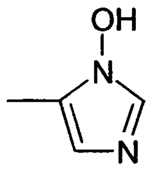 ; 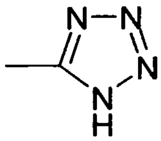 ; 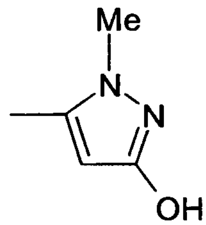 ; 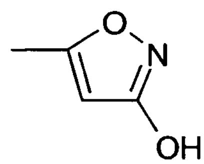 ;

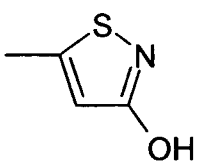 ; 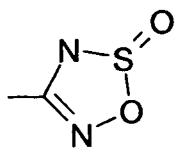 ; 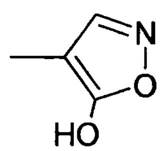 ; 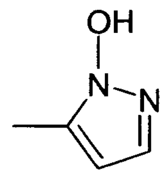

- $R_{10}$ représente un hydrogène ou un groupe $(C_1-C_4)$alkyle
- $R_1$ représente:

  un groupe $(C_1-C_4)$alkyle, phényle, benzyle, $(C_1-C_4)$alcoxy, ou $(C_1-C_3)$alkylène-O-$(C_1-C_3)$alkyle, lesdits groupes étant non substitués ou substitués par un ou plusieurs substituants choisis chacun indépendamment parmi un groupe $(C_1-C_4)$alcoxy, un groupe hydroxy et/ou un atome de fluor ;
  un trifluorométhyle ;
  et/ou un groupement $-NR_{16}R_{17}$ ;

- $R_{12}$ et $R_{13}$ représentent chacun indépendamment un atome d'hydrogène ou un groupe $(C_1-C_6)$alkyle éventuellement substitué par un ou plusieurs substituants choisis chacun indépendamment parmi un atome de fluor, -OH et/ou $-OR_{14}$ ,
- ou $R_{12}$ et $R_{13}$ ensemble avec l'atome d'azote auquel ils sont liés constituent un radical hétérocyclique de 4 à 7 chainons pouvant comporter un second hétéroatome choisi parmi un atome d'azote, d'oxygène ou de soufre ;
- n représente 0, 1 ou 2 ;
- $R_{14}$ représente un groupe $(C_1-C_4)$alkyle non substitué ou substitué par un ou plusieurs atomes de fluor ;
- $R_{15}$ représente un atome d'hydrogène ou un groupe $(C_1-C_4)$alkyle ;
- $R_{16}$ et $R_{17}$ représentent chacun indépendamment :

  . un atome d'hydrogène ;
  . et/ou un groupe benzyle non substitué ou substitué une ou plusieurs fois par un substituant choisi chacun indépendamment parmi un atome d'halogène, un groupe $(C_1-C_4)$alkyle, trifluorométhyle, hydroxyle, $(C_1-C_4)$ alcoxy et/ou cyano ;
  . et/ou un groupe $(C_1-C_6)$alkyle éventuellement substitué par un ou plusieurs un ou plusieurs substituants choisis chacun indépendamment parmi un atome d'halogène, -OH et/ou $-OR_{14}$ ;

- $R_{18}$ représente un atome d'hydrogène ou un groupe $(C_1-C_4)$alkyle non substitué ou substitué par un ou plusieurs atomes de fluor ;
- Alk représente un groupe $(C_1-C_4)$alkyle ;

ou son sel.

**2.** Composé selon la revendication 1 de formule (I) **caractérisé en ce que** Y représente un atome d'oxygène, ou son sel.

**3.** Composé selon la revendication 1 de formule (I) **caractérisé en ce que** Y correspond à une liaison directe, ou son sel.

**4.** Composé selon la revendication 1 de formule (I) **caractérisé en ce que** Y représente un groupe $-S(O)_n$, ou son sel.

**5.** Composé selon la revendication 1 de formule (I) **caractérisé en ce que** Y représente un groupe $-O(SO_2)-$, ou son sel.

**6.** Composé selon la revendication 1 de formule (I) **caractérisé en ce que** Y représente un groupe $-N(R_{18})-$, ou son sel.

**7.** Composé selon l'une quelconque des revendications précédentes **caractérisé en ce que** le substituant $R_6$ représente un groupe Y-A-$R_9$ et le substituant $R_3$ représente un atome d'hydrogène, un atome d'halogène, un groupement -CN, $-S(O)_nR_{14}$, - $OSO_2R_{14}$, un groupe ($C_1$-$C_6$)alkyle non substitué ou substitué une ou plusieurs fois par un atome de fluor, ou un groupe ($C_1$-$C_6$)alcoxy non substitué ou substitué par un ou plusieurs un atome de fluor, ou son sel.

**8.** Composé selon l'une quelconque des revendications 1 à 6 **caractérisé en ce que** le substituant $R_3$ représente un groupe Y-A-$R_9$ et le substituant $R_6$ représente un atome d'hydrogène, un atome d'halogène, un groupement -CN, $-S(O)_nR_{14}$, - $OSO_2R_{14}$, un groupe ($C_1$-$C_6$)alkyle non substitué ou substitué une ou plusieurs fois par un atome de fluor, ou un groupe ($C_1$-$C_6$)alcoxy non substitué ou substitué par un ou plusieurs un atome de fluor, ou son sel.

**9.** Composé selon l'une quelconque des revendications précédentes **caractérisé en ce que** A représente un groupe alkylène en ($C_1$-$C_4$) non substitué, ou son sel.

**10.** Composé selon l'une quelconque des revendications précédentes **caractérisé en ce que** $R_9$ représente un groupe $-OR_{12}$, $-NR_{12}R_{13}$, $-CONR_{12}R_{13}$, $-NR_{15}COR_{12}$, $-CONHNH_2$, -CONHOH, $-S(O)_nR_{14}$, $-SO_2NR_{12}R_{13}$, $-NR_{18}SO_2R_{14}$ ou $-NR_{15}SO_2NR_{12}R_{13}$, ou son sel.

**11.** Composé selon l'une quelconque des revendications précédentes **caractérisé en ce que** :

- $R_1$ représente un atome d'hydrogène ou un groupe ($C_1$-$C_4$)alkyle non substitué ou substitué par un ou plusieurs atomes de fluor ;
- $R_2$ représente :
- soit un radical homopipéridin-1-yle, pipéridin-1-yle, pyrrolidin-1-yle ou azétidin-1-yle, lesdits radicaux étant substitués une ou deux fois par un substituant choisi chacun indépendamment parmi un atome de fluor, un groupe ($C_1$-$C_4$)alkyle , ($C_1$-$C_4$)alcoxy, trifluorométhyle, $-OCF_3$, $-CH_2OH$, $-CONH_2$ et/ou un groupe phényle, ledit groupe phényle étant non substitué on substitué une ou deux fois par un substituant choisi chacun indépendamment parmi un atome d'halogène, un groupe - $CF_3$, un groupe méthoxy et/ou un groupe trifluorométhoxy ;
- soit un groupe amino ($C_1$-$C_6$)alkyle substitué par un ou plusieurs substituants choisis chacun indépendamment parmi un atome de fluor, un groupe hydroxyle, - $CONH_2$ et/ou un groupe phényle, ledit groupe phényle étant non substitué ou substitué une ou deux fois par un substituant choisi chacun indépendamment parmi un atome d'halogène, un groupe $-CF_3$, un groupe méthoxy et/ou un groupe trifluorométhoxy ;

ou son sel.

**12.** Composé selon la revendication 11 **caractérisé en ce que** le radical $R_2$ représente en particulier un radical homopipéridin-1-yle, pipéridin-1-yle, pyrrolidin-1-yle ou azétidin-1-yle, lesdits radicaux étant substitués une ou deux fois par un substituant choisi chacun indépendamment parmi un atome de fluor, un groupe ($C_1$-$C_4$)alkyle, ($C_1$-$C_4$)alcoxy, trifluorométhyle, $-OCF_3$, $-CH_2OH$, $-CONH_2$ et/ou un groupe phényle, ledit groupe phényle étant non substitué on substitué une ou deux fois par un substituant choisi chacun indépendamment parmi un atome d'halogène, un groupe $-CF_3$, un groupe méthoxy et/ou un groupe trifluorométhoxy ;
ou son sel.

**13.** Composé selon l'une quelconque des revendications 1 à 10 **caractérisé en ce que** :

- $R_1$ et $R_2$ ensemble avec l'atome d'azote auquel ils sont liés constituent :

  soit un radical pipérazin-1-yle ou 1,4-diazépan-1-yle, lesdits radicaux étant substitués par un substituant choisi parmi un groupe phényle, benzodioxolyle, benzodioxolylméthyle, tétrahydrofuranylcarbonyle, $-COR_{11}$, et/ou $-CH_2COR_{11}$, le groupe phényle étant lui-même non substitué ou substitué une ou plusieurs fois par un substituant choisi chacun indépendamment parmi un atome d'halogène, un groupe ($C_1$-$C_4$) alkyle, trifluorométhyle, hydroxyle, ($C_1$-$C_4$)alcoxy et/ou cyano ; soit un radical homopipéridin-1-yle, pipéridin-1-yle, pyrrolidin-1-yle ou azétidin-1-yle, lesdits radicaux étant substitués une ou deux fois par un substituant choisi chacun indépendamment parmi :

  - un atome de fluor, un groupe cyano, $-COR_{11}$, $-NR_{12}R_{13}$, $-NHCOR_{14}$, $-CH_2COR_{11}$, $-SO_2R_{14}$ et/ou $-SO2NR_{12}R_{13}$ ;

- et/ou un groupe phényle, pyridinyle ; lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
- et/ou un groupe benzyle substitué une ou plusieurs fois par un substituant choisi chacun indépendamment parmi un atome d'halogène, un groupe $(C_1-C_4)$alkyle, trifluorométhyle, hydroxyle, $(C_1-C_4)$ alcoxy et/ou cyano ;
- et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou $OCF_3$ ;
- et/ou un groupe aminophényle, aminobenzyle, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
- et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
- et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$ alkyle, $(C_{1-}C_4)$alcoxy et/ou cyano, ledit groupe $(C_1-C_4)$alkyle étant non substitué ou substitué une ou plusieurs fois par un atome de fluor ;

ou son sel.

**14.** Composé selon la revendication 13 **caractérisé en ce que** :

- $R_1$ et $R_2$ ensemble avec l'atome d'azote auquel ils sont liés constituent un radical homopipéridin-1-yle, pipéridin-1-yle, pyrrolidin-1-yle ou azétidin-1-yle, lesdits radicaux étant substitués une ou deux fois par un substituant choisi chacun indépendamment parmi :

  - un atome de fluor, un groupe cyano, $-COR_{11}$, $-NR_{12}R_{13}$, $-NHCOR_{14}$, - $CH_2COR_{11}$, $-SO_2R_{14}$ et/ou $-SO_2NR_{12}R_{13}$;
  - et/ou un groupe phényle, pyridinyle ; lesdits groupes étant non substitués ou substitués une ou plusieurs fois par un substituant choisi chacun indépendamment parmi un atome d'halogène, un groupe $(C_1-C_4)$ alkyle, trifluorométhyle, hydroxyle, $(C_1-C_4)$alcoxy et/ou cyano ;
  - et/ou un groupe benzyle substitué une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et cyano ;
  - et/ou un groupe pipéridin-1-yle, pyrrolidin-1-yle, azétidin-1-yle, lesdits groupes étant non substitués ou substitués une ou plusieurs fois par un substituant choisi chacun indépendamment parmi un atome de fluor, un groupe $(C_1-C_4)$alkyle, $(C_1-C_4)$alcoxy, hydroxy, trifluorométhyle et/ou $OCF_3$ ;
  - et/ou un groupe aminophényle, aminobenzyle, lesdits groupes étant non substitués ou substitués une ou plusieurs fois par un substituant choisi chacun indépendamment parmi un atome d'halogène, un groupe $(C_1-C_4)$alkyle, trifluorométhyle, hydroxyle, $(C_1-C_4)$alcoxy et/ou cyano ;
  - et/ou un groupe amino $(C_1-C_6)$alkyle non substitué ou substitué une ou plusieurs fois par un substituant choisi chacun indépendamment parmi un atome d'halogène, un groupe hydroxyle, $(C_1-C_4)$alcoxy et/ou cyano ;
  - et/ou un groupe amino $(C_3-C_7)$cycloalkyle non substitué ou substitué une ou plusieurs fois par un substituant choisi chacun indépendamment parmi un atome d'halogène, un groupe hydroxyle, $(C_1-C_4)$alkyle, $(C_1-C_4)$alcoxy et/ou cyano, ledit groupe $(C_1-C_4)$alkyle étant non substitué ou substitué une ou plusieurs fois par un atome de fluor ;

ou son sel.

**15.** Composé selon la revendication 1 **caractérisé en ce que** le composé est choisi parmi le groupe constitué de :

- 1[4-(2,4-dichlorophényl)-5-[4-(3-hydroxypropoxy)-phényl]-1-méthyl-1H-pyrrole-2-carbonyl]-4-(3-fluoro-benzylamino)-pipéridine-4-carboxamide,
- 1-[5-(4-carbamoylméthoxy)-phényl-4-(2,4-dichlorophényl)-1-méthyl-1H-pyrrole-2-carbonyl]-4-phénylpipéridine-4-carboxamide,
- 1-[4-(2-chlorophényl)-5-[4-(3-hydroxypropoxy)-phényl]-1-méthyl-1H-pyrrole-2-carbonyl]-4-(4-fluoro-benzylamino)-pipéridine-4-carboxamide,
- et leurs sels.

**16.** Procédé de préparation d'un composé de formule (I) **caractérisé en ce que** l'on traite l'acide de formule (II) ou un dérivé fonctionnel de cet acide de formule (II) :

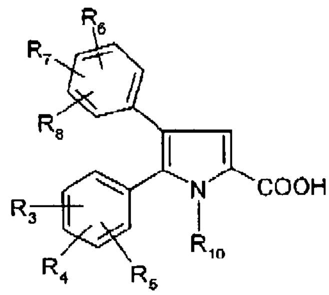

**(II)**

dans laquelle $R_3$, $R_4$, $R_5$, $R_6$, $R_7$, $R_8$ et $R_{10}$ sont tels que définis dans l'une quelconque des revendications 1 à 10 et 15, avec une amine de formule $HNR_1R_2$ dans laquelle $R_1$ et $R_2$ sont tels que définis dans l'une quelconque des revendications 1 et 11 à 15.

**17.** Procédé de préparation selon la revendication 16 **caractérisé en ce que** l'on traite un dérivé fonctionnel de formule (IIter) :

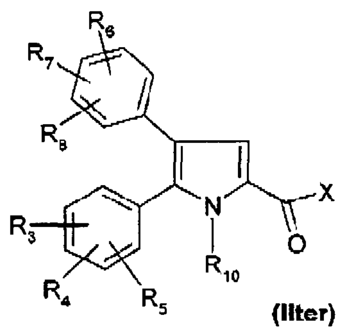

**(IIter)**

dans laquelle :

- X représente un groupe hydroxyle, un atome d'halogène, $(C_1-C_4)$alcoxy ou benzyloxy ;
- et $R_3$, $R_4$, $R_5$, $R_6$, $R_7$, $R_8$ et $R_{10}$ sont tels que défmis dans l'une quelconque des revendications 1 à 10 et 15.

avec une amine de formule $HNR_1R_2$ dans laquelle $R_1$ et $R_2$ sont tels que définis dans l'une quelconque des revendications 1 et 11 à 14.

**18.** Composé de formule (IIter) :

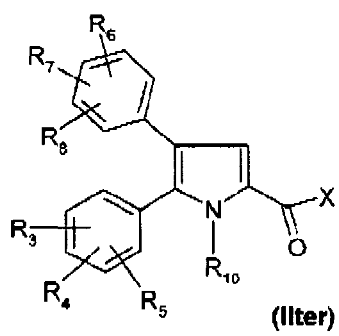

**(IIter)**

dans laquelle :

- X représente un groupe hydroxyle, un atome d'halogène, $(C_1-C_4)$alcoxy ou benzyloxy ;
- et $R_3$, $R_4$, $R_5$, $R_6$, $R_7$, $R_8$ et $R_{10}$ sont tels que définis pour les composés de formule (I) selon l'une quelconque des revendications 1 à 10.

**19.** Médicament, **caractérisé en ce qu'**il comprend un composé de formule (I) selon l'une quelconque des revendications 1 à 15, ou un sel d'addition de ce composé de formule (I) à un acide pharmaceutiquement acceptable.

**20.** Composition pharmaceutique, **caractérisée en ce qu'**elle comprend un composé de formule (I) selon l'une quelconque des revendications 1 à 15, ou un sel d'addition de ce composé de formule (I) à un acide pharmaceutiquement acceptable ainsi qu'au moins un excipient pharmaceutiquement acceptable.

**21.** Utilisation d'un composé selon l'une quelconque des revendications 1 à 15 pour la préparation d'un médicament destiné au traitement et/ou prévention des désordres psychiatriques, de la dépendance et du sevrage à une substance, des troubles cognitifs, des troubles de l'attention et de la vigilance, des maladies neurodégénératives aigües et chroniques.

**22.** Utilisation d'un composé selon l'une quelconque des revendications 1 à 15 pour la préparation d'un médicament destiné au traitement et/ou prévention des troubles du métabolisme, des troubles de l'appétit, des troubles de l'appétence, de l'obésité, de la boulimie, du diabète, du syndrome métabolique, de la dyslipidémie.

**23.** Utilisation d'un composé selon l'une quelconque des revendications 1 à 15 pour la préparation d'un médicament destiné au traitement et/ou prévention de la douleur, des douleurs neuropathiques, des douleurs aiguës périphériques, des douleurs chroniques d'origine inflammatoire, des douleurs induites par un traitement anticancéreux.

**24.** Utilisation d'un composé selon l'une quelconque des revendications 1 à 15 pour la préparation d'un médicament destiné au traitement et/ou prévention des troubles gastro-intestinaux, des vomissements, des troubles diarrhéiques, des ulcères, des maladies du foie.

**25.** Utilisation d'un composé selon l'une quelconque des revendications 1 à 15 pour la préparation d'un médicament destiné au traitement et/ou prévention des phénomènes inflammatoires, des maladies du système immunitaire, de l'arthrite rhumatoïde, des maladies entrainant une démyélinisation, de la sclérose en plaque, des maladies des os et de l'ostéoporose.

**26.** Utilisation d'un composé selon l'une quelconque des revendications 1 à 15 pour la préparation d'un médicament destiné au traitement et/ou prévention des maladies et troubles du mouvement, en particulier des dyskinésies ou de la maladie de Parkinson, des démences séniles et de la maladie d'Alzheimer.

## Claims

**1.** Compound of Formula (I):

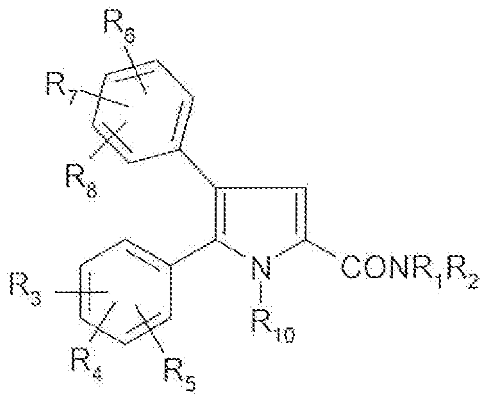

(I)

in which:

- $R_1$ represents a hydrogen atom or a $(C_1\text{-}C_4)$alkyl group which is unsubstituted or substituted with one or more fluorine atoms;
- $R_2$ represents:
- either a homopiperidin-1-yl, piperidin-1-yl, pyrrolidin-1-yl or azetidin-1-yl radical, said radicals being unsubstituted or substituted once or twice with a substituent, each chosen independently from a fluorine atom, a $(C_1\text{-}C_4)$alkyl, $(C_1\text{-}C_4)$alkoxy, trifluoromethyl, $-OCF_2$,$-CH_2OH$ or $-CONH_2$ and/or to phenyl group, said phenyl group being unsubstituted or substituted once or twice with a substituent, each chosen independently from a halogen atom, a $-CF_3$ group, a methoxy group and/or a trifluoromethoxy group;
- or an amino$(C_1\text{-}C_6)$alkyl group which is unsubstituted or substituted with one or more substituents, each chosen independently from a fluorine atom, a hydroxyl group, a $-CONH_2$ group and/or a phenyl group, said phenyl group being or substituted once or twice with a substituent, each chosen independently from a halogen atom, a $-CF_3$ group, a methoxy group and/or a trifluoromethoxy group;
- or $R_1$ and $R_3$, together with the nitrogen atom to which they are attached, constitute:

  - either a piperazin-1-yl or 1,4-diazepan-1-yl radical, said radicals being unsubstituted or substituted with a substituent chosen from a phenyl, benzodioxolyl, benzodioxolylmethyl, tetrahydrofuranylcarbonyl, $-COR_{11}$, and/or - $CH_2COR_{11}$ group, the phenyl group itself being unsubstituted or substituted one or more times with a substituent, each chosen independently from a halogen atom, or a $(C_1\text{-}C_4)$alkyl, trifluoromethyl, hydroxy, $(C_1\text{-}C_4)$alkoxy and/or cyano group;
  - or a homopiperidin-1-yl, piperidin-1-yl, pyrrolidin-1-yl or azetidin-1-yl radical, said radicals being unsubstituted or substituted once or twice with a substituent, each independently chosen from:

    - a fluorine atom, or a cyano, $-COR_{11}$, $-NR_{12}R_{13}$, $-NHCOR_{14}$, $-CH_2COR_{11}$, $-SO_2R_{14}$, and/or $-SO_2NR_{12}R_{13}$ group;
    - and/or a phenyl group or a pyridinyl group; said groups being unsubstituted or substituted one or more times with a substituent, each chosen independently from a halogen atom or a $(C_1\text{-}C_4)$alkyl, trifluoromethyl, hydroxyl, $(C_1\text{-}C_4)$alkoxy and/or cyano group;
    - and/or a benzyl group which is unsubstituted or substituted one or more times with a substituent, each chosen independently from a halogen atom, or a $_1\text{-}C_4)$alkyl, trifluoromethyl, hydroxyl, $(C_1\text{-}C_4)$alkoxy and/or cyano group;
    - and/or a piperidin-1-yl, pyrrolidin-1-yl or azetidin-1-yl group, said groups being unsubstituted or substituted one or more times with a substituent, each chosen independently from a fluorine atom, or a $(C_1\text{-}C_4)$alkyl, $(C_1\text{-}C_4)$alkoxy, hydroxyl, trifiuoromethyl and/or $OCF_3$ group;
    - and/or an aminophenyl or aminobenzyl group, said groups being unsubstituted or substituted one or more times with a substituent, each chosen independently from a halogen atom, or a $(C_1\text{-}C_4)$alkyl, trifluoromethyl, hydroxyl, $(C_1\text{-}C_4)$alkoxy and/or cyano group;
    - and/or an amino$(C_1\text{-}C_6)$alkyl group which is unsubstituted or substituted one or more times with as substituent, each chosen independently from a halogen atom, a hydroxyl group, a $(C_1\text{-}C_4)$alkoxy group and/or a cyano group;
    - and/or an amino$(C_3\text{-}C_7)$cycloalkyl group which is unsubstituted or substituted one or more times with

a substituent, each chosen independently from a halogen atom, a hydroxyl group, a $(C_1-C_4)$alkyl group, a $(C_1-C_4)$alkoxy group and/or a cyano group, said $(C_1-C_4)$alkyl group being unsubstituted or substituted one or more times with a fluorine atom;

- $R_3$, $R_4$, $R_5$, $R_6$, $R_7$, $R_8$ each independently represent a hydrogen atom, a halogen atom, a -CN, $-S(O)_nR_{14}$ or $-OSO_2R_{14}$ group, or a $(C_1-C_6)$alkyl group which is unsubstituted or substituted one or more times with a fluorine atom and/or a $(C_1-C_6)$alkoxy group which is unsubstituted or substituted with one or more fluorine atoms, on the condition that one of the two substituents $R_3$ and $R_6$ represents a Y-A-$R_9$ group;
- Y represents a direct bond, an oxygen atom, or an $-S(O)_n-$, $-OSO_2-$ or $-N(R_{13})-$ group;
- A represents a $(C_1-C_4)$ alkylene group which is unsubstituted or substituted one or more times with a substituent, each chosen independently from a $(C_1-C_3)$alkyl group and/or with a fluorine atom;
- $R_9$ represents an $-OR_{12}$, -CN, $-CO_2H$, $NR_{12}R_{13}$,$-CONR_{12}R_{13}$,
- $NR_{15}COR_{12}$, $-CONHNH_2$, -CONHOH, $-CONHSO_2R_{14}$, - $S(O)_nR_{14}$,
- $SO_2NR_{12}R_{13}$, $-NR_{16}SO_2R_{14}$ or $-NR_{15}SO_2NR_{12}R_{13}$, or an aromatic heterocycle chosen from;

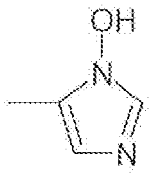 ; 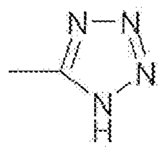 ; 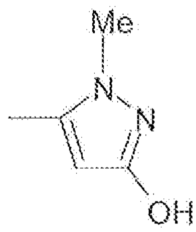 ; 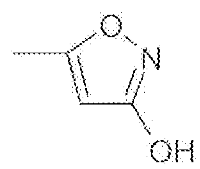 ;

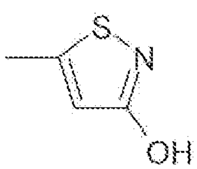 ; 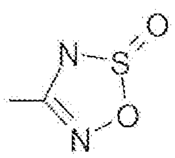 ; 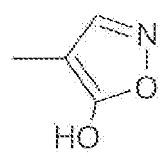 ; 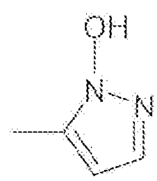

- $R_{10}$ represents a hydrogen or a $(C_1-C_4)$alkyl group;
- $R_{11}$ represents:
- a $(C_1-C_4)$alkyl, phenyl, benzyl, $(C_1-C_4)$alkoxy or $(C_1-C_3)$alkylene-O-$(C_1-C_3)$alkyl group, said groups being unsubstituted or substituted with one or more substituents, each chosen independently from a $(C_1-C_4)$alkoxy group, a hydroxy group and/or a fluorine atom;
- a trifluoromethyl;
- and/or an $-NR_{16}R_{17}$ group;
- $R_{12}$ and $R_{13}$ each independently represent a hydrogen atom or a $(C_1-C_6)$alkyl group optionally substituted with one or more substituents, each chosen independently from a fluorine atom, -OH and/or $-OR_{14}$,
- or $R_{12}$ and $R_{13}$, together with the nitrogen atom to which they are attached, constitute a 4- to 7-membered heterocyclic radical 1 which may comprise a second heteroatom chosen from a nitrogen, oxygen or sulphur atom;
- n represents 0, 1 or 2;
- $R_{14}$ represents a $(C_1-C_4)$alkyl group which is unsubstituted or substituted with one or more fluorine atoms;
- $R_{15}$ represents a hydrogen atom or a $(C_1-C_4)$alkyl group;
- $R_{16}$ and $R_{17}$ each independently represent;

  - a hydrogen atom;
  and/or a benzyl group which is unsubstituted or substituted one or more times with a substituent, each chosen independently from a halogen atom, or a $(C_1-C_4)$alkyl, trifluoromethyl, hydroxyl, $(C_1-C_4)$ alkoxy and/or cyano group;
  - and/or a $(C_1-C_6)$alkyl group optionally substituted with one or more substituents, each chosen independently from a halogen atom, and/or $-OR_{14}$;
  - $R_{18}$ represents a hydrogen atom or a $(C_1-C_4)$alkyl group which is unsubstituted or substituted with one or more fluorine atoms;

- Alk represents a $(C_1-C_4)$alkyl group;

or the salt thereof.

**2.** Compound according to Claim 1, of Formula (I), **characterised in that** Y represents an oxygen atom, or the salt thereof.

**3.** Compound according to Claim 1, of Formula (I), **characterized in that** Y corresponds to a direct bond, or the salt thereof.

**4.** Compound according to Claim 1, of Formula (I), **characterized in that** Y represents an $-S(O)_n$ group, or the salt thereof.

**5.** Compound according to Claim 1, of Formula (I), **characterised in that** Y represents an $-O(SO_2)$-group, or the salt thereof.

**6.** Compound according to Claim 1, of Formula (I), **characterized in** chat Y represents an $N(R_{18})$-group, or the salt thereof.

**7.** Compound according to any one of the preceding claims, **characterized in that** the substituent $R_6$ represents a Y-A-$R_9$ group and the substituent $R_3$ represents a hydrogen atom, a halogen atom, a-CN, $-S(O)_nR_{14}$ or $-OSO_2R_{14}$ group, a $(C_1-C_6)$alkyl group which is unsubstituted or substituted one or more times with a fluorine atom, or a $(C_1-C_6)$ alkoxy group which is unsubstituted or substituted with one or more fluorine atoms, or the salt thereof.

**8.** Compound according to any one of Claims 1 to 6, **characterised in that** the substituent $R_3$ represents a Y-A-$R_9$ group and a substituent $R_6$ represents a hydrogen atom, a halogen atom, a - CN, $-S(O)_nR_{14}$ or $-OSO_2R_{14}$ group, a $(C_1-C_6)$alkyl group which is unsubstituted or substituted one or more times with a fluorine atom, or a $(C_1-C_6)$alkoxy group which is unsubstituted or substituted with one or more fluorine atoms, or the salt thereof.

**9.** Compound according to any one of the preceding claims, **characterised in that** A represents an unsubstituted $(C_1-C_4)$ alkylene group, or the salt thereof.

**10.** Compound according to any one of the preceding claims, **characterized in that** $R_9$ represents an $-OR_{12}$, $-NR_{12}R_{13}$, $-CONR_{12}R_{13}$, $NR_{15}COR_{12}$,

- $CONHNH_2$, -CONHOH, $-S(O)_nR_{14}$, $-SO_2NR_{12}R_{13}$, $NR_{18}SO_2R_{14}$ or
- $NR_{15}SO_2NR_{12}R_{13}$ group, or the salt thereof.

**11.** Compound according to any one of the preceding claims, **characterized in that**:

- $R_1$ represents a hydrogen atom or a $(C_1-C_4)$alkyl group which is unsubstituted or substituted with one or more fluorine atoms;
- $R_2$ represents:

  - either a homopiperidin-1-yl, piperidin-1-yl, pyrrolidin-1-yl or azetidin-1-yl radical, said radicals being substituted once or twice with a substituent, each chosen independently from a fluorine atom, a $(C_1-C_4)$alkyl, $(C_1-C_4)$alkoxy, trifluoromethyl, $-OCF_3$, $-CH_2OH$ or $-CONH_2$ group and/or a phenyl group, said phenyl group being unsubstituted or substituted once or twice with a substituent, each chosen independently from a halogen atom, a $-CF_3$ group, a methoxy group and/or a trifluoromethoxy group;
  - or an amico$(C_1-C_6)$alkyl group substituted with one or more substituents, each chosen independently from a fluorine atom, a hydroxyl group, a $-CONH_2$ group and/or a phenyl group, said phenyl group being unsubstituted or substituted once or twice with a substituent, each independently chosen from a halogen atom, a $-CF_3$ group, a methoxy group and/or a trifiuoromethoxy group;

and the salt thereof.

**12.** Compound according to Claim 11, **characterized in that** $R_2$ radical represents in particular a homopiperidin-1-yl, piperidin-1-yl pyrrolidin-1-yl or azeridin-1-yl radical, said radicals being substituted once or twice with a substituent,

each chosen independently from a fluorine atom, a $(C_1-C_4)$alkyl, $(C_1-C_4)$alkoxy, triuoromethyl, $OCF_3$, $-CH_2OH$ or $-CONH_2$ group and/or a phenyl group, said phenyl group being unsubstituted or substituted once or twice with a substituent, each chosen independently from a halogen atom, a- $CF_3$ group, a methoxy group and/or a trifluoromethoxy group;
or the salt thereof.

**13.** Compound according to any one of Claims 1 to 10, **characterized in that** :

- $R_1$ and $R_2$, together with the nitrogen atom to which they are linked, constitute:
- either a piperazin-1-yl or 1,4-diazepan-1-yl radical, said radicals being substituted with a substituent chosen from a phenyl, benzodioxolyl, benzodioxolylmethyl, tetrahydrofuranylcarbonyl, $COR_{11}$ and/or $-CH_2COR_{11}$ group, the phenyl group itself being unsubstituted or substituted one or more times with a substituent, each chosen independently from a halogen atom, a $(C_1-C_4)$alkyl group, a trifluoromethyl group, a hydroxyl group, a $(C_1-C_4)$ group and/or a cyano group;
or a homopiperidin-1-yl, piperiding-1-yl, pyrrolidin-1-yl or azetidin-1-yl radical, said radicals being substituted once or twice with a substituent, each chosen independently from:

  - a fluorine atom, or a cyano, $-COR_{11}$, $-NR_{12}R_{13}$,
  - $NHCOR_{14}$, $-CH_2COR_{11}$, $-SO_2R_{14}$ and/or $-SO_2NR_{12}R_{12}$ group;
  - and/or a phenyl or pyridinyl group; said groups being unsubstituted or substituted one or more times with a substituent, each chosen independently from a halogen atom, a $(C_1-C_4)$alkyl group, a trifluoromethyl group, a hydroxyl group, a $(C_1-C_4)$alkoxy group and/or a cyano group;
  - and/or a benzyl group substituted one or more times with a substituent, each chosen independently from a halogen atom, a $(C_1-C_4)$alkyl group, a trifluoromethyl group, a hydroxy group, a $(C_1-C_4)$ alkoxy group and/or a cyano group;
  - and/or a piperidin-1-yl, pyrrolidin-1-yl or azetidin-1-yl group, said groups being unsubstituted or substituted one or more times with a substituent, each chosen independently from a fluorine atom, a $(C_1-C_4)$alkyl group, a $(C_1-C_4)$alkoxy group, a hydroxyl group, a trifluoromethyl group an $OCF_3$ group;
  - and/or an aminophenyl or aminobenzyl group, said groups being unsubstibuted or substituted one or more times with a substituent, each chosen independently from a halogen atom, a $(C_1-C_4)$alkyl group, a trifluoromethyl group, a hydroxyl group, a $(C_1-C_4)$alkoxy group and/or a cyano group;
  - and/or an amino $(C_1-C_6)$ alkvl group which is unsubstituted or substituted one or more times with a substituent, each chosen independently from a halogen atom, a hydroxyl group, a $(C_1-C_4)$ alkoxy group and/or a cyano group:
  - and/or an amino $(C_3-C_7)$cycloalkyl group which is unsubstituted or substituted one or more times with a substituent, each chosen independently from a halogen atom, a hydroxyl group, a $(C_1-C_4)$alkyl group, a $(C_1-C_4)$alkoxy group and/or a cyano group, said $(C_1-C_4)$alkyl group being unsubstituted or substituted one or more times with a fluorine atom;

or the salt thereof.

**14.** Compound according to Claim 13, **characterised in that**:

- $R_1$ and $R_2$, together with the nitrogen atom to which they are attached, constitute a homopiperidinyl-yl, piperidin-1 pyrrolidin-1-yl or azetidin-1-yl radical, said radicals being substituted once or twice with a substituent, each chosen independently from:
- a fluorine atom, or a cyano, $-COR_{11}$, $-NR_{12}R_{13}$, $-NHCOR_{14}$, $-CH_3COR_{11}$, $-SO_2R_{14}$ and/or $-SO_2NR_{12}R_{13}$ group;
- and/or a phenyl or pyridinyl group; said groups being unsubstituted or substituted one or more times with a substituent, each chosen independently from a halogen atom, a $(C_1-C_4)$ alkyl group, a trifluoarومethyl group, a hydroxyl group, a $(C_1-C_4)$alkoxy group and/or a cyano group;
- and/or a benzyl group substituted one or more times with a substituent each chosen independently from a halogen atom, a $(C_1-C_4)$alkyl group, a trifluoromethyl group, a hydroxyl group, a $(C_1-C_4)$alkoxy group and a cyano group;
- and/or a piperiding-1-yl, pyrrolidin-1-yl or azetidin-1-yl group, said groups being unsubstituted or substituted one or more times with a substituent, each chosen independently from a fluorine atom, a $(C_1-C_4)$alkyl group, a $(C_1-C_4)$ alkoxy group, a hydroxyl group, a trifluoromethyl group and/or an $-OCF_3$ group;
- and/or an aminophenyl or aminobenzyl group, said groups being insubstituted or substituted one or more times with a substituent, each chosen independently from a halogen atom, a $(C_1-C_4)$alkyl group, a trifluoromethyl

group, a hydroxyl group, a group a cyano group;
- and/or an amino ($C_1$-$C_6$) alkyl group which is unsubstituted or substituted one or more times with a substituent, each chosen independently from a halogen atom, a hydroxyl group, a ($C_1$-$C_4$)alkoxy group and/or a cyano group;
- and/or an amino ($C_3$-$C_7$)cycloalkylgroup which is unsubstituted or substituted one or more times with a substituent, each chosen independetly from a halogen atom, a hydroxyl group, a ($C_1$ -$C_4$)alkyl group a ($C_1$-$C_4$) alkoxy group and/or or a cyano group, said ($C_1$-$C_4$)alkyl group being unsubstituted or substituted one or more times with a fluorine atom;

or the salt thereof.

**15.** Compound according to Claim 1, **characterized in that** the compound is chosen from the group consisting of:

- 1-[4-(2,4-dichlorophenyl)-5-[4-(3-hydroxypropoxy)phenyl]-1-methyl-1H-pyrrole-2-carbonyl]-1-(3-fluorobenzylamino)piperidine-4-carboxamide,
- 1-[5-(4-carbamoylmethoxy)phenyl-4-(2,4-dichlorophenyl)-1-methyl-1H-pyrrole-2-carbonyl]-4-phenylpiperidine-4-carboxamide,
- 1-[4-(2-chlorophenyl)-5-[4-(3-hydroxypropoxy)phenyl]-1-methyl-1H-pyrrole-2-carbonyl]-4-(4-fluoro-benzylamino)piperidine-4-carboxamide,
- and the salts thereof.

**16.** Method for preparing a compound of Formula (I), **characterized in that** the acid of Formula (II) or a functional derivative of this acid of Formula (II):

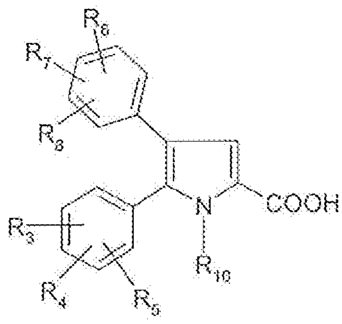

(II)

in which $R_3$, $R_4$, $R_5$, $R_6$, $R_7$, $R_8$ and $R_{10}$ are as defined in any one of Claims 1 to 10 and 15, is treated with an amine of formula $HNR_1R_2$ in which $R_1$ and $R_2$ are as defined in any one of Claims 1 and to 15.

**17.** Method of preparation according to Claim 16, **characterized in that** a functional derivative of formula (IIb) :

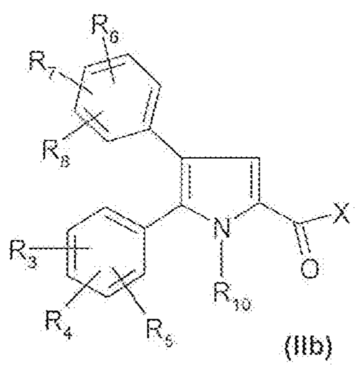

(IIb)

in which:

- X represents a hydroxyl group, a halogen atom, ($C_1$-$C_4$)alkoxy or benzyloxy;
- and $R_3$, $R_4$, $R_5$, $R_6$, $R_7$, $R_8$ and $R_{10}$ are as defined in any one of Claims 1 to 10 and 15,

is treated with an amine of formula $HNR_1R_2$ in which $R_1$ and $R_2$ are as defined in any one of Claims 1 and 11 to 14.

**18.** Compound of Formula (IIb);

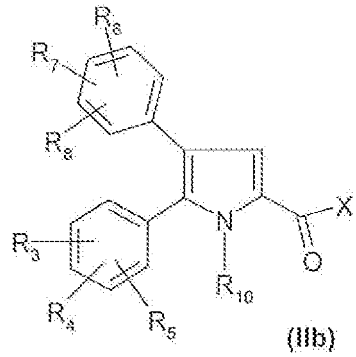

(IIb)

in which:

- X represents a hydroxyl group, a halogen atom, $(C_1-C_4)$alkoxy or benzyloxy;
- and $R_3$, $R_4$, $R_5$, $R_6$ $R_7$, $R_8$ and $R_{10}$ are as defined for the compounds of Formula (I) according to any one of Claims 1 to 10.

**19.** Medicament, **characterised in that** it comprises a compound of Formula (I) according to any one of Claims 1 to 15, or an addition salt of this compound of Formula (I) with a pharmaceutically acceptable acid.

**20.** Pharmaceutical composition, **characterized in that** it comprises a compound of Formula (I) according to any one of Claims 1 to 15, or an addition salt of this compound of Formula (I) with a pharmaceutically acceptable acid, and also: at least one pharmaceutically acceptable excipient.

**21.** Use of a compound according to any one of Claims 1 to 15, for the preparation of a medicament for use in the treatment and/or prevention of psychiatric disorders; dependence on and withdrawal from a substance, cognitive disorders, attention and consciousness disorders, and acute and chronic neurodegenerative diseases,

**22.** Use of a compound, according to any one of Claims 1 to 15, for the preparation of a medicament for use in the treatment and/or prevention of metabolic disorders, appetite disorders, appetence disorders, obesity, bulimia, diabetes, metabolic syndrome and dyslipidaemia.

**23.** Use of a composition according to any one of Claims 1 to 15, for the preparation of a medicament for use in the treatment and/or prevention of pain, neuropathic pain, acute peripheral pain, chronic pain of inflammatory origin, and pain induced by an anticancer treatment.

**24.** Use of a compound according to any one of Claims 1 to 15, for the preparation of a medicament for use in the treatment and/or prevention of gastrointestinal disorders, vomiting, diarrhoea disorders, ulcers and liver diseases.

**25.** Use of a compound according to any one of Claims 1 to 15, for the preparation of a medicament for use in the treatment and/or prevention of inflammatory phenomena, immune system diseases, rheumatoid arthritis, diseases resulting in demyelination, Multiple sclerosis, bone diseases and osteoporosis.

**26.** Use of a compound according to any one of Claims 1 to 15, for the preparation of a medicament for use in the treatment and/or prevention of motor diseases and disorders, in particular dyskinesia or Parkinson's diseases, senile dementia and Alzheimer's disease.

## Patentansprüche

**1.** Verbindung der Formel (I):

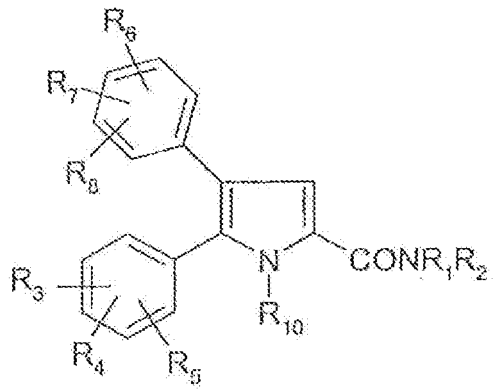

(I)

worin:

- $R_1$ für ein Wasserstoffatom oder eine $(C_1-C_4)$-Alkylgruppe, die gegebenenfalls durch ein oder mehrere Fluoratome substituiert ist, steht;
- $R_3$ für:
- entweder einen Homopiperidin-1-yl, Piperidin-1-yl-, Pyrrolidin-1-yl- oder Azetidin-1-yire.st, wobei 1 die Reste gegebenenfalls ein- oder zweifach durch einen jeweils unabhängig voneinander unter Fluoratom, einer $(C_1-C_4)$-Alkylgruppe, einer $(C_1-C_4)$-Alkoxygruppe, einer Trifluormethylgruppe, einer $-CCF_3$-Gruppe, einer $-CH_2OH$-Gruppe, einer $-CONH_2$-Gruppe und/oder einer Phenylgruppe ausgewählten Substituenten substituiert sind, wobei die Phenylgruppe gegebenenfalls ein- oder zweifach durch einen jeweils unabhängig voneinander unter einem Halogenatorn, einer $-CF_3$-Gruppe, einer Methoxygrüppe und/oder einer Trifluormerhoxygruppe ausgewählten Substituenten substituiert ist;
- oder eine Amino-$(C_1-C_6)$-alkylgruppe, die gegebenenfalls durch einen oder mehrere jeweils unabhängig voneinander unter einem Fluoratom, einer Hydroxylgruppe, einer $-CONH_2$-Gruppe und/oder einer Phenylgruppe ausgewählte Substituenten substituiert ist, wobei die Phenylgruppe gegebenen-falls ein- oder zweifach durch einen jeweils unabhängig voneinander unter einem Halogenatom, einer $-CF_3$-Gruppe, einer Methoxygruppe und/ oder einer Trifluormethoxygruppe ausgewählten Substituenten substituiert ist;

steht;

- oder $R_1$ und $R_2$ zusammen mit dem Stickstoffatom, an das sie gebunden sind:
- entweder einen Piperazin-1-yl- oder 1,4-Drazepan-1-ylrest, wobei die Reste gegebenenfalls durch einen unter einer Phenylgruppe, einer Benzodioxolylgruppe, einer Benzodioxolylmethylgruppe, einer Tetrahydrofuranyl carbonylgruppe, einer $-COR_{11}$-Gruppe und/oder einer $-CH_2COR_{11}$-Gruppe ausgewählten Substituenten substituiert, sind, wobei die phenylgruppe selbst gegebenenralls ein-oder mehrfach durch einen jeweils unabhängig voneinander unter einem Halogenatom, einer $(C_1-C_4)$-Alkylgruppe, einer Trifluromethylgruppe, einer Hydroxylgruppe, einer $(C_1-C_4)$-Alkoxygruppe und/oder einer Cyanogruppe ausgewählten Substituenten substituiert ist;
- oder einen Homopiperidin- 1- yl-, Piperidin- 1- yl-, pyrrolidin- 1- yl- oder Azetidin- 1- ylrest, wobei die Reste gegebenenfalls ein- oder zweifach, durch einen jeweils unabhängig voneinander unter:

  . einem Fluoratom, einer Cyanogruppe, einer $-COR_{11}$-Gruppe, einer $-NR_{12}R_{13}$-Gruppe, einer $-NHCOR_{14}$-Gruppe, einer $-CH_2COR_{11}$-Gruppe, einer $-SO_2R_{11}$-Gruppe und/oder einer $-SO_2NR_{13}R_{13}$-Gruppe
  . und/oder einer Phenylgruppe oder einer Pyridinylgruppe; wobei die Gruppen gegebenenfalls ein- oder mehrfach durch einen jeweils unabhängig voneinander unter einem Halogenatom, einer $(C_1-C_{4)}$-Alkylgruppe, einer Trifluormethylgruppe, einer Hydroxylgruppe, einer $(C_1-C_4)$-Alkoxygruppe und/oder einer Cyanogruppe ausgewählten Substituenten substituiert sind;
  . und/oder einer Benzylgruppe, die gegebenenfalls ein- oder mehrfach durch einen jeweils unabhängig voneinander unter einem Halogenatom, einer $(C_1-C_4)$-Alkylgruppe, einer Trifiuormethylgruppe; einer Hydroxylgruppe, einer $(C_1-C_4)$-Alkoxygruppe und/oder einer Cyanogruppe ausgewählten Substituent.en substituiert ist;
  . und/oder einer Piperidin-1-yl-, Pyrrolidin-1-yl-oder Azetidin-1-ylgruppe, wobei die Gruppen gegebenenfalls

ein- oder mehrfach durch einen jeweils unabhängig voneinander unter einem Fluoratom, einer ($C_1$-$C_4$)-Alkylgruppe, einer ($C_1$-$C_4$)-Alkoxygruppe, einer Hydroxygruppe, einer Trifluor-methylgruppe und/oder einer $OCF_3$-Gruppe ausgewählten Substituenten substituiert sind;

. und/oder einer Aminophenyl- oder aminobenzylgruppe, wobei die Gruppen gegebenenfalls ein- oder mehrfach durch einen jeweils unabhängig voneinander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten substituiert sind;

. und/ader einer Amino-($C_1$-$C_4$)-alkylgruppe, die gegebenenfalls ein- oder mehrfach durch einen jeweils unabhängig, voneinander unter einem Halogenatom, einer Hydroxylgruppe, einer ($C_1$-$C_4$)-Alkoxygruppe und/oder einer Cyanogruppe ausgewählten Substituenten substituiert ist;

. und/oder einer Amino-($C_3$-$C_7$)-cycloalkylgruppe, die gegebenenfalls ein- oder mehrfach, durch einen jeweils unabhängig voneinander unter einem. Halogenatom, einer Hydroxylgruppe, einer ($C_1$-$C_4$)-Alkylgruppe, einer ($C_1$-$C_4$)-Alkoxygruppe und/oder einer Cyanogruppe ausgewählten Substituenten substituiert ist, wobei die ($C_1$-$C_4$)-Alkylgruppe gegbenenfalls ein- oder mehrfach durch ein Fluoracom substituiert ist;

ausgewählten Substituenten substituiert sind; bilden;

- $R_3$, $R_4$, $R_5$, $R_6$, $R_7$ und $R_8$ jeweils unabhängig voneinander für ein Wasserstoffatom, ein Halogenatom, eine -CN-Gruppe, eine -$S(O)_nR14$-Gruppe, eine -$OSO_2R_{14}$-gruppe, eine ($C_1$-$C_6$)-Alkylgruppe, die gegebenenfalls ein- oder mehrfach durch ein Fluoratom substituiert ist, und/oder eine ($C_1$-$C_6$)-Alkoxygruppe, die gegebenenfalls ein- oder mehr-Frach durch ein Fluoratom substituiert ist, stehen, mit der Maßgabe, daß einer der beiden Substituenten $R_3$ und $R_6$ für eine Y-A-$R_9$-Gruppe steht;
- Y für eine direkte Bindung, ein Sauerstotfatom, eine -$S(O)_n$-gruppe, eine -$OSO_1$-Gruppe oder eine -N($R_{18}$)-Gruppe steht;
- A für eine ($C_1$-$C_4$)-Alkylengruppe, die gegebenenfalls ein- oder mehrfach durch einen jeweils unabhängig voneinander unter einer ($C_1$-$C_4$)-Alkylgruppe ausgewählten. Substituenten und/oder durch ein Fluoratom substituiert ist, steht;
- $R_9$ für eine -$OR_{12}$-, -CN-, -$CO_2H$-, $NR_{12}R_{13}$-, -$CONR_{12}R_{13}$-., -$NR_{15}COR_{12}$-, -$CONHNH_2$-, -CONHO-, -$CONHSO_2R_{14}$-, -$S(O)_nR_{14}$-, "$SO_2NR_{12}R_{13}$-, -$NR_{15}SO_2R_{14}$- oder -$NR_{15}SO_2NR_{12}R_{13}$-Gruppe oder einen unter;

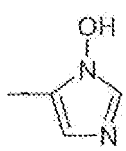 ; 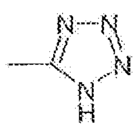 ; 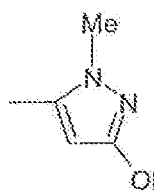 ; 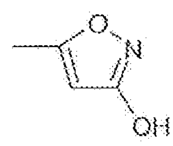 ;

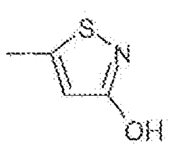 ; 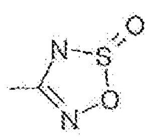 ; 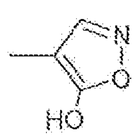 ; 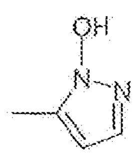

ausgewählten aromatischen Heterocyclus steht;

- $R_{10}$ für Wasserstoff oder eine ($C_1$-$C_4$)-Alkylgruppe steht;
- $R_{11}$ für;

eine ($C_1$-$C_4$)-Alkylgruppe, eine Phenylgruppe, eine Benzylgruppe, eine ($C_1$-$C_4$)-Alkoxygruppe oder eine ($C_1$-$C_3$)-Alkylen-O-($C_1$-$C_3$)-alkylgruppe, wobei die Gruppen gegebenenfalls durch einen oder mehrere jeweils unabhängig voneinander unter einer ($C_1$-$C_4$)-Alkoxygruppe, einer Hydroxygruppe und/oder einem Fluoratom ausgewählte Substituenten substituiert sind;
Trifluormethyl
und/oder eine -$NR_{16}R_{17}$-Gruppe
steht;

- und $R_{13}$ jeweils unabhängig voneinander für ein Wasserstoffatom oder eine ($C_1$-$G_4$)-Alkylgruppe, die gegebenenfalls, durch einen oder mehrere jeweils unabhängig voneinander unter einem Fluoratom, -OH und/oder -$RO_{14}$ ausgewählte Substituenten substiist, stehen;
- oder $R_{12}$ und $R_{13}$ zusammen mit dem Stickstoffatom, an das sie gebunden sind, einen 4- bis 7-gliedrigen heterocyclischen Rest, der ein zweites, unter einem Stickstoffatom, einem Sauerstoffatom oder einem Schwefelatom ausgewähltes Heteroatom enthalten kann, bilden;
- n für 0, 1 oder 2 steht;
- $R_{14}$ für eine ($C_1$-$C_4$)-Alkylgruppe, die gegebenenfalls durch ein oder mehrere Fluoratome substituiert ist, steht;
- $R_{15}$ für ein Wasserstoffatom oder eine ($C_1$-$C_4$)-Alkylgruppe steht;
- $R_{16}$ und $R_{17}$ jeweils unabhängig voneinander für:

  . ein Wasserstoffatom
  . und/oder eine Benzylgruppe, die gegebenenfalls ein- oder mehrfach durch einen jeweils unabhängig voneinander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten substituiert ist;
  . und/oder eine ($C_1$-$C_6$)-Alkylgruppe, die gegebenenfalls ein- oder mehrfach durch einen oder mehrere jeweils unabhängig voneinander unter einem Halogenatom, -OH und/oder -$OR_{14}$ ausgewählte substituiert ist;

stehen;

- $R_{18}$ für ein Wasserstoffatom oder eine ($C_1$-$C_4$)-Alkylgruppe, die gegebenenfalls durch ein oder mehrere Fluoratome substituiert ist, steht;
- Alk für eine ($C_1$-$C_4$)-Alkylgruppe steht;

oder ein Salz davon.

2. Verbindung nach Anspruch 1 der Formel (I), **dadurch gekennzeichnet, daß** Y für ein Sauerstoffatom steht, oder ein Salz davon,

3. Verbindung nach Anspruch 1 der Formel (I), **dadurch gekennzeichnet, daß** Y einer direkten Bindung entspricht, oder ein Salz davon,

4. Verbindung nach Anspruch 1 der Formel (I), **dadurch gekennzeichnet, daß** Y für eine -$S(O)_n$-Gruppe steht, oder ein Salz davon.

5. Verbindung nach Anspruch 1 der Formel (I), **dadurch gekennzeichnet, daß** Y für eine -$O(SO_2)$-Gruppe steht, oder ein Salz davon.

6. Verbindung nach Anspruch 1 der Formel (I), **dadurch gekennzeichnet, daß** Y für eine -N (R1a)-Gruppe steht, oder ein Salz davon.

7. Verbindung nach einem der vorhergehenden Ansprüche, **dadurch gekennzeichnet, daß** der Substituent $R_6$ für eine Gruppe Y-A-$R_9$ steht und der Substituent $R_3$ für ein Wasserstoffatom, ein Halogenatom, eine -CN-Gruppe, eine -$S(O)_nR_{14}$-Gruppe, eine -$OSO_2R_{14}$-Gruppe, eine ($C_1$-$C_6$)-Alkyl-gruppe die gegebenenfalls ein- oder mehrfach durch ein Fluoratom substituiert ist, oder eine ($C_1$-$C_6$)-Alkoxygruppe, die gegebenenfallse ein- oder mehrfach durch ein Fluoratom substituiert ist, steht, oder ein Salz davon.

8. Verbindung nach einem der Ansprüche 1 bis 6, **dadurch gekennzeichnet, daß** der Substituent $R_3$ für eine Gruppe Y-A-$R_9$ steht und der Substituent $R_6$ für ein Wasserstoffatom, ein Halogenatom, eine -CN-Gruppe, eine - -S $(O)_nR_{14}$,-Gruppe, eine -$OSO_2R_{14}$-Gruppe, eine ($C_1$-$C_6$)-Alkylgruppe, die gegebenenfalls ein-oder mehrfach durch ein Fluoratom substituiert ist, oder eine ($C_1$-$C_6$)-Alkoxygruppe, die gegebenenfalls ein- oder mehrfach durch ein Fluoratom substituiert ist, steht, oder ein Salz davon.

9. Verbindung nach einem der vorhergehenden: Ansprüche, **dadurch gekennzeichnet, daß** A für eine unsubstituierte ($C_1$-$C_4$)-Alkylengruppe steht, oder ein Salz davon.

**10.** Verbindung nach einem der vorhergehenden Anspruche, **dadurch gekennzeichnet, daß** $R_9$ für eine- $OR_{12}$-, -$NR_{12}R_{13}$-, -$CONR_{12}R_{13}$-, -$NR_{15}COR_{12}$-, -$CONHNH_2$-, -CONHOH-, -$S(O)_nR_{14}$-, -$SO_2NR_{12}R_{13}$-, -$NR_{18}SO_2R_{14}$- oder -$NR_{15}SO_2NR_{12}R_{13}$-Gruppe steht, oder ein Salz davon.

**11.** Verbindung nach einem der vorhergehenden Ansprüche, **dadurch gekennzeichnet, daß**:

- $R_1$ für ein Wesserstoffatom oder eine ($C_1$-$C_4$)-Alkylgruppe, die gegebenenfalls durch ein oder mehrere: Fluoratome substituiert ist, steht;
- $R_3$ für:

  - entweder einen Homopiperidin-1-yl-, Piperidin-1-yl-, Pyrrolidin-1-yl- oder Azetidin-1-ylrest, wobei die Reste ein- oder zweifach durch einen jeweils unabhängig voneinander unter einem Fluoratom, einer ($C_1$-$C_4$)-Alkylgruppe, einer ($C_1$-$C_4$)-Alkoxygruppe, einer Trifluormethylgruppe, einer -$OCF_3$-Gruppe, einer -$CH_2OH$-Gruppe, einer -$CONH_2$-Gruppe und/oder einer Phenylgruppe ausgewählten Substituenten substituiert sind, wobei die Phenylgruppe gegebenenfalls ein- oder zweifach durch einen jeweils unabhängig voneinander unter einem Halogenatom, einer -$CF_3$-Gruppe, einer Methoxygruppe und/oder einer Trifluermethoxygruppe ausgewählten Substituenten substituiert ist;
  - oder eine Amino-($C_1$-$C_6$)-alkylgruppe, die durch einen oder mehrere jeweils unabhängig voneinander unter einem Fluoratom, einer Hydroxylgruppe, einer
  - $CONH_2$-Gruppe und/oder einer Phenylgruppe ausgewählte Substituenten substituiert ist, wobei die Phenylgruppe gegebenenfalls ein- oder zweifach durch einen jeweils unabhängig voneinander unter einem Halogenatom, einer -CF-Gruppe, einer Methoxygruppe und/oder einer Trifluormethoxygruppe ausgewählten Substituenten substituiert ist; steht;

oder ein Salz davon.

**12.** Verbindung nach Anspruch 11, **dadurch gekennzeichnet, daß** der Rest $R_2$ insbesondere für einen Homopiperidin-1-yl-, Piperidin-1-yl, Pyrrolidin-1-yl- oder Arzetidin-1-ylrest, wobei die Reste ein-oder zweifach durch einen jeweils unabhängig voneinander unter einem Fluoratom, einer ($C_1$-$C_4$)-Alkylgruppe, einer ($C_1$-$C_4$)-Alkoxygruppe, einer Trirluormethylgruppe, einer -$OCF_3$-Gruppe, einer -$CH_2OH$-Gruppe einer -$CONH_3$-Gruppe und/oder einer Phenylgruppe ausgewählten Substituenten substituiert sind, wobei die Phenylgruppe gegebenenfalls ein- ader zweifach durch einen jeweils unabhängig voneinander einer Halogenatom, einer -$CF_3$-Gruppe, einer Methoxygruppe und/ oder einer Trifluormethmethoxygruppe ausgewählten Substituenten substituiert ist; steht; oder ein Salz davon.

**13.** Verbindung nach einem der Ansprüche 1 bis 10, **dadurch gekennzeichnet, daß**:

$R_1$ und $R_3$ zusammen mit dem Stickstoffatom, an das sie gebunden sind;

entweder einen Piperazin-1-yl- oder 1,4-Diasepan-1-ylrest, wobei die Reste durch einen unter einer Phenylgruppe, einer Benzodioxolylgruppe, einer Benzodioxolylmethylgruppe, einer Tetrahydrofuranylcarbonylgruppe, einer -$COR_{11}$-Gruppe und/oder einer -$CH_2COR_{11}$-Gruppe ausgewählten Substituenten substituiert sind, wobei 1 die Phenylgruppe selbst 1; gegebenenfalls ein- oder mehrfach durch einen jeweils unabhängig voneinander unter eine Halogenatom, einer ($C_1$-$C_4$)-Alkylgruppe, einer Trifluromethylgruppe, einer Hydroxylgruppe, einer ($C_1$-$C_4$-Alkoxygruppe und/ oder einer Cyanogruppe ausgewählten Substituenten substituiert ist;

öder einen Homopiperidin-1-yl-, Piperidin-1-yl-, Pyrrolidin-1-yl- oder Azetidin-1-ylrest, wobei die Reste ein- oder zweifach durch einen jeweils unabhängig voneinander unter:

. einem Fluoratom, einer Cyanogruppe, einer -$COR_{11}$-einer -$NR_{12}R_{13}$-Gruppe, einer -$NHCOR_{14}$-Gruppe, einer -$CH_2COR_{11}$-Gruppe, einer -$SO_2R_{11}$-Gruppe und/oder einer -$SO_2NR_{12}R_{13}$-Gruppe

. und/oder einer Phenylgruppe oder einer Pyridinylgruppe; wobei die Gruppen gegebenenfalls ein- oder mehrfach durch einen jeweils unabhängig voneinander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten substituiert sind;

. und/oder einer Benzylgruppe, die ein- oder mehrfach, durch einen jeweils unabhängig vonernander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten substituiert ist;

. und/oder einer Piperidin-1-yl-, Pyrrolidin-1-yl-oder Azetidin-1-ylgruppe, wobei die Gruppen gegebenenfalls ein- oder mehrfach durch einen jeweils unabhängig voneinander unter einem Fluoratom, einer ($C_1$-$C_4$)-Alkylgruppe, einer ($C_1$-$C_4$)-Alkoxygruppe, einer Hydroxygruppe, einer Trifluoro-Methylgruppe, und/oder einer $OCF_3$-Gruppe ausgewählten Substrtuenten substituiert sind,
. und/oder einer Aminophenyl- oder Aminobenzyl gruppe, wobei die Gruppen gegebenenfalls ein- oder mehrfach durch einen jeweils unabhängig voneinander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/öder einer Cyanogruppe ausgewählten Substituenten substituiert sind,
. und/oder einer Amino-($C_1$-$C_6$)-alkylgruppe, die gegebenenfalls ein- oder mehrfach, durch einen jeweils unabhängig voneinander unter einem Halogenatom., einer Hydroxylgruppe, einer ($C_1$-$C_4$)-Alkoxygruppe und/oder einer Cyanogruppe ausgewählten Substituenten substituiert ist;
. und/oder einer Amino-($C_3$-$C_7$)-cycloalkylgruppe, die gegebenenfalls ein- oder mehrfach durch einen jeweils unabhängig voneinander unter einem Halogenatom, einer Hydroxylgruppe, einer ($C_1$-$C_4$)-Alkylgruppe einer ($C_1$-$C_4$)-Alkoxygruppe und/oder einer Cyanogruppe ausgewählten Substituenten substituiert, ist, wobei die ($C_1$-$C_4$)-Alkylgruppe gegebenenfalls ein- oder mehrfach durch ein Fluoratom substituiert ist;

ausgewählten Substituenten substituiert sind:
bilden;
oder ein Salz davon.

**14.** Verbindung nach Anspruch 13, **dadurch gekennzeichnet, daß**:

$R_1$ = und $R_2$ zusammen mit dem Stickstoffatom, an das sie gebunden sind;

einen Homopiperidin-1-yl- piperidin-1-yl-, Pyrrolidin-1-yl- oder Azetidin-1-ylrest, wobei die Reste ein- oder zweifach durch einen jeweils unabhängig voneinander unter:

. einem Fluoratom, einer Cyanogruppe, einer $-COR_{11}$-Gruppe, einer $-NR_{12}R_{13}$-Gruppe; einer $-NECOR_{14}$ Gruppe, einer $-CH_2COR_{11}$-Gruppe, einer $-SO_2R_{14}$-Gruppe und/oder einer $-SO_2NR_{12}R_{13}$-Gruppe
. und/oder einer Phenylgruppe oder einer Pyridinylgruppe; wobei die Gruppen gegebenenfalls, ein- oder mehrfach durch einen jeweils unabhängig voneinander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten substituiert sind;
. und/oder einer Senzylgruppe, die ein- oder mehrfach durch, einen, jeweils unabhängig voneinender unter einem Halögenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten subist"
. und/oder einer Piperidin-1-yl-, Pyrrolidin-1-yl-oder Azetidin-1-ylgruppe, wobei die Gruppen gegebenenfalls ein- oder mehrfach durch einen jeweils unabhängig voneinander unter einem Fluoratom, einer ($C_1$-$C_4$)-Alkylgruppe, einer ($C_1$-$C_4$)-Alkoxygruppe, einer Hydroxygruppe, einer Trifluormethylgruppe und/oder einer $OCF_3$-Gruppe ausgewählten Substituenten substituiert sind;
. und/oder einer Aminophenyl- oder Aminobenzylgruppe, wobei die Gruppen gegebenenfalls ein- öder mehrfach durch einen jeweils unabhängig voneinander unter einem Halogenatom, einer ($C_1$-$C_4$)-Alkylgruppe, einer Trifluormethylgruppe, einer Hydroxylgruppe, einer ($C_1$-$C_4$)-Alkoxygruppe und/oder einer Cyanogruppe ausgewählten Substituenten substituiert sind;
. und/oder einer Amino-($C_1$-$C_4$)-alkylgruppe; die gegebenenfalls ein- oder mehrfach durch einen jeweils unabhängig voneinander unter einem Halogenatom, einer Hydroxylgruppe, einer ($C_1$-$C_4$)-Alkoxygruppe und/oder einer Cyanogruppe ausgewählten Substituenten substituiert ist;
. und/oder einer Amino-($C_3$-$C_7$)-cycloalkylgruppe, die gegebenenfalls ein- oder mehrfach, durch einen jeweils unabhängig voneinander unter einem Halogenatom, einer Hydroxylgruppe, einer ($C_1$-$C_4$)-Alkylgruppe, einer ner und/oder einer Cyanogruppe ausgewählten Substituenten substituiert ist, wobei 1 die ($C_1$-$C_4$)-Alkylgruppe gegebenenfalls ein- oder mehrfach durch ein Fluoratom substituiert ist;

ausgewählten Substituenten substituiert sind;
bilden;
oder ein Salz davon.

**15.** Verbindung nach Anspruch 1, **dadurch gekennzeichnet, daß** die Verbindung aus der Gruppe bestehend aus:

- 1-[4-(2,4-Dichlorphenyl)-5-[4-(3-hydroxypropoxy)phenyl]-1-methyl-1H-pyrrol-2-carbonyl]-4-(3-fluorbenzylamino)piperidin-4-carboxamid,
- 1-[5-(4-Carbamoylmethoxy)phenyl-4-(2,4-dichlorphenyl)-1-methyl-1H-pyrrol-2-carbonyl]-4-phenylpiperidin-4-carboxamid,
- 1-[4-(2-Chlorphenyl)-5-[4-(3-hydroxyprcpoxy)-phenyl]-1-methyl-1H-pyrrol-2-carbonyl]-4-(4-fluor-benzylamino)piperidin-4-carboxamid und Salzen davon

ausgewählt ist.

**16.** Verfahren zur Herstellung einer Verbindung, der Formel (I), **dadurch gekennzeichnet, daß** man die Säure der Formel (II) eder ein funktionelles Derivat dieser Säure der Formel (II) :

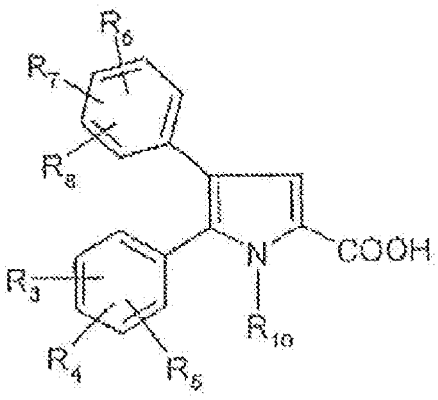

(II)

worin $R_3$, $R_4$, $R_5$, $R_6$, $R_7$, $R_8$ und $R_{10}$ die in einem der Ansprüche 1 bis 10 und 15 angegebene Bedeutung besitzen, mit einem Amin der Formel $HMR_1R_2$, worin $R_1$ und $R_2$ die in einem der Ansprüche 1 und 11 bis 15 angegebene Bedeutung besitzen, behandelt.

**17.** Herstellungsverfahren nach Anspruch 16, **dadurch gekennzeichnet, daß** man ein funktionelles Derivat der Formel (IIb):

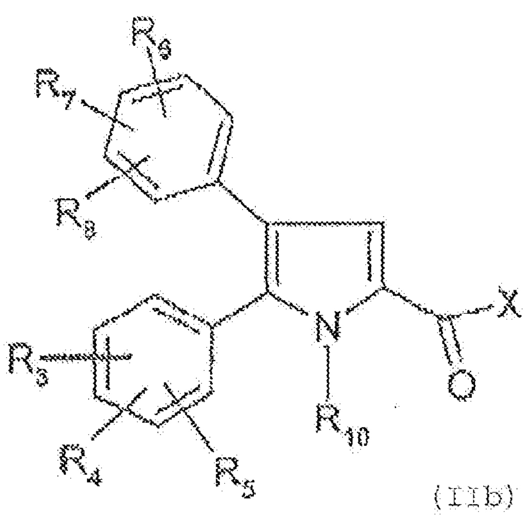

(IIb)

worin:

- X für eine Hydroxylgruppe, ein Halogenatom, $(C_1-C_4)$-Alkoxy oder Benzyloxy steht
- und $R_3$, $R_4$, $R_5$, $R_6$, $R_7$, $R_8$ und $R_{10}$ die in einem der Ansprüche 1 bis 10 # und 15 angegebene Bedeutung besitzen,

mit einem Amin der Formel $HNR_1R_2$, worin $R_1$ und $R_2$ die in einem der Ansprüche 1 und 11 bis 14 angegebene Bedeutung besitzen, behandelt.

**18.** Verbindung der Formel (IIb):

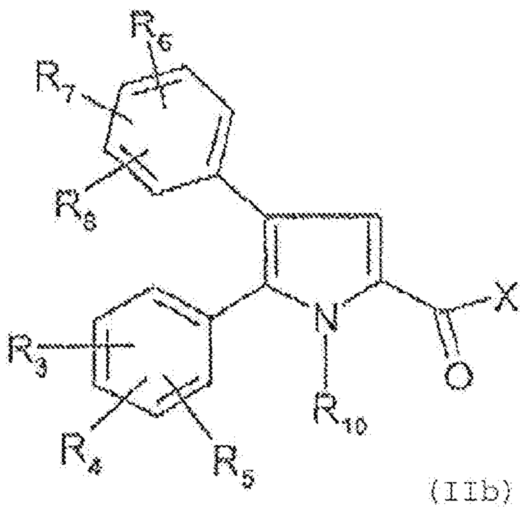

(IIb)

worin:

- x für eine Hydroxylgruppe, ein Halogenatom, ($C_1$-$C_4$)-Alkoxy oder Benzyloxy steht
- und $R_2$, $R_4$, $R_5$, $R_6$, $R_7$, $R_8$ und $R_{10}$ die für die Verbindungen der Formel (I) in einem der Ansprüche 1 bis 10 angegebene Bedeutung besitzen.

**19.** Arzneimittel, **dadurch gekennzeichnet, daß** es eine Verbindung der Formel (I) nach einem der Ansprüche 1 bis 15 oder ein Additionssalz dieser Verbindung der Formel (I) mit einer pharmazeutisch unbedenklichen Säure umfaßt.

**20.** Pharmazeutische Zusammensetzung, **dadurch gekennzeichnet, daß** sie eine Verbindung der Formel (I) nach einem der Ansprüche 1 bis 15 oder ein Additionssalz dieser Verbindung der Formel (I) mit einer pharmazeutisch unbedenklichen Säure sowie mindestens einen pharmazeutisch unbedenklichen Hilfsstoff umfaßt.

**21.** Verwendung einer Verbindung nach einem der Ansprüche 1 bis 15 zur Herstellung eines Arzneimittels zur Behandlung und/oder Prävention von psychiatrischen Störungen, Substanzabhängigkeit und -entwöhnung, kognitiven Störungen, Aufmerksamkeits- und Nachsamkeitsstörungen und akuten und chronischen neurodegenerativen Erkrankungen.

**22.** Verwendung einer Verbindung nach einem der Ansprüche 1 bis 15 zur Herstellung eines Arzneimittels zur Behandlung und/oder Prävention von Stoffwechselstörungen, Appetitstörungen, Appetenzstörungen, Obesitas, Bulimie, Diabetes, metabolischem Syndrom und Dyslipidämie.

**23.** Verwendung einer Verbindung nach einem der Ansprüche 1 bis 15 zur Herstellung eines Arzneimittels zur Behandlung und/oder Prävention von Schmerzen, neuropathischen Schmerzen, peripheren akuten Schmerzen, entzündungsbedingten chronischen Schmerzen und durch Antikrebsbehandlung induzierten Schmerzen.

**24.** Verwendung einer Verbindung nach einem der Ansprüche 1 bis 15 zur Herstellung eines Arzneimittels zur Behandlung und/oder Prävention von Magen-Darm-Störungen, Erbrechen, Durchrallstörungen, Geschwüren und Lebererkrankungen,

**25.** Verwendung einer Verbindung nach einem der Ansprüche 1 bis 15 zur Herstellung eines Arzneimittels zur Behandlung und/oder Prävention von Entzündungsphänomenen, Erkrankungen des Immunsystems, rheumatoider Arthritis, Erkrankungen, die zu Demyelinisierung führen, multipler Sklerose, Knochenerkrankungen und Osteoporose.

**26.** Verwendung einer Verbindung nach einem der Ansprüche 1 bis 15 zur Herstellung eines Arzneimittels zur Behandlung und/oder Prävention von Bewegungserkrankungen und -störungen, insbesondere Dyskinesien oder Parkinson-Krankheit, senilen Demenzen und Alzheimer-Krankheit.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006024777 A


### Littérature non-brevet citée dans la description


- *Chem. Comm.,* 1988, 475-477
- *J. Chem. Soc. Perkin Trans. 1,* 2002, 622-628
- **Green et al.** Protective Group in Organic Synthesis. John Wiley & Sons, Inc, 2007
- *J. Med. Chem.,* 1964, vol. 7, 619-622
- *J. Org. Chem.,* 1990, vol. 55, 4207-4209
- **M. Rinaldi-Carmona et al.** *FEBS Letters,* 1994, vol. 350, 240-244
- **M. Bouaboula et al.** *J. Biol. Chem.,* 1995, vol. 270, 13973-13980
- **M. Rinaldi-Carmona et al.** *J. Pharmacol. Exp. Ther.,* 1996, vol. 278, 871-878
- **M. Bouaboula et al.** *J. Biol. Chem.,* 1997, vol. 272, 22330-22339
- **M. Rinaldi-Carmona et al.** *Life Sciences,* 1995, vol. 56, 1941-1947
- **M. Rinaldi-Carmona et al.** *J. Pharmacol. Exp. Ther.,* 2004, vol. 310, 905-914
- **Rinaldi-Carmona M. et al.** *JPET,* 2004, vol. 310, 905-914